# EUROPÄISCHE PATENTSCHRIFT

(11) **EP 2 150 551 B1**
(45) Veröffentlichungstag und Bekanntmachung des Hinweises auf die Patenterteilung: **16.01.2013**
(21) Anmeldenummer: 08749391.2
(22) Anmeldetag: 08.05.2008
(51) Int. Cl.: C07D 413/10, C07D 498/06, A61K 31/506, A61K 31/437, A61K 31/424, A61P 35/00

(54) **2-OXO-3-BENZYL-BENZOXAZOL-2-ONE DERIVATE UND VERWANDTE VERBINDUNGEN ALS MET-KINASE INHIBITOREN ZUR BEHANDLUNG VON TUMOREN**
2-OXO-3-BENZYL-BENZOXAZOL-2-ONE DERIVATIVES AND RELATED COMPOUNDS AS MET KINASE INHIBITORS FOR THE TREATMENT OF TUMORS
DÉRIVÉS DE 2-OXO-3-BENZYL-BENZOXAZOLE-2-ONE ET COMPOSÉS APPARENTÉS UTILISÉS COMME INHIBITEURS DE KINASE MET DANS LE TRAITEMENT DE TUMEURS

(30) Priorität: 06.06.2007 DE 102007026341
(43) Veröffentlichungstag der Anmeldung: 10.02.2010
(73) Patentinhaber: Merck Patent GmbH, 64293 Darmstadt (DE)
(72) Erfinder: SCHADT, Oliver, 63517 Rodenbach (DE); DORSCH, Dieter, 64372 Ober-Ramstadt (DE); STIEBER, Frank, 69121 Heidelberg (DE); BLAUKAT, Andree, 69198 Schriesheim (DE)
(86) Internationale Anmeldenummer: PCT/EP2008/003696
(87) Internationale Veröffentlichungsnummer: WO 2008/148449

(56) Entgegenhaltungen:
- WO-A1-2004/058762
- WO-A1-2007/057093
- US-B1- 6 242 461
- FLOUZAT, CHRISTINE ET AL: "Synthesis and N-substitution of an uncommon heterocyclic system: oxazolo[5,4-b]pyridin-2(1H)-one" TETRAHEDRON LETTERS, Bd. 33, Nr. 32, 1992, Seiten 4571-4574, XP002496354
- UCAR, HUSEYIN ET AL: ""Fries Like" Rearrangement: a novel and efficient method for the synthesis of 6-acyl-2(3H)-benzoxazolones and 6-acyl-2(3H)- benzothiazolones" TETRAHEDRON, Bd. 54, Nr. 9, 1998, Seiten 1763-1772, XP002496355
- DATABASE CA [Online] CHEMICAL ABSTRACTS SERVICE, COLUMBUS, OHIO, US; 2002, DUSHAMOV, D. A. ET AL: "Acylation of 6-halobenzoxazolin-2-ones by acid chlorides in the presence of a small quantity of iron(III) chloride hexahydrate" XP002496356 gefunden im STN Database accession no. 2002:552455 & O'ZBEKISTON KIMYO JURNALI, Bd. 2, 2002, Seiten 13-16,
- DATABASE CA [Online] CHEMICAL ABSTRACTS SERVICE, COLUMBUS, OHIO, US; 1979, DOMAGALINA, EUGENIA ET AL: "Acylation of benzoxazolin-2-ones and 3-hydroxy-1,2-benzisoxazoles" XP002496357 gefunden im STN Database accession no. 1979:474510 & POLISH JOURNAL OF PHARMACOLOGY AND PHARMACY, Bd. 30, Nr. 5, 1978, Seiten 717-723,
- DATABASE CA [Online] CHEMICAL ABSTRACTS SERVICE, COLUMBUS, OHIO, US; 1967, NITTA, YOSHIHIRO ET AL: "Benzoxazolone derivatives" XP002496358 gefunden im STN Database accession no. 1967:105000 & JP 42 001467 B1 (CHUGAI PHARMACEUTICAL CO., LTD.) 24 Januar 1967
- DATABASE CA [Online] CHEMICAL ABSTRACTS SERVICE, COLUMBUS, OHIO, US; 1965, SAM, JOSEPH ET AL: "Hydrogenation of 3-(2-nitrobenzoyl)-2-benzoxazolinone to 1-hydroxy-3-(2-hydroxyphenyl)quinazoline-2 ,4-dione" XP002496359 gefunden im STN Database accession no. 1965:51634 & JOURNAL OF HETEROCYCLIC CHEMISTRY, Bd. 1, Nr. 5, 1964, Seiten 245-246,

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Die vorliegende Erfindung betrifft insbesondere Verbindungen und die Verwendung von Verbindungen, bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.

Ein anderer Met-Kinase-Inhibitor zur Krebstherapie ist von J.G. Christensen et al. in Cancer Res. 2003, 63(21), 7345-55 beschrieben. Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung einzelne Verbindungen gemäß Anspruch 1 umfaßt von der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und - verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Die vorliegende Erfindung richtet sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die beanspruchten Verbindungen der Formel I gemäß Anspruch 1 auch bei der Behandlung gewisser Krebsformen einsetzen. Weiterhin können die beanspruchten Verbindungen der Formel I gemäß Anspruch 1 verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die beanspruchten Verbindungen der Formel I gemäß Anspruch 1 zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstum, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelliast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK ZU MET-KINASE-INHIBITOREN

Thiadiazinone sind in WO 03/037349 offenbart 4,5-Dihydropyrazoles zur Krebsbekämpfung kennt man aus WO 03/079973 A2.

Chinolinderivate sind in EP 1 411 046 A1 beschrieben. Pyrrol-indolin-derivate sind in WO 02/096361 A2 offenbart. 1-Acyldihydropyrazolderivate kennt man aus WO 2007/019933.

Darüber hinaus sind andere Met-Kinase-Inhibitoren aus WO 2005/004607, WO 20051030140, WO 2006/014325, WO 2006/021881 und WO 2006/021881 bekannt.

Andere Benzoxazolonderivate kennt man aus:
D1: WO 2004/058762 A
D2: US-B1-6 242 461
D3: Flouzat, Christine et al., Tetrahedron Letters Vol. 33, Nr. 32, 1992, 4571-4574
D4: Ucar, Huseyin et al., Tetrahedron Vol. 54, Nr. 9, 1998, 1763-1772
D5: Database CA [Online] Chemical Abstracts Service, Columbus, Ohio, US; Dushamov, D.A. et al., XP002496356 gefunden im STN Database accession no. 2002:552455
D6: Database CA [Online] Chemical Abstracts Service, Columbus, Ohio, US; Domagalina, E. et al., XP002496357 gefunden im STN Database accession no. 1979:474510
D7: Database CA [Online] Chemical Abstracts Service, Columbus, Ohio, US; Nitta, Y. et al., XP002496358 gefunden im STN Database accession no. 1967:105000
D8: Database CA [Online] Chemical Abstracts Service, Columbus, Ohio, US; Sam, J. et al., XP002496359 gefunden im STN Database accession no. 1965:51634
D9: WO 2007/057093 A.

### ZUSAMMENFASSUNG DER ERFINDUNG

Die Erfindung betrifft einzelne Verbindungen gemäß Anspruch 1 umfaßt von der Formel I 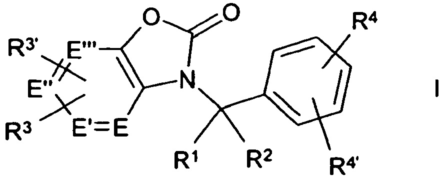 worin
- E, E', E", E"': jeweils unabhängig voneinander C oder N,
- R¹, R²: jeweils unabhängig voneinander H oder A,
- R¹ und R²: zusammen auch (CH₂)p, worin 1 oder 2 CH₂-Gruppe(n) durch O und/oder NH ersetzt sein können,
- R³: H, (CH₂)ₙCONH₂, (CH₂)nCONHA, (CH₂)ₙCONAA', A, COA, OH, OA, CONH(CH₂)ₘNH₂. CONH(CH₂)ₘNHA, CONH(CH₂)ₘNAA', CO(CH₂)ₘNH₂, CO(CH₂)ₘNHA, CO(CH₂)ₘNAA', CO(CH₂)ₘHet, CH(OH)A, CN, Het, Hal, CONH(CH₂)ₘNA-COOA. SO₂A, NH(CH₂)ₘNH₂, NH(CH₂)ₘNHA, NH(CH₂)mNAA', (CH₂)ₙCOOH, (CH₂)ₙCOOA, O(CH₂)ₘNH₂, O(CH₂)ₘNHA, O(CH₂)ₘNAA', OHet, N=CH-NAA', N=CH-NHA, N=CH-NH₂, O(CH₂)ₘHet, O(CH₂)ₘOH, O(CH₂)ₘOA, SO₂(CH₂)ₘOH, OCH(A)CH₂Het. OCH₂CH(OH)CH₂NHA, OCH₂C(AA')CH₂NAA', OCH₂CH(A)CH₂NAA', OCH₂CH(OH)CH₂OH, O(CH₂)ₘCONAA' oder O(CH₂)ₘCOHet,
- R³': H oder Hal,
- R⁴: Het¹, NHCOOR⁵, NHCONHR⁵, NHCOCONHR⁵, NO₂ oder NHCOA,
- R⁴': H oder Hal,
- R⁴ und R⁴': zusammen auch NHCONH,
- R⁵: A, (CH₂)ₘNH₂, (CH₂)ₘNHA, (CH₂)ₘNAA' oder (CH₂)ₘHet,
- Het: einen ein- oder zweikernigen gesättigten, ungesättigten oder aromatischen Heterocyclus mit 1 bis 4 N-, O- und/oder S-Atomen, der unsubstituiert ist oder ein-, zwei- oder dreifach durch Hal, A, OR⁶, N(R⁶)₂, NO₂, CN, COOR⁶, CON(R⁶)₂, NR³COA, NR⁶SO₂A, SO₂N(R⁶)₂, Pyridyl, S(O)ₘA, NHCOOA, NHCON(R⁶)₂, CHO, COA, =S, =NH, =NA und/oder =O (Carbonylsauerstoff) substituiert sein kann,
- Het¹: einen einkernigen aromatischen Heterocyclus mit 1 bis 4 N-, O- und/oder S-Atomen, der unsubstituiert ist oder ein-, zwei- oder dreifach, jeweils unabhängig voneinander, durch R³ substituiert sein kann,
- R⁶: H oder A,
- A, A': jeweils unabhängig voneinander unverzweigtes oder verzweigtes Alkyl mit 1-10 C-Atomen, worin 1-7 H-Atome durch OH, F, Cl und/oder Br ersetzt sein können, und/oder worin eine oder zwei CH₂-Gruppen durch O, S, SO, SO₂ und/oder CH=CH-Gruppen ersetzt sein können, oder cyclisches Alkyl mit 3-7 C-Atomen,
- Hal: F, Cl, Br oder I,
- m: 1, 2, 3 oder 4,
- n: 0, 1, 2, 3 oder 4
- p: 1, 2, 3, 4 oder 5, und, falls R³ an E' und R³' an E" gebunden ist,
- R³ und R³': zusammen auch CH=CH-CH=CH,
bedeuten,
sowie ihre pharmazeutisch verwendbaren Solvate, Salze, Tautomere und Stereoisomere, einschließlich deren Mischungen in allen Verhältnissen,

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel 1 gemäß Anspruch 1, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I gemäß Anspruch 1 und ihre Salze sowie ein Verfahren zur Herstellung von Verbindungen der Formel I sowie ihrer pharmazeutisch verwendbaren Salze, Solvate, Tautomeren und Stereoisomeren, dadurch gekennzeichnet, daß man
a) eine Verbindung der Formel II 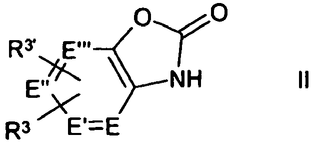 worin E, E', E", E"', R³ und R^{3'} die in Anspruch 1 entsprechenden Bedeutungen haben,
   mit einer Verbindung der Formel III 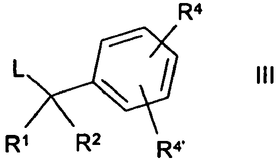 worin R¹, R², R⁴ und R^{4'} die in Anspruch 1 entsprechenden Bedeutungen haben und
   L Cl, Br, I oder eine freie oder reaktionsfähig funktionell abgewandelte OH-Gruppe bedeutet,
   umsetzt,
   oder
b) einen Rest R³ und/oder R⁴ in einen anderen Rest R³ und/oder R⁴ umwandelt, indem man
   i) eine Aminogruppe acyliert,
   ii) eine Carboxygruppe zu einem Amid umsetzt,
und/oder
eine Base oder Säure der Formel I in eines ihrer Salze umwandelt.

Vor- und nachstehend haben die Reste R¹, R², R³, R^{3'}, R⁴, R^{4'}, E, E', E" und E"' die bei der Formel I angegebenen Bedeutungen, sofern nicht ausdrücklich etwas anderes angegeben ist.

Abkürzungen:
- TFA: Trifluoressigsäure
- DCM: Dichlormethan

A, A' bedeuten, jeweils unabhängig voneinander, Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cyclisches Alkyl (Cycloalkyl) bedeutet vorzugsweise Cyclopropyl, Cyclobutyl, Cylopentyl, Cyclohexyl oder Cycloheptyl.

Het bedeutet, ungeachtet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3-oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder-8-chinolyl, 1,2,3,4-Tetrahydro-1-,-2-,-3-, -4-, -5-, -6-, -7- oder -8-isochinolyl, 2-, 3-, 5-, 6-, 7-oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

In einer weiteren Ausführungsform bedeutet Het vorzugsweise einen ein- oder zweikernigen gesättigten, ungesättigten oder aromatischen Heterocyclus mit 1 bis 3 N-, O- und/oder S-Atomen, der unsubstituiert ist oder ein-, zwei- oder dreifach durch A, Pyridyl und/oder =O (Carbonylsauerstoff) substituiert sein kann.

Het bedeutet besonders bevorzugt Piperidinyl, Pyrrolidin-yl, Morpholin-4-yl, Piperazin-yl, 1,3-Oxazolidin-3-yl, Imidazolidinyl, Oxazolyl, Oxadiazolyl, Thiazolyl, Thienyl, Furanyl, Pyridyl, 1-Aza-bicyclo[2.2.2]oct-3-yl, Pyridazinyl, Dihydropyridazinyl oder Pyrazolyl, wobei die Reste auch ein- oder zweifach durch A, Pyridyl und/oder =O (Carbonylsauerstoff) substituiert sein können.

Het¹ bedeutet, ungeachtet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

Het¹ bedeutet besonders bevorzugt 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4-oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl, die unsubstituiert oder ein- oder zweifach durch A, O(CH₂)ₘNH₂, O(CH₂)ₘNHA, O(CH₂)ₘNAA', Het, OHet, N=CH-NAA', N=CH-NHA, N=CH-NH₂, O(CH₂)ₘHet, OCH(A)CH₂Het, OCH₂CH(OH)CH₂NHA, O(CH₂)ₘCOHet, O(CH₂)ₘCONAA', OCH₂C(AA')CH₂NAA', OCH₂CH(A)CH₂NAA', OCH₂CH(OH)CH₂OH und/oder CONH(CH₂)ₘNAA' substituiert sind.

E bedeutet C oder N; E', E", E"' bedeuten vorzugsweise C.

R⁶ bedeutet vorzugsweise H, Methyl, Ethyl, Propyl, Isopropyl, Butyl oder tert.-Butyl.

Hal bedeutet vorzugsweise F, Cl oder Br, aber auch 1, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II und III sind in der Regel bekannt. Sind sie neu, so können sie aber nach an sich bekannten Methoden hergestellt werden.

Verbindungen der Formel können vorzugsweise erhalten werden, indem man eine Verbindung der Formel II mit einer Verbindung der Formel III umsetzt.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt in der Regel in Gegenwart eines säurebindenden Mittels vorzugsweise einer organischen Base wie DIPEA, Triethylamin, Dimethylanilin, Pyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, 1-Methyl-pyrrolidinon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt ist Acetonitril, Dichlormethan, NMP und/oder DMF.

Es ist weiterhin möglich, eine Verbindung der Formel 1 in eine andere Verbindung der Formel I umzuwandeln, indem man einen Rest R³ und/oder R⁴ in einen anderen Rest R³ und/oder R⁴ umwandelt.

Z.B. kann man freie Aminogruppen in üblicher Weise mit einem Säurechlorid oder -anhydrid acylieren, zweckmäßig in einem inerten Lösungsmittel wie Dichlormethan oder THF und /oder in Gegenwart einer Base wie Triethylamin oder Pyridin bei Temperaturen zwischen -60° und +30°.

Weiterhin kann man ein Oxy-amidinderivat zu einem Oxadiazolderivat cyclisieren, vorzugsweise in THF mit dem Burgess-Reagenz bei Temperaturen zwoschen 60° und 80°.

Man kann auch eine Carbonsäure unter Standardbedingungen in ein Carbonsäureamid umwandeln, vorzugsweise durch Umsetzung mit einem Amin.

Es ist ferner möglich, eine Verbindung der Formel I in eine andere Verbindung der Formel I umzuwandeln, indem man einen Rest R⁴ in einen anderen Rest R⁴ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen. Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lass en sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern. Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Salze, Solvate und Stereoisomere, einschließlich deren Mischungen in allen Verhältnissen, und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I gemäß Anspruch 1 und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen der Formel I gemäß Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Ebenfalls umfasst ist die Verwendung der Verbindungen der Formel I gemäß Anspruch 1 und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Die vorliegende Erfindung umfasst auch die Verwendung der Verbindungen der Formel I gemäß Anspruch 1 und/oder ihre physiologisch unbedenklichen Salze und Solvate zur Herstellung eines Arzneimittels zur Behandlung oder Vorbeugung von Retina-Vaskularisierung.

Verfahren zur Herstellung eines Arzneimittels zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.

Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I gemäß Anspruch 1 können an Patienten zur Behandlung von Krebs, insbesondere schnell wachsenden Tumoren, verabreicht werden.

Gegenstand der Erfindung ist somit die Verwendung von Verbindungen der Formel I gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Met-Kinase.

Bevorzugt ist die Verwendung von Verbindungen der Formel I gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden. Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Herstellung eines Arzneimittels zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I gemäß Anspruch 1 können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (CI 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανβ3-Funktion und Angiostatin);
(vi) gefäßschädigende Mittel, wie Combretastatin A4 und in den internationalen Patentanmeldungen WO 99102166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffrnann-La Roche) |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | TAS-103 (Taiho) |
| | 7-Ethyl-10-hydroxycamptothecin | Elsamitrucin (Spectrum) |
| | Topotecan | J-107088 (Merck & Co) |
| | Dexrazoxanet (TopoTarget) | BNP-1350 (BioNumerik) |
| | Pixantron (Novuspharrna) | CKD-602 (Chong Kun Dang) |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin | Losoxantron |
| | (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholinodoxorubicin | |
| | Mitoxantron (Novantron) | |
| | | |
| Antimitotische Mittel | Paclitaxel | SB 408075 |
| | Docetaxel | (GlaxoSmithKline) |
| | Colchicin | E7010 (Abbott) |
| | Vinblastin | PG-TXL (Cell |
| | Vincristin | Therapeutics) |
| | Vinorelbin | IDN 5109 (Bayer) |
| | Vindesin | A 105972 (Abbott) |
| | Dolastatin 10 (NCI) | A 204197 (Abbott) |
| | Rhizoxin (Fujisawa) | LU 223651 (BASF) |
| | Mivobulin (Warner-Lambert) | D 24851 (ASTA Medica) |
| | Cemadotin (BASF) | ER-86526 (Eisai) |
| | RPR 109881A (Aventis) | Combretastatin A4 (BMS) |
| | TXD 258 (Aventis) | Isohomohalichondrin-B |
| | Epothilon B (Novartis) | (PharmaMar) |
| | T 900607 (Tularik) | ZD 6126 (AstraZeneca) |
| | T 138067 (Tularik) | PEG-Paclitaxel (Enzon) |
| | Cryptophycin 52 (Eli Lilly) | AZ10992 (Asahi) |
| | Vinflunin (Fabre) | !DN-5109 (Indena) |
| | Auristatin PE (Teikoku Hormone) | AVLB (Prescient NeuroPharma) |
| | BMS 247550 (BMS) | Azaepothilon B (BMS) |
| | BMS 184476 (BMS) | BNP- 7787 (BioNumerik) |
| | BMS 188797 (BMS) | CA-4-Prodrug (OXiGENE) |
| | Taxoprexin (Protarqa) | Dolastatin-10 (NrH) |
| | | CA-4 (OXiGENE) |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International) |
| | Glufosfamid (Baxter International) | Apaziquon (Spectrum Pharmaceuticals) |
| | Albumin + 32P (Isotope Solutions) | 06-Benzylquanin |
| | Thymectacin (NewBiotics) | (Paligent) |
| | Edotreotid (Novartis) | |
| | | |
| Farnesyltransferase-Inhibitoren | Arglabin (NuOncology Labs) | Tipifarnib (Johnson & Johnson) |
| | Ionafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid |
| | Tariquidar (Xenova) | (Eli Lilly) |
| | MS-209 (Scherinq AG) | Biricodar-Dicitrat (Vertex) |
| | | |
| Histonacetyltransferase-Inhibitoren | Tacedinalin (Pfizer) | Pivaloyloxymethylbutyrat |
| | SAHA (Aton Pharma) | (Titan) |
| | MS-275 (Scherinq AG) | Depsipeptid (Fujisawa) |
| | | |
| Metalloproteinase-Inhibitoren Ribonucleosidred uktase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health) |
| | Triapin (Vion) | |
| | | |
| TNF-alpha-Agonisten / Antaqonisten | Virulizin (Lorus Therapeutics) | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie |
| | Oncophage (Antigenics) | (Anosys) |
| | GMK (Progenics) | Pentrix (Australian Cancer Technology) |
| | Adenokarzinom-Impfstoff (Biomira) | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe | MGV (Progenics) |
| | (CTL Immuno) | !3-Alethin (Dovetail) |
| | Melanom-Impfstoff (CTL | CLL-Thera (Vasogen) |
| | Immuno) | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | Arzoxifen (Eli Lilly) |
| | Dexamethason | |
| | | |
| Photodynamische Mittel | Talaporfin (Light Sciences) | Pd-Bacteriopheophorbid |
| | Theralux | (Yeda) |
| | (Theratechnologies) | Lutetium-Texaphyrin |
| | Motexafin-Gadolinium | (Pharmacyclics) |
| | (Pharmacyclics) | Hypericin |
| | | |
| Tyrosinkinase-Inhibitoren | Imatinib (Novartis) | Kahalid F (PharmaMar) |
| | Leflunomid | CEP- 701 (Cephalon) |
| | (Sugen/Pharmacia) | CEP-751 (Cephalon) |
| | ZDI839 (AstraZeneca) | MLN518 (Millenium) |
| | Erlotinib | PKC412 (Novartis) |
| | (Oncogene Science) | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PK1166 (Novartis) | IMC-1C11 (ImClone) |
| | GW2016 (GlaxoSmithKline) | |
| | EKB-509 (Wyeth) | |
| | EKB-569 (Wyeth) | |
| Verschiedene | SR-27897 (CCK-A-Inhibitor, | BCX-1777 (PNP-Inhibitor, |
| Mittel | Sanofi-Synthelabo) | BioCryst) Ranpirnase |
| | Tocladesin (cyclisches-AMP-Agonist, Ribapharm) | (Ribonuclease-Stimulans, Alfacell) |
| | Alvocidib (CDK-Inhibitor, Aventis) | Galarubicin (RNA-Synthese-Inhibitor, Dong-A) |
| | CV-247 (COX-2-Inhibitor, Ivy Medical) | Tirapazamin |
| | P54 (COX-2-Inhibitor, Phytopharm) | (Reduktionsmittel, SRI International) |
| | CapCell™ (CYP450-Stimulans, Bavarian Nordic) | N-Acetylcystein (Reduktionsmittel, Zambon) |
| | GCS-IOO (gal3-Antagonist, GlycoGenesys) | R-Flurbiprofen (NFkappaB-Inhibitor, Encore) |
| | G17DT-Immunogen (Gastrin-Inhibitor, Aphton) | 3CPA (NF-kappaB-Inhibitor, Active Biotech) |
| | Efaproxiral (Oxygenator, Allos Therapeutics) | Seocalcitol (Vitamin-D-Rezeptor-Agonist, Leo) |
| | PI-88 (Heparanase-Inhibitor, Progen) | 131-I-TM-601 (DNA-Antagonist, TransMolecular) |
| | Tesmilifen (Histamin-Antagonist, YM BioSciences) | Eflornithin (ODC-Inhibitor, ILEX Oncology) |
| | Histamin (Histamin-H2-Rezeptor- Agonist, Maxim) | Minodronsäure (Osteoclasten-Inhibitor, Yamanouchi) |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi-Synthelabo) | Rituximab (CD20-Antikörper, Genentech) |
| | CCI-779 (mTOR-Kinase-Inhibitor, Wyeth) | Gemtuzumab (CD33-Antikörper, Wyeth Ayerst) |
| | Exisulind (PDE-V-Inhibitor, Cell Pathways) | PG2 (Hämatopoese-Verstärker, Pharmagenesis) |
| | CP-461 (PDE-V-Inhibitor, Cell Pathways) | |
| | AG-2037 (GART-Inhibitor, Pfizer) | Immunol™ (Triclosan-Oralspülung, Endo) |
| | WX-UK1 (Plasminogenaktivator-Inhibitor, Wilex) | Triacetyluridin (Uridin-Prodrug, Wellstat) |
| | PBI-1402 (PMN-Stimulans, | SN-4071 (Sarkom-Mittel, Signature BioScience) |
| | ProMetic LifeSciences) | TransMID-107™ (Immunotoxin, KS Biomedix) |
| Bortezomib (Proteasom-Inhibitor, Millennium) | | PCK-3145 (Apoptose-Förderer, Procyon) |
| SRL-172 (T-Zell-Stimulans, SR Pharma) | | Doranidazol (Apoptose-Förderer, Pola) |
| TLK-286 (Glutathion-S-Transferase-Inhibitor, Telik) | | CHS-828 (cytotoxisches Mittel, Leo) |
| PT-100 (Wachstumsfaktor-Agonist, Point Therapeutics) | | trans-Retinsäure (Differentiator, NIH) MX6 (Apoptose-Förderer, MAXIA) |
| Midostaurin (PKC-Inhibitor, Novartis) | | Apomin (Apoptose-Förderer, ILEX Oncology) |
| Bryostatin-1 (PKC-Stimulans, GPC Biotech) | | Urocidin (Apoptose-Förderer, Bioniche) |
| CDA-11 (Apoptose-Förderer, Everlife) | | Ro-31-7453 (Apoptose-Förderer, La Roche) |
| SDX-101 (Apoptose-Förderer, Salmedix) | | Brostallicin (Apoptose-Förderer, Pharmacia) |
| Ceflatonin (Apoptose-Förderer, ChemGenex) | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I gemäß Anspruch 1 wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity- (Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidase-konjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.

Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 pl 0,9% NaCl-Lösung gewaschen. Die Messung der gebundenen Radioaktivität erfolgt mittels eines Szintillationsmessgerätes (Topcount NXT, Fa. Perkin-Elmer).

Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µpl ATP (Endkonzentration 1 µM kalt, 0,35 µCi ³³P-ATP)
50 µl Gemisch Met Kinase/Substrat in Assaypuffer;
   (10 ng Enzymlwell, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
      pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-lonisation) M⁺ |
| | FAB (Fast Atom Bombardment) (M+H)⁺ |
| | ESI (Electrospray lonization) (M+H)⁺ |
| APCI-MS (atmospheric pressure chemical ionization - mass spectrometry) (M+H)⁺. | |

### HPLC-Analysen (Methode A)

Säule: Chromolith RP18e 50*4.6 mm
Fluß: 4 ml/min
Solvent A: 0.05 M wässrige NaHPO₄
Solvent B: Acetonitril + 10 % Wasser
Gradient 8 min
0-1 Min: 99:1 -> 99:1
1-7 min: 99:1 - 1:99
7-8 min: 1:99 -> 1:99

### HPLC-Analyse (Methode B)

Flußrate: 2 ml/min
99:01 - 0:100 Wasser + 0.1 %(Vol.) TFA : Acetonitril + 0.1 %(Vol.) TFA
0.0 bis 0.2 min: 99:01
0.2 bis 3.8 min: 99:01-> 0:100
3.8 bis 4.2 min: 0:100
Säule: Chromolith Performance RP18e; 100 mm lang, Innendurchmesser 3 mm, Wellenlänge: 220nm

### HPLC-Analysen (Methode C)

Säule: Chromolith RP18e 50*4.6 mm
Fluß: 4 ml/min
Solvent A: 0.1 M Trifluoressigsäure in Wasser
Solvent B: 0.1 M Trifluoressigsäure in Acetonitril:Wasser (9:1)
Gradient 8 min
0-1 Min: 99:1 -> 99:1
1-7 min: 99:1 - 1:99
7-8 min: 1:99 -> 1:99

### LC-MS Methode:

Säule: Chromolith RP18e 50*4.6 mm
Fluß: 2.4 ml/min
Solvent A: 0.1 M Trifluoressigsäure in Wasser
Solvent B: 0.1 M Trifluoressigsäure in Acetonitril
0.0 bis 2.6 min: 96:04 (Solvent A:Solvent B) → 100% Solvent B
2.6 bis 3.3 min: 100% Solvent B

### Beispiel 1

Die Herstellung von [3-(5-Methoxy-2-oxo-benzooxazol-3-ylmethyl)-phenyl]-carbaminsäure-3-(4-methyl-piperazin-1-yl)-propylester ("A1") erfolgt analog nachstehendem Schema

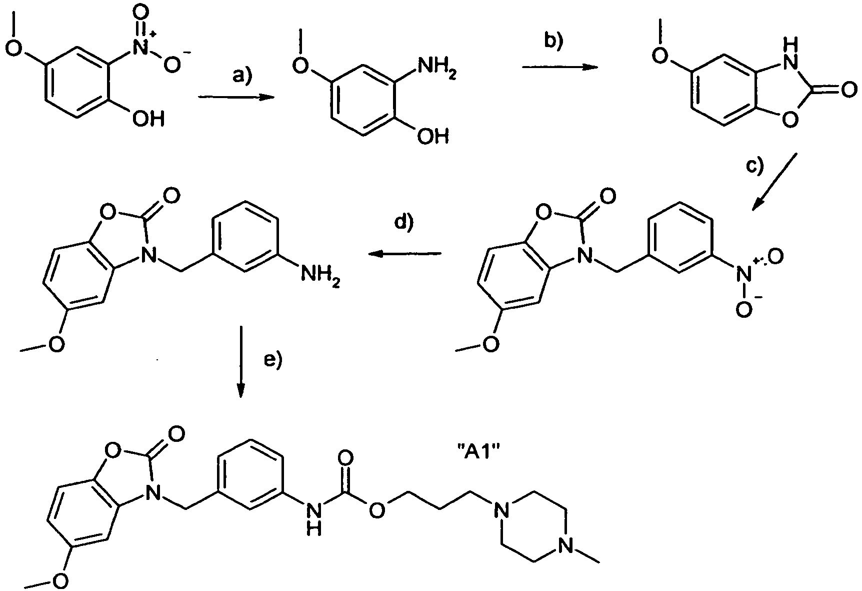

1.1 3,56 g 4-Methoxy-2-nitrophenol werden in 35 ml Methanol gelöst, unter Inertgasatmosphäre mit 1 g Pd/C-5% versetzt und durch Wasserstoffzugabe bei Normaldruck hydriert bis kein Edukt mehr im DC sichtbar ist.

Die nach Filtration erhaltene Hydrierlösung wird am Rotationsverdampfer zum Rückstand abgezogen. Der Rückstand wird in Aceton gelöst, über Celite unter Verwendung von Aktivkohle abgesaugt und die Mutterlauge zum Rückstand abgezogen. Der Rückstand wird mit Ether verrieben, abgesaugt und im Vakuumtrockenofen bei 50 °C getrocknet; F. 134-136°; ESI: 140 (M+H); HPLC Rt. = 2.19 min (Methode A); Ausbeute: 1,78 g (64%) 2-Amino-4-methoxyphenol.

1.2 In einem 100 ml Kolben, versehen mit Magnetrührer und Trockenröhrchen, werden 1,78 g 2-Amino-4-methoxyphenol in 20 ml THF gelöst, unter Rühren mit 2,12 g 1,1'-Carbonyldiimidazol versetzt und 1 h bei RT nachgerührt. Die dunkelbraune Reaktionslösung wird eingeengt, man gibt 50 ml Wasser zu, wobei es zu einer Fällung kommt. Diese wird abgetrennt. Es wird gut mit Wasser gewaschen, das Kristallisat in Dichlormethan aufgenommen, das Restwasser abgetrennt, unter Zugabe von Aktiv-Kohle getrocknet, über Celite abgesaugt und die Mutterlauge zum Rückstand abgezogen. Der Rückstand wird mit Ether verrieben, abgesaugt und getrocknet; F.173-175°; ESI: 166 (M+H); HPLC: Rt. 3,55 min (Methode A);
Ausbeute: 1,28 g (61 %) 5-Methoxy-3*H*-benzooxazol-2-on.

1.3 In einem 100 ml Rundkolben, versehen mit Magnetrührer, Kühler und Trockenröhrchen, werden 1,28 g 5-Methoxy-2-benzoxazolinon in 20 ml Acetonitril suspendiert, mit 1,88 g 3-Nitrobenzylbromid und 4,37 g Kaliumcarbonat versetzt und 1 h bei 80°C Badtemperatur gerührt. Es wird in Wasser gegossen, gut durchgerührt und abgesaugt. Das Kristallisat wird in Dichlormethan gelöst, das Restwasser abgetrennt, getrocknet, filtriert und das Lösungsmittel wird entfernt. Der Rückstand wird mit Ether verrührt, wieder abgesaugt und getrocknet;
F. 125-126°; ESI: 301 (M+H); HPLC: Rt. = 5.20 min (Methode A);
Ausbeute: 1,92 g (83%) 5-Methoxy-3-(3-nitro-benzyl)-3*H*-benzooxazol-2-on.

1.4 1,9 g 5-Methoxy-3-(3-nitro-benzyl)-3*H*-benzooxazol-2-on werden in einer Mischung aus 10 ml THF und 10 ml Methanol gelöst, unter Inertgasatmosphäre mit 1 g RaNi versetzt und durch Wasserstoffzugabe bei Normaldruck hydriert bis kein Edukt mehr im DC sichtbar ist. Die durch Filtration vom Katalysator befreite Lösung wirde über Na₂SO₄ getrocknet und anschließend eingeengt bis ein dicker Kristallbrei vorliegt. Dieser Kristallbrei wird mit ca. 200 ml Diethylether verdünnt, abgesaugt, mit Ether gewaschen und im Vakuumtrockenschrank bei 50 °C getrocknet;
F. 118 °; ESI: 271 (M+H); HPLC: Rt. = 4.56 (Methode A);
Ausbeute: 1,19 g (69%) 3-(3-Aminobenzyl)-5-methoxy-2-benzoxazolinon.

1.5 In einem Reaktionsgläschen, versehen mit einem Magnetrührer, werden 324,34 mg 3-(3-Aminobenzyl)-5-methoxy-2-benzoxazolinon in 5 ml Dichlormethan suspendiert, mit 252,03 µl Triethylamin versetzt, unter Kühlen und Rühren 145,35 mg Bis-(trichlormethyl)-carbonat (Triphosgen) vorsichtig zugegeben und 10 Minuten bei RT gerührt. Dann wird mit 208,88 mg 3-(4-Methyl-1-piperazinyl)-1-propanol versetzt und 24 h bei RT im fest verschlossenen Reaktionsgläschen im multiplen Synthesegerät gerührt. Das Reaktionsgemisch wird mit Dichlormethan verdünnt, mit Wasser gewaschen, getrocknet, filtriert und das Lösungsmittel wird entfernt. Der Rückstand wird auf Kieselgel aufgezogen und über eine Flash-Säule am FlashMaster mit 20 g LiChroprep 60 (25-40µm) und Dichlormethan + 0-50% Methanol chromatographiert. Der Rückstand wird in Methanol gelöst, mit etherischer Salzsäure versetzt, mit Ether das Salz gefällt und die überstehende Lösung abgegossen. Das Salz wird mit Methanol/Ether kristallisiert, abgesaugt, mit Ether gewaschen und getrocknet; F. 120°, ab 150° Zersetzung; ESI: 455 (M+H); HPLC: Rt. = 4.00 (Methode A);
Ausbeute: 383 mg (61 %) "A1".

### Herstellung von [3-(5-Methyl-2-oxo-oxazolo[4,5-b]pyridin-3-ylmethyl)-phenyl]-carbaminsäure-3-(4-methyl-piperazin-1-yl)-propylester ("B1")

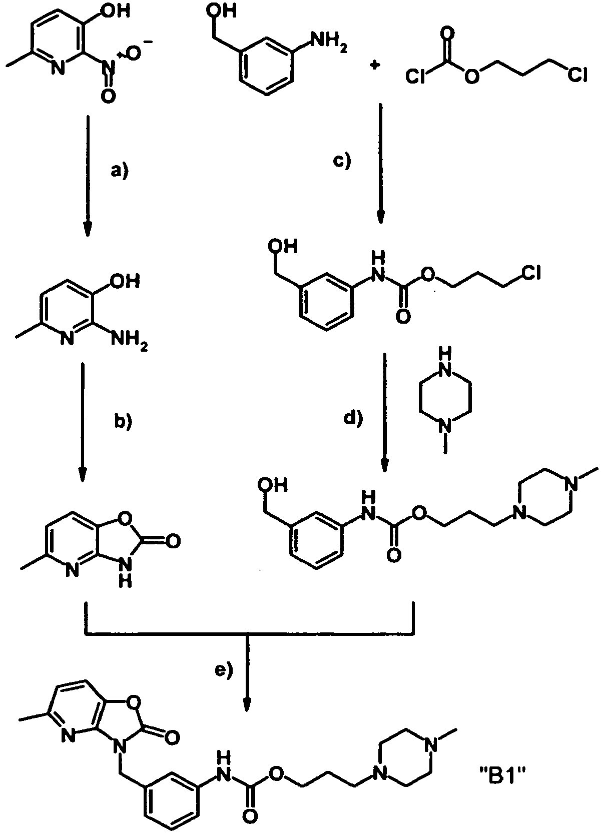

### Stufe a:

### Herstellung von 2-Amino-6-methyl-pyridin-3-ol:

Reduktion des 6-Methyl-2-nitro-pyridin-3-ol entsprechend Beispiel 1.1 liefert das gewünschte Produkt; ESI: 125 (M+H), Rt. = 0.51 min (Methode B).

### Stufe b:

### Herstellung von 5-Methyl-3H-oxazolo[4,5-b]pyridin-2-on:

Umsetzung des 2-Amino-6-methyl-pyridin-3-ols mit CDI (Carbonyldiimidazol) entsprecht dem Beispiel 1.2 liefert das gewünschte Produkt; ESI: 151 (M+H), Rt. = 1.58 min (Methode B).

### Stufe c:

### Herstellung von (3-Hydroxymethyl-phenyl)-carbaminsäure-3-chlorpropylester :

3.7 g (30 mmol) 3-Aminobenzylalkohol werden in 50 ml Aceton gelöst und mit 3.2 g (30 mmol) Natriumcarbonat versetzt. Zu dieser Suspension werden bei 25°C 5.7 g (36 mmol) 3-Chlorpropylchlorformiat zudosiert. Das Reaktionsgemisch wird 18 h bei Raumtemperatur nachgerührt. Dem Reaktionsgemisch wird zur Hydrolyse Wasser zugegeben, anschließend wird der Feststoff abfiltriert. Die Lösung wird destillativ aufkonzentriert, wobei sich das Produkt als Öl abscheidet. Die Produktphase wird abgetrennt. Die wässrige Phase wird mit Ethylacetat extrahiert. Die organischen Phasen werden vereinigt, mit Wasser gewaschen und über Natriumsulfat getrocknet. Anschließend wird zum Rückstand eingeengt. Das Rohprodukt wir ohne weitere Aufreinigung direkt weiter umgesetzt; ESI: 244 (M+H).

### Stufe d:

### Synthese von (3-Hydroxymethyl-phenyl)-carbaminsäure-3-(4-methylpiperazin-1-yl)-propylester:

Eine Lösung von 2.4 g (10 mmol) (3-Hydroxymethyl-phenyl)-carbaminsäure-3-chlorpropylester in 10 ml Acetonitril wird mit 10g (100 mmol) N-Methylpiperazin versetzt. Die Lösung wird 4 h lang refluxiert. Dann wird durch Zugabe von Wasser hydrolysiert, auf ca. 65 °C abgekühlt und mit Ethylacetat versetzt. Dann wird auf Raumtemperatur abgekühlt. Dabei fällt das Produkt als Feststoff zwischen organischer und wässriger Phase an. Das Produkt wird abfiltriert und mit Wasser, Acetonitril, sowie Ethylacetat nachgewaschen. Anschließend wird es bei 50 °C mehrere Stunden getrocknet; ESI: 308 (M+H).

### Stufe e:

### Herstellung von [3-(5-Methyl-2-oxo-oxazolo[4,5-b]pyridin-3-ylmethyl)-phenyl]-carbaminsäure-3-(4-methyl-piperazin-1-yl)-propylester:

98 mg (0.65 mmol) 5-Methyl-3H-oxazolo[4,5-b]pyridin-2-on, 200 mg (0.65 mmol) (3-Hydroxymethyl-phenyl)-carbaminsäure-3-(4-methyl-piperazin-1-yl)-propylester und 325 mg (0.98 mmol) polymergebundenes Triphenylphosphin (3mmol/g) werden in 5 ml DMF suspendiert und 30 min geschüttelt. Anschließend werden 229 mg (0.98 mmol) *Di-tert.-*butylazodicarboxylat zugegeben. Das Reaktionsgemisch wird bei Raumtemperatur geschüttelt. Das Reaktionsgemisch wird filtriert, mit THF nachgewaschen und das Filtrat eingedampft. Der Rückstand wird mittels Säulenchromatographie an Kieselgel aufgereinigt.
Produkt: 68 mg "B1"; ESI: 440 (M+H), Rt. = 2.11 min (Methode B); ¹H-NMR (DMSO-d₆, δ in ppm): 9.62 (1H, b); 7.64 (1H, d); 7.39-7.45 (2H, m); 7.26 (1 H, t); 7.04 (1 H, d); 7.00 (1 H, d); 4.96 (2H, s), 4.11 (2H, t); 2.77 (3H, s); 2,48-2,52 (überlagert, 10H, m); 2.48 (3H, s); 1.59 (2H, m).

### Herstellung von {3-[1-(5,6-Difluor-2-oxo-benzoxazol-3-yl)-ethyl]-phenyl}-carbaminsäure-2-(4-methyl-piperazin-1-yl)-ethylester ("A27")

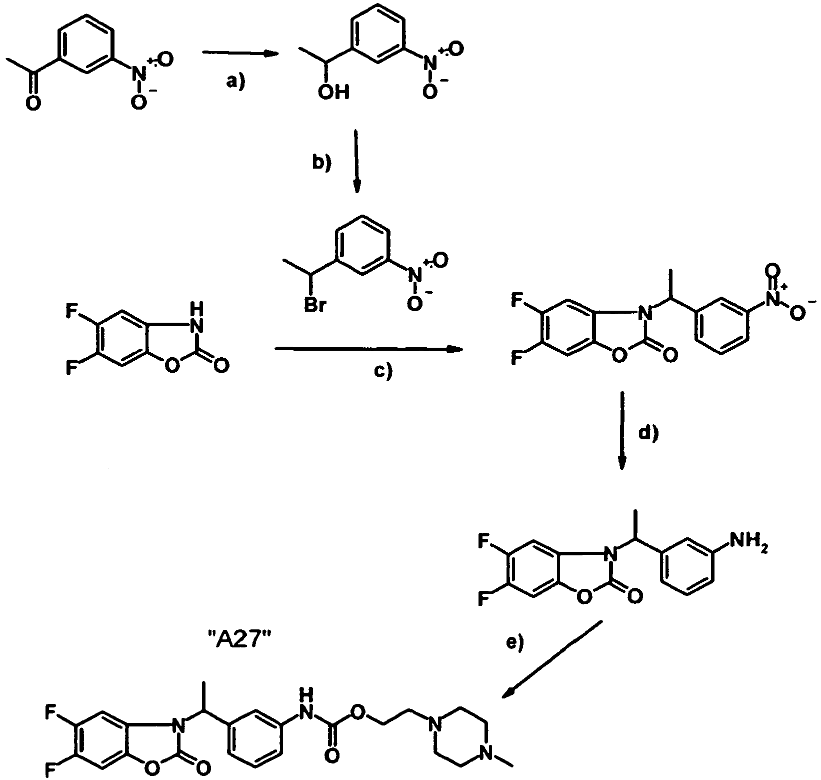

### Stufe a:

### Herstellung von 1-(3-Nitro-phenyl)-ethanol:

26.4 g (160 mmol) 1-(3-Nitro-phenyl)-ethanon werden in 270 ml Methanol suspendiert und unter Eiskühlung portionsweise mit 6.1 g (160 mmol) Natriumborhydrid versetzt. Anschließend wird das Reaktionsgemisch 3 h ohne Kühlung nachgerührt, mit 300 ml Dichlormethan verdünnt und mit 3 x 200 ml Wasser gewaschen. Die organische Phase wird über Natriumsulfat getrocknet und zum Rückstand eingedampft.
Produkt: 26.15 g; HPLC: Rt. = 3,87 Min (Methode A).

### Stufe b:

### Herstellung von 1-(1-Brom-ethyl)-3-nitro-benzol:

26.15 g (156 mmol) 1-(3-Nitro-phenyl)-ethanol werden in 130 ml Eisessig gelöst und unter Eiskühlung wurden 55 ml (313 mmol) 33% HBr in Eisessig zugetropft. Das Reaktionsgemisch wird 5 Tage bei Raumtemperatur gerührt. Anschließend wird mit 300 ml DCM verdünnt, mit 3 x 200 ml H₂O und 200 ml gesättigter NaHCO₃-Lösung gewaschen, über Natriumsulfat getrocknet und zum Rückstand eingeengt und aus Petrolether kristallisiert.
Produkt: 30.4 g; HPLC: Rt. = 5,39 Min (Methode A).

### Stufe c:

### Herstellung von 5,6-Difluor-3-[1-(3-nitro-phenyl)-ethyl]-3H-benzoxazol-2-on:

500 mg (2.9 mmol) 5,6-Difluor-3H-benzoxazol-2-on, 672 mg (2.9 mmol) (1-Brom-ethyl)-3-nitro-benzol und 1.58 g (11.4 mmol) Kaliumcarbonat werden in 6 ml Acetonitril suspendiert und 6 h bei 60°C gerührt. Anschließend wird mit 30 ml MTBE verdünnt, mit 3 x 20 ml H₂O gewaschen, über Natriumsulfat getrocknet und zum Rückstand eingeengt. Das Rohprodukt wird mittels Säulenchromatographie an Kieselgel aufgereinigt.
Produkt: 638 mg; ESI: 321 (M+H); HPLC: Rt. = 5,52 Min (Methode A).

### Stufe d:

### Herstellung von 3-[1-(3-Amino-phenyl)-ethyl]-5,6-difluor-3H-benzoxazol-2-on:

633 mg (1.98 mmol) 5,6-Difluor-3-[1-(3-nitro-phenyl)-ethyl]-3H-benzoxazol-2-on werden in 10 ml THF gelöst und mit 700 mg Raney-Nickel (wasserdass) unter Wasserstoffatmosphäre hydriert. Nach 24 h wird die Reaktionslösung filtriert, das Filtrat zum Rückstand eingedampft und aus Diethylether/Petrolether kristallisiert.
Produkt: 500 mg; ESI: 291 (M+H); HPLC: Rt. = 5,01 Min (Methode A).

### Stufe e:

### Herstellung von {3-[1-(5,6-Difluor-2-oxo-benzoxazol-3-yl)-ethyl]-phenyl}-carbaminsäure 2-(4-methyl-piperazin-1-yl)-ethylester:

250 mg (0.86 mmol) 3-[1-(3-Amino-phenyl)-ethyl]-5,6-difluor-3H-benzoxazol-2-on, 137 mg (0.95 mmol) 2-(4-Methyl-piperazin-1-yl)-ethanol und 200 µl (1.81 mmol) N-Methylmorpholin werden in 10 ml Dichlormethan suspendiert, 10 min bei Raumtemperatur gerührt und mit 128 mg (0.43 mmol) Bis(trichlormethyl)-carbonat versetzt. Das Reaktionsgemisch wird 16 h bei Raumtemperatur gerührt. Anschließend wird mit 30 ml Dichlormethan verdünnt, mit 2 x 20 ml gesättigter Natriumhydrogencarbonatlösung gewaschen, über Natriumsulfat getrocknet und zum Rückstand eingeengt. Das Rohprodukt wird mittels Säulenchromatographie an Kieselgel aufgereinigt, in Aceton gelöst, mit HCl in Ether erwärmt und nach beendeter Kristallisation abgesaugt und getrocknet.
Produkt ("A27"): 95 mg, Produkt liegt als Hydrochlorid vor;
F. 236-238°C (Zersetzung); ESI: 461; HPLC: Rt. = 4.21 Min (Methode A); ¹H-NMR (DMSO-d₆, δ in ppm): 9.801 (SB, 1H), 7.728 (DD, 1H), 7.475 (M, 2H), 7.324 (M, 2H), 7.128 (D, 1H), 5.543 (M, 1 H), 4.408 (SB, 2H), 4.023 - 3.110 (M, 10H), 2.809 (SB, 3H), 1.843 (D, 3H).

Analog erhält man die nachstehenden Verbindungen

| Nr. | Name und/oder Struktur | LCMS-Retentionszeit [min] / LCMS-Masse [M+H]⁺ / F. [°C] |
|---|---|---|
| "A6" | [3-(6-Chlor-2-oxo-benzoxazol-3-ylmethyl)-phenyl]-carbaminsäure-3-(4-methyl-piperazin-1-yl)-propylester | 1,673 / 459,8 /243 (Zersetzung) |
| "A7" | [3-(5-Methyl-2-oxo-benzoxazol-3-ylmethyl)-phenyl]-carbaminsäure-3-(4-methyl-piperazin-1-yl)-propylester | 1,629 / 438,8 /228 (Zersetzung) |
| "A8" | [3-(5-Acetyl-2-oxo-benzoxazol-3-ylmethyl)-phenyl]-carbaminsäure-3-(4-methyl-piperazin-1-yl)-propylester | 1.514/466.8 /174 (Zersetzung) |
| "A1" | [3-(2-Oxo-oxazolo[4,5-b]pyridin-3-ylmethyl)-phenyl]-carbaminsäure-3-dimethylamino-propylester | ESI: 371 (M+H), HPLC: Rt. = 3.53 min (Methode A) |
| | 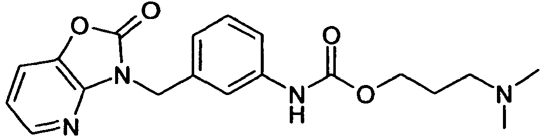 | |
| | Hydrochlorid | |
| "A16" | [3-(2-Oxo-benzoxazol-3-ylmethyl)-phenyl)-carbaminsäure-3-dimethylamino-propylester | 138-140 ESI: 370 (M+H), HPLC: Rt. = 3.89 min (Methode A) |
| "A17" | [3-(2-Oxo-benzoxazol-3-ylmethyl)-phenyl]-carbaminsäure-2-(4-methyl-piperazin-1-yl)-ethylester | 1,509 / 410,8 /237 (Zersetzung) ESI: 411 (M+H), HPLC: Rt. = 3.87 min (Methode A) |
| | 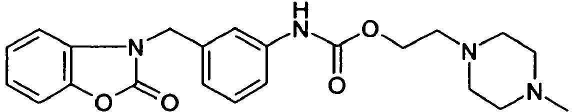 | |
| | Hydrochlorid | |
| ¹H NMR (250 MHz, DMSO-d₆) δ [ppm] 9.757 (s, 1 H), 7.452 (sb, 1 H), 7.419 (d, 1 H), 7.370 (d, 1 H), 7.286 (t, 1H), 7.185-7.129 (m, 3H), 7.053 (d, 1 H), 5.011 (s, 2H), 4.343 (t, 2H), 3.803-3.403 (m, 10H), 2.794 (s, 3H) | | |
| "A18" | [3-(2-Oxo-benzoxazol-3-ylmethyl)-phenyl]-carbaminsäure-3-(4-methyl-piperazin-1-yl)-propylester | 1,524 / 424,8 205 (Zersetzung) ESI: 425 (M+H), HPLC: Rt. = 3.92 min (Methode A) |
| ¹H NMR (250 MHz, DMSO-d₆) δ [ppm] 9.676 (s, 1 H), 7.453 (sb, 1 H), 7.402 (d, 1H), 7.370 (d, 1 H), 7.272 (t, 1 H), 7.167-7.130 (m, 3H), 7.035 (d, 1 H), 5.004 (s, 2H), 4.122 (t, 2H), 3.456 (m, 10H), 2.795 (s, 3H), 2.006 (t, 2H) | | |
| "A19" | [3-(5,6-Difluor-2-oxo-benzoxazol-3-ylmethyl)-phenyl]-carbaminsäure-3-(4-methyl-piperazin-1-yl)-propylester | 1,609 / 460,8 /264-265 (Zersetzung) ESI: 461 (M+H), HPLC: Rt. = 4.13 min (Methode A) |
| "A20" | [3-(6-Methoxy-2-oxo-benzoxazol-3-ylmethyl)-phenyl]-carbaminsäure-3-(4-methyl-piperazin-1-yl)-propylester | 1,561 / 454488 /201 (Zersetzung) ESI: 455 (M+H), HPLC: Rt. = 4.00 min (Methode A) |
| "A21" | [3-(6-Methyl-2-oxo-benzoxazol-3-ylmethyl)-phenyl]-carbaminsäure-3-(4-methyl-piperazin-1-yl)-propylester | 1,631/438.8/196 (Zersetzung) ESI: 439 (M+H), HPLC: Rt. = 4.16 min (Methode A) |
| "A22" | [3-(6-Acetyl-2-oxo-benzoxazol-3-ylmethyl)-phenyl]-carbaminsäure-3-(4-methyl-piperazin-1-yl)-propylester | 1,497 1466,8 /234 (Zersetzung) ESI: 467 (M+H), HPLC: Rt. = 3.84 min (Methode A) |
| "A23" | {3-[5-(4-Methyl-piperazin-1-yl)-2-oxo-benzoxazol-3-ylmethyl]-phenyl}-carbaminsäure-ethylester | 1,598/411,2/166-170 (Zersetzung) ESI: 411 (M+H), HPLC: Rt. = 3.81 min (Methode A) |
| | 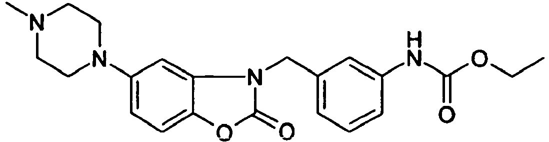 | |
| | Hydrochlorid | |
| "A24" | [3-(5-Cyan-2-oxo-benzoxazol-3-ylmethyl)-phenyl]-carbaminsäure-3-(4-methyl-piperazin-1-yl)-propylester, Hydrochlorid | 1.514/450.2 /212-214 ESI: 450 (M+H), HPLC: Rt. = 3.89 min (Methode A) |
| "A25" | [3-(5-Ethylsulfonyl-2-oxo-benzoxazol-3-ylmethyl)-phenyl]-carbaminsäure-3-(4-methyl-piperazin-1-yl)-propylester, Dihydrochlorid | 1,482/517,2/244 (Zersetzung) ESI: 517 (M+H), HPLC: Rt. = 3.79 min (Methode A) |
| "A26" | {3-[1-(5,6-Difluor-2-oxo-benzoxazol-3-yl)-ethyl]-phenyl}-carbaminsäure-3-(4-methyl-piperazin-1-yl)-propylester | 1,659/475,2/ 00-201 ESI: 475 (M+H), HPLC: Rt = 4.24 min (Methode A) |
| | 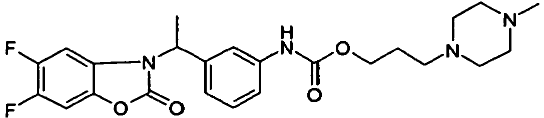 | |
| | Hydrochlorid | |
| ¹H NMR (250 MHz, DMSO-d₆) δ [ppm] 9.715 (s, 1H), 7.730 (dd, 1H), 7.489 (s, 1H), 7.451 (d, 1H), 7.340-7.286 (m, 2H), 7.106 (d, 1H), 5.532 (q, 1 H), 4.140 (t, 2H), 3.71-3,05 (m, 10H), 2.816 (s, 3H), 2.086 (sb, 2H), 1.839 (d, 3H) | | |
| "A28a" | 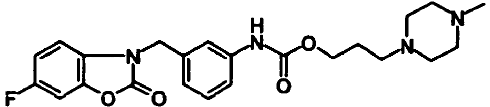 | 4,03 (Methode A) / 443 |
| | Hydrochlorid | |
| "A28b" | 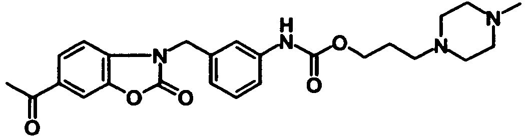 | 3,84 (Methode A) / 467 |
| | Hydrochlorid | |

### Beispiel 2

### Die Herstellung von {3-[6-(2-Dimethylamino-ethylcarbamoyl)-2-oxo-benzoxazol-3-ylmethyl]-phenyl}-carbaminsäure-ethylester ("A29") erfolgt analog nachstehendem Schema

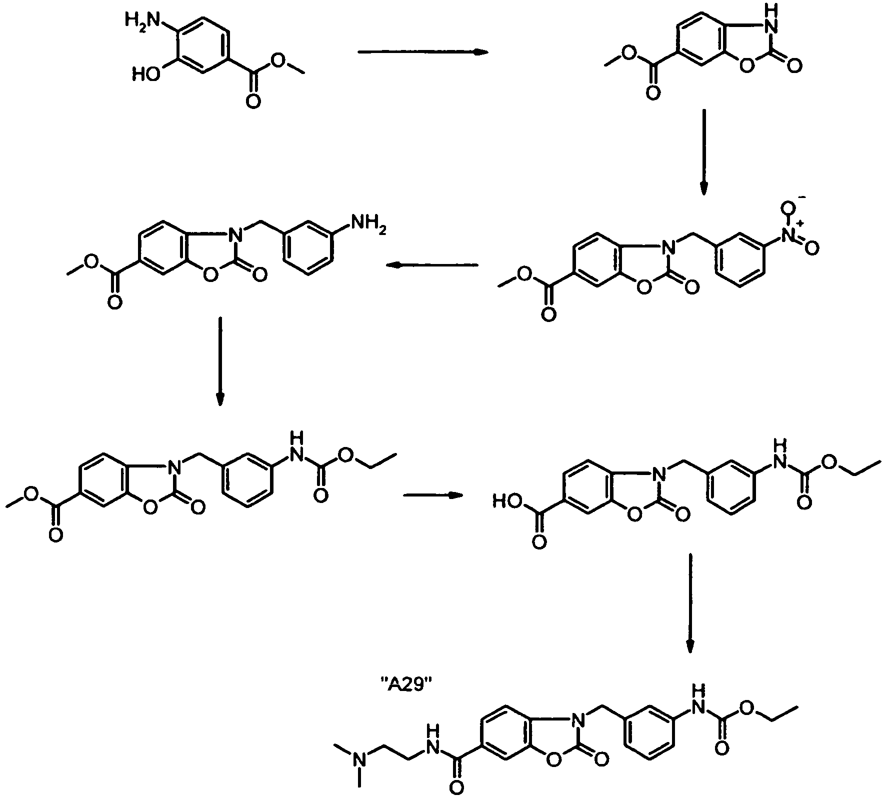

2.1 In einem 250 ml Einhalskolben mit Rückflusskühler und Trockenröhrchen werden 9,36 g (0,056 mol) 3-Hydroxy-4-Aminobenzoesäure-methylester und 9,85 g 1,1'-Carbonyldimidazol in 125 ml THF gelöst und 3 h refluxiert. Zur Aufarbeitung wird das Lösungsmittel am Rotationsverdampfer entfernt, der Rückstand in Dichlormethan aufgenommen und 3x mit 1 N HCl und 1x mit Wasser gewaschen. Die organische Phase wird über Natriumsulfat getrocknet und am Rotationsverdampfer zum Rückstand eingeengt; Ausbeute 9,92 g (92 %) 2-Oxo-2,3-dihydro-benzoxazol-6-carbonsäure-methylester; ESI: 194 (M+H); HPLC: Rt. = 2.57 min (Methode B).

2.2 1 g (5.2 mmol) der Substanz 2-Oxo-2,3-dihydro-benzoxazol-6-carbonsäure-methylester werden in 20 ml Acetonitril gelöst, mit 2.8 g (20.3 mmol) Kaliumcarbonat und 1.24 g ( 5.7 mmol) m-Nitrobenzylbromid versetzt und 16 h unter Rückfluss erhitzt. Das Reaktionsgemisch wird nach dem Abkühlen mit 30 ml Dichlormethan versetzt und mit 2 x 20 ml Wasser extrahiert, die organische Phase über Natriumsulfat getrocknet und das Lösungsmittel abdestilliert. Der Rückstand wird in Methanol angeschlämmt, abgesaugt und mit Diethylether gewaschen. Die Substanz wird ohne weitere Aufreinigung weiter umgesetzt; Ausbeute: 1.15 g (67 %) 3-(3-Nitrobenzyl)-2-oxo-2,3-dihydro-benzoxazol-6-carbonsäure-methylester; F. 149-151°C; ESI: 329 (M+H); HPLC: Rt. = 5.12 min (Methode A).

2.3 594 mg (1.8 mmol) 3-(3-Nitrobenzyl)-2-oxo-2,3-dihydro-benzoxazol-6-carbonsäure-methylester werden in 10 ml Methanol gelöst und mit 0.6 g Raney-Nickel unter Wasserstoffatmosphäre gerührt. Nach wenigen Stunden wird die Bildung eines Niederschlages beobachtet, daher werden 10 ml THF zugegeben und es wird weiter unter Wasserstoffatmosphäre hydriert. Nach 16 h wird die Reaktion gestoppt, der Katalysator abgesaugt und mit Methanoi/THF nachgewaschen. Der Rückstand wird eingedampft; Ausbeute: 562 mg 3-(3-Aminobenzyl)-2-oxo-2,3-dihydro-benzoxazol-6-carbonsäure-methylester. Die Substanz wird ohne weitere Aufreinigung weiter umgesetzt; ESI: 299 (M+H); HPLC: Rt. = 2.07 min (Methode B).

2.4 In einem Rundkolben werden 562 mg (1.88 mmol) 3-(3-Amino-benzyl)-2-oxo-2,3-dihydro-benzoxazol-6-carbonsäure-methylester in 20 ml Dichlormethan gelöst, mit 152 µL (1.88 mmol) Pyridin versetzt und unter Kaltwasserkühlung 183 µl (1.88 mmol) Ethylchloroformiat zugetropft. Es wird 1 Stunde bei RT nachgerührt. Dabei fallen feine Kristalle aus. Das Reaktionsgemisch wird mit weiteren 40 ml DCM versetzt und mit 20 ml 1 N HCl gewaschen. Die organische Phase wird mit 20 ml Wasser neutral gewaschen, über Natriumsulfat getrocknet und das Lösungsmittel abdestilliert; Ausbeute: 539 mg (77%) 3-(3-Ethoxycarbonylamino-benzyl)-2-oxo-2,3-dihydro-benzoxazol-6-carbonsäure-methylester. Die Substanz wird ohne weitere Aufreinigung weiter umgesetzt; ESI: 371 (M+H); HPLC: Rt. = 2.89 min (Methode B).

2.5 In einem 50-ml Rundkolben werden 517 mg (1.4 mmol) 3-(3-Ethoxycarbonylamino-benzyl)-2-oxo-2,3-dihydro-benzoxazol-6-carbonsäure-methylester mit 10 ml Wasser und 10 ml konz. HCl versetzt. Die Suspension wird 4 Stunden refluxiert. Es werden weitere 10 ml konz. HCl zugegeben und 16 h refluxiert. Es werden noch 2 mal je 10 ml konz. HCl zugegeben und jeweils weitere 16 h refluxiert.

Das Gemisch wird auf Raumtemperatur abgekühlt und der Niederschlag wird abgesaugt und mit Wasser gut gewaschen; Ausbeute: 417 mg (84 %) 3-(3-Ethoxycarbonylamino-benzyl)-2-oxo-2,3-dihydro-benzoxazol-6-carbonsäure. Die Substanz wird ohne weitere Aufreinigung weiter umgesetzt; ESI: 357 (M+H); HPLC: Rt. = 2.54 min (Methode B).

2.6 100 mg (0,28 mmol) 3-(3-Ethoxycarbonylamino-benzyl)-2-oxo-2,3-dihydro-benzoxazol-6-carbonsäure werden in 2 ml DMF gelöst und mit 109 mg (0.56 mmol) EDCI, 39 mg (0.56 mmol) HOBt und 63 µL (0.56 mmol) N-Methylmorpholin versetzt. Anschließend werden 37 µl (0.34 mmol) 2-Dimethylaminoethylamin zugegeben und die Reaktionslösung wird 3 Tage bei Raumtemperatur gerührt.

Das Reaktionsgemisch wird mittels präparativer HPLC aufgereinigt und die sauberen Fraktionen gefriergetrocknet.

Ausbeute: 89 mg (59 %) "A29" TFA-Salz; HPLC: RT= 2,175 min (Methods B); LC-MS: [M+H]⁺ = 427 bei RT= 1,431 min.

Die Herstellung von 2-Oxo-3-(2-oxo-2,3-dihydro-1H-benzoimidazol-5-ylmethyl)-2,3-dihydro-benzoxazol-5-carbonsäure-(2-dimethylamino-ethyl)-amid ("A35") erfolgt analog nachstehendem Schema

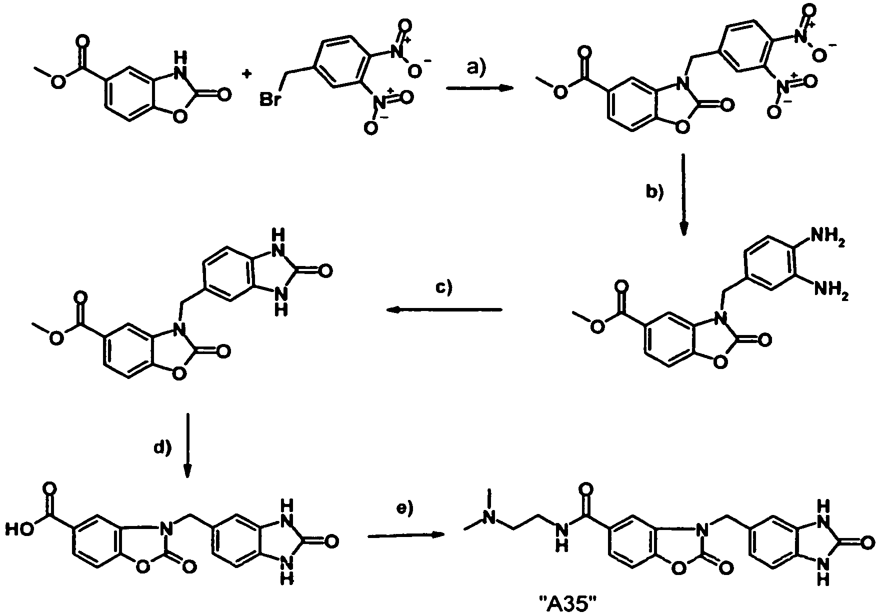

### Stufe a)

### Herstellung von 3-(3,4-Dinitro-benzyl)-2-oxo-2,3-dihydro-benzoxazol-5-carbonsäuremethylester:

2 g (10.4 mmol) 2-Oxo-2,3-dihydro-benzoxazol-5-carbonsäuremethylester, 2.97 g (11.4 mmol) 4-Bromomethyl-1,2-dinitro-benzol (hergestellt entsprechend DE 3904797) und 5,7 g (41.4 mmol) Kaliumcarbonat werden in 50 ml Acetonitril suspendiert und 1 h bei 80°C gerührt. Das Reaktionsgemisch wird auf 50 ml Wasser gegossen, mit 500 ml MTBE extrahiert, getrocknet und zum Rückstand eingeengt. Der Rückstand wird mittels Säulenchromatographie an Kieselgel aufgereinigt; Produkt: 1.5 g; ESI: 374 (M+H).

### Stufe b:

### Herstellung von 3-(3,4-Diamino-benzyl)-2-oxo-2,3-dihydro-benzoxazol-5-carbonsäuremethylester:

1.45 g (3.8 mmol) 3-(3,4-Dinitro-benzyl)-2-oxo-2,3-dihydro-benzoxazol-5-carbonsäuremethylester werden in 20 ml THF gelöst und mit 1 g Raney-Nickel (wassernass) unter Wasserstoffatmosphäre hydriert. Nach 24 h wird die Reaktionslösung filtriert und das Filtrat zum Rückstand eingedampft; Produkt: 1.1 g, ESI: 314 (M+H).

### Stufe c:

### Herstellung von 2-Oxo-3-(2-oxo-2,3-dihydro-1 H-benzimidazol-5-ylmethyl)-2,3-dihydro-benzoxazol-5-carbonsäuremethylester:

1.1 g (3.5 mmol) 3-(3,4-Diamino-benzyl)-2-oxo-2,3-dihydro-benzoxazol-5-carbonsäuremethylester und 626 mg (3,9 mmol) 1,1'-Carbonyldiimidazol werden in 10 ml THF 24 h bei Raumtemperatur gerührt. Das Reaktionsgemisch wird auf 150 ml Wasser gegeben, der entstandene Niederschlag wird abgesaugt und im Vakuum getrocknet; Produkt: 1.1 g; ESI: 340 (M+H).

### Stufe d:

### Herstellung von 2-Oxo-3-(2-oxo-2,3-dihydro-1 H-benzimidazol-5-ylmethyl)-2,3-dihydro-benzoxazol-5-carbonsäure:

1.1 g (3.24 mmol) 2-Oxo-3-(2-oxo-2,3-dihydro-1 H-benzimidazol-5-ylmethyl)-2,3-dihydro-benzoxazol-5-carbonsäuremethylester werden in 20 ml Wasser suspendiert, mit 30 ml konz. Salzsäure versetzt und 24 h bei 100°C Badtemperatur gerührt. Es werden weitere 20 ml konz. HCl zugegeben und das Reaktionsgemisch 3 Tage bei 130 °C Badtemperatur gerührt. Die Suspension wird filtriert, mit Wasser gewaschen und der Rückstand im Trockenschrank bei 50 °C getrocknet; Produkt: 996 mg; F. 230-231°C; ESI 326; HPLC: Rt. = 4.24 min (Methode A).

### Stufe e:

### Herstellung von 2-Oxo-3-(2-oxo-2,3-dihydro-1 H-benzimidazol-5-ylmethyl)-2,3-dihydro-benzoxazol-5-carbonsäure-(2-dimethylamino-ethyl)-amid:

330 mg (0.76 mmol) 2-Oxo-3-(2-oxo-2,3-dihydro-1H-benzimidazol-5-ylmethyl)-2,3-dihydro-benzoxazol-5-carbonsäure werden in 3 ml DMF gelöst und mit 294 mg (1.52 mmol) EDCI, 106 mg (0.76 mmol) HOBt und 157 µL (1.52 mmol) N-Methylmorpholin versetzt. Anschließend werden 81 mg (0.91 mmol) 2-Dimethylaminoethylamin zugegeben und die Reaktionslösung wird 3 Tage bei Raumtemperatur gerührt. Anschließend wird das Reaktionsgemisch auf Wasser gegeben und mit Dichlormethan extrahiert. Die organische Phase wird über Natriumsulfat getrocknet, zum Rückstand eingedampft und mittels Säulenchromatographie an Kieselgel aufgereinigt. Das Produkt wird nochmals mittels präparativer HPLC aufgereinigt; 100 mg "A35" Trifluormethylacetat; ESI 397 (M+H), HPLC: Rt.= 3,89 min (Methode A).

Analog erhält man die nachstehenden Verbindungen

| Nr. | Name und/oder Struktur | LCMS-Retentionszeit [min] / LCMS-Masse [M+H]⁺ /F. [°C] |
|---|---|---|
| "A3" | [3-(5-Dimethylcarbamoyl-2-oxo-benzoxazol-3-ylmethyl)-phenyl]-carbaminsäure-3-(4-methylpiperazin-1-yl)-propylester | 1,435 / 496,2 HPLC: Rt. = 3.60 min (Methode A) |
| | 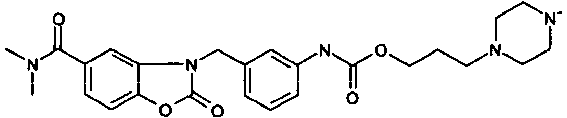 | |
| | Trifluoracetat | |
| "A4" | [3-(5-Dimethylcarbamoyl-2-oxo-benzoxazol-3-ylmethyl)-phenyl]-carbaminsäure-3-piperazin-1-yl-propylester | 1,432 / 482,2 HPLC: Rt. = 3.57 min (Methode A) |
| "A5" | [3-(5-Propylcarbamoyl-2-oxo-benzoxazol-3-ylmethyl)-phenyl]-carbaminsäure-3-(4-methylpiperazin-1-yl)-propylester | 1,520 / 510,2 HPLC: Rt. = 3.97 min (Methode A) |
| "A10" | [3-(5-Propylcarbamoyl-2-oxo-benzoxazol-3-ylmethyl)-phenyl]-carbaminsäure-3-piperazin-1-yl-propylester | 245-253 (Zersetzung) ESI: 496 (M+H), HPLC: Rt. = 4.03 min (Methode A) |
| "A12" | [3-(5-Dimethylcarbamoyl-2-oxo-benzoxazol-3-ylmethyl)-phenyl]-carbaminsäure-3-dimethylamino-propylester | 86-93 ESI: 441 (M+H), HPLC: Rt. = 3.65 min (Methode A) |
| "A13" | [3-(5-Propylcarbamoyl-2-oxo-benzoxazol-3-ylmethyl)-phenyl]-carbaminsäure-3-dimethylamino-propylester | 85-91 ESI: 455 (M+H), HPLC: Rt. = 3.92 min (Methode A) |
| ¹H NMR (250 MHz, DMSO-d₆) δ [ppm] 9.624 (s, 1 H), 8.415 (t, 1 H), 7.679 (dd, 1 H), 7.627 (s, 1 H), 7.464-7.447 (m, 2H), 7.404 (d, 1 H), 7.270 (t, 1 H), 6.998 (d, 1 H), 5.035 (s, 2H), 4.066 (t, 2H), 3.199 (m, 2H), 2.275 (t, 2H), 2.118 (s, 6H), 1.719 (m, 2H), 1.516 (m, 2H), 0.873 (t, 3H) | | |
| "A14" | [3-(6-Methoxycarbonyl-2-oxo-benzoxazol-3-ylmethyl)-phenyl]-carbaminsäure-3-dimethylamino-propylester | 149-151 ESI: 428 (M+H), HPLC: Rt. = 4.03 min (Methode A) |
| "A15" | [3-(5-Methoxycarbonyl-2-oxo-benzoxazol-3-ylmethyl)-phenyl]-carbaminsäure-3-dimethylamino-propylester | ESI: 428 (M+H), HPLC: Rt. = 3.97 min (Methode A) |
| "A30" | {3-[5-(2-Dimethylamino-ethylcarbamoyl)-2-oxo-benzoxazol-3-ylmethyl]-phenyl}-carbaminsäureethylester | 1,586 / 427,2 / 160-161 ESI: 427 (M+H), HPLC: Rt. = 3.73 min (Methode A) |
| "A31" | {3-[5-(3-Dimethylamino-propylcarbamoyl)-2-oxo-benzoxazol-3-ylmethyl]-phenyl}-carbaminsäureethylester | 1,602 1441,2 / 116-119 ESI: 441 (M+H), HPLC: Rt. = 3.76 min (Methode A) |
| "A32" | (3-{1-[5-(4-Dimethylamino-butylcarbamoyl)-2-oxo-benzoxazol-3-yl]-ethyl}-phenyl)-carbaminsäure-ethylester | 1,661 / 469,2 ESI: 469 (M+H), HPLC: Rt. = 3.92 min (Methode A) |
| | 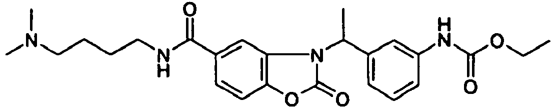 | |
| "A33" | (3-{1-[5-(2-Dimethylamino-ethylcarbamoyl)-2-oxo-benzoxazol-3-yl]-ethyl}-phenyl)-carbaminsäure-ethylester | 1,644 / 441,2 ESI: 441 (M+H), HPLC: Rt. = 3.81 min (Methode A) |
| "A34" | 2-Oxo-3-(2-oxo-2,3-dihydro-1H-benzimidazol-5-ylmethyl)-2,3-dihydro-benzoxazol-5-carbonsäure-(4-dimethylamino-butyl)-amid | 1,314/424,2 ESI: 424 (M+H), HPLC-MS: Rt. = 1.31 min |
| | 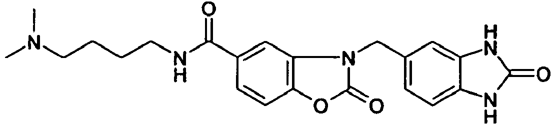 | |
| | Trifluoracetat | |
| "A36" | (3-{1-[5-(3-Dimethylamino-propylcarbamoyl)-2-oxo-benzoxazol-3-yl]-ethyl}-phenyl)-carbaminsäure-ethylester | 1,655 /455,2 /112-119 / ESI: 455 (M+H), HPLC: Rt. = 3.89 min (Methode A) |
| "A37" | 3-(3-Ethoxycarbonylamino-benzyl)-2-oxo-2,3-dihydro-benzoxazol-6-carbonsäure-methylester | ESI: 371 (M+H), HPLC: Rt. = 2.89 min (Methode B) |
| | 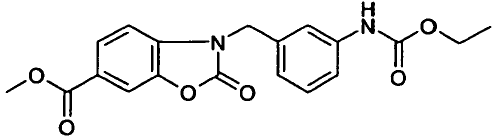 | |
| "A38" | {3-[6-(3-Methylamino-propylcarbamoyl)-2-oxo-benzoxazol-3-ylmethyl]-phenyl}carbaminsäure-ethylester, Trifluoracetat | 2.168/427 (bei RT = 1.475) |
| | (erhältlich aus "A40" mit TFA in DCM) | ESI: 427 (M+H), HPLC: Rt. = 2.17 min (Methode B) |
| "A39" | {3-[6-(3-Dimethylamino-propylcarbamoyl)-2-oxo-benzoxazol-3-ylmethyl]-phenyl}-carbaminsäure-ethylester, Trifluoracetat | 2.186 /441 (bei RT = 1.463 min) ESI: 441 (M+H), HPLC: Rt. = 2.19 min (Methode B) |
| "A40" | (3-{6-[3-(tert.-Butoxycarbonyl-methyl-amino)-propylcarbamoyl]-2-oxo-benzoxazol-3-ylmethyl}-phenyl)-carbaminsäure-ethylester | 2.984 /527 (bei RT = 2.235 min) ESI: 527 (M+H), HPLC: Rt. = 2.98 min (Methode B) |
| | 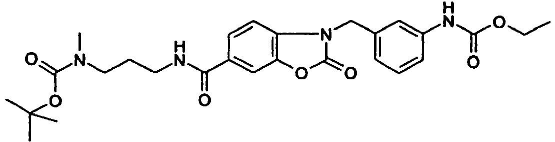 | |
| "A53" | 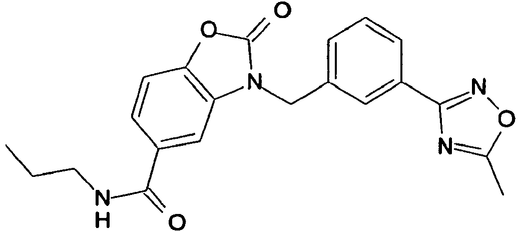 | |

### Beispiel 3

Die Herstellung von (3-{6-[2-(4-Methyl-piperazin-1-yl)-acetyl]-2-oxo-benzoxazol-3-ylmethyl}-phenyl)carbaminsäure-ethylester ("A41") erfolgt analog nachstehendem Schema

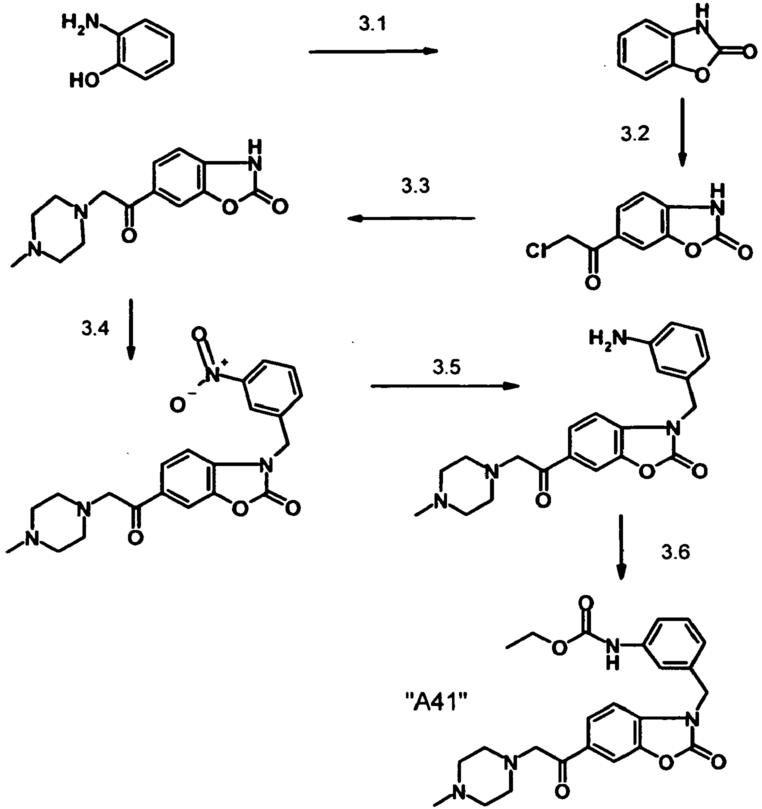

3.3 1.6 ml (14.2 mmol) 1-Methylpiperazin wurden in 50 ml Ethanol vorgelegt, mit 4.3 ml (31.2 mmol) Triethylamin versetzt und unter Rühren bei Raumtemperatur werden 3 g (14.2 mmol) 6-Chloracetyl-2-benzoxazolinon zugegeben. Das Reaktionsgemisch wird über Nacht bei Raumtemperatur gerührt. Es werden nochmal 1 ml (8.9 mmol) 1-Methylpiperazin zugegeben, 15 h bei RT und anschließend 24 h bei 70°C gerührt. Nach dem Abkühlen werden die ausgefallenen Kristalle abgesaugt, mit Methanol gewaschen und getrocknet;
Ausbeute: 0,8 g 6-[2-(4-Methyl-piperazin-1-yl)-acetyl]-3H-benzoxazol-2-on; HPLC: Rt= 1,263 min. (Methode B); LC-MS: M+H = 276 g/mol.

### Beispiel 4

Die Herstellung von 3-[3-(5-Methyl-[1,2,4]oxadiazol-3-yl)-benzyl]-6-[2-(4= methyl-piperazin-1-yl)-acetyl]-3H-benzoxazol-2-on ("A42") erfolgt analog nachstehendem Schema

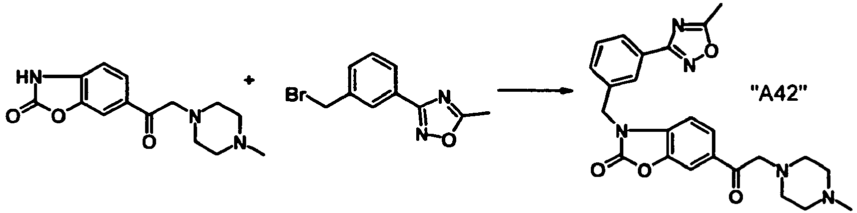

309 mg (0.72 mmol) 6-[2-(4-Methyl-piperazin-1-yl)-acetyl]-3H-benzoxazol-2-on und 387 mg (2.8 mmol) Kaliumcarbonat werden in 10 ml Acetonitril suspendiert und mit 200 mg (0.79 mmol) 3-[3-(Brommethyl)phenyl]-5-methyl-1,2,4-oxadiazol versetzt. Das Reaktionsgemisch wird 5 Tage bei 100° gerührt. Nach dem Abkühlen wird filtriert und das Filtrat eingeengt. Der Rückstand wird mittels präparativer HPLC aufgereinigt.
Ausbeute: 42,1 mg (10%) "A42" TFA-Salz; HPLC: Rt= 2,003 min.
(Methode B); LC-MS: [M+H]⁺ = 448 bei Rt= 1,282 min.

### Beispiel 5

Die Herstellung von 3-[3-(5-Methyl-[1,2,4]oxadiazol-3-yl)-benzyl]-6-[3-(4-methyl-piperazin-1-yl)-propionyl]-3H-benzoxazol-2-on ("A43") erfolgt analog nachstehendem Schema

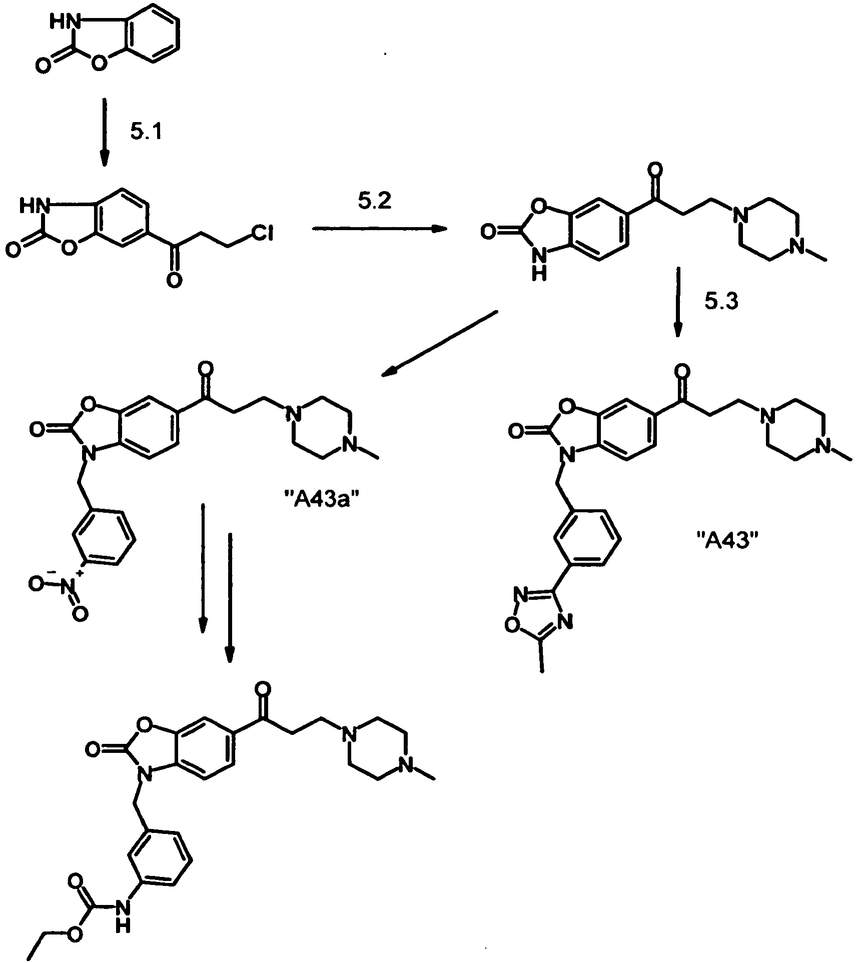

5.1 In einem 1 l Dreihalskolben mit Rührer, Kühler, Thermometer und Tropftrichter werden 95.2 g (0.7 mol) Aluminiumchlorid vorgelegt und unter Rühren 27 g (0.2 mol) Benzoxazolon [Herstellung analog Beispiel 2.1] zugegeben, wobei nach kurzer Zeit ein rührfähiger, dunkelbrauner Brei entsteht (schwach exotherme Reaktion). Es werden 5 Minuten nachgerührt und dann 29,7 ml (0.3 mol) 3-Chlorpropionylchlorid langsam zugetropft. Anschließend wird 2 h bei 80° gerührt. Nach dem Abkühlen wird mit 100 ml Dichlormethan verdünnt, 15 Minuten gerührt und die Reaktionslösung in 500 g Eis eingerührt. Der Niederschlag wird abgesaugt, mit wenig Dichlormethan und dann mit Wasser gewaschen und getrocknet. Das Rohkristallisat (41,2 g) wird in 100 ml Isopropanol suspendiert, abgesaugt, mit 50 ml Isopropanol und dann mit MTB-Ether gewaschen und getrocknet; Ausbeute: 34.1 g (76%) 6-(3-Chlor-propionyl)-3H-benzoxazol-2-on.

5.2 2.95 ml (26.6 mmol) 1-Methylpiperazin, 4 g (29.2 mmol) Kaliumcarbonat und 44 g (266 mmol) Kaliumiodid werden in 70 DMF vorgelegt, mit 6 g (26.6 mmol) 6-(3-Chlor-propionyl)-3H-benzoxazol-2-on versetzt und bei RT über Nacht gerührt. Das Reaktionsgemisch wird abgesaugt und mehrmals mit THF gewaschen. Der Rückstand wird in Natriumhydrogencarbonatlösung gelöst, die wässrige Phase wird mit NaCl versetzt und zweimal mit je 250 ml Ethylacetat extrahiert. Die organische Phase wird über Natriumsulfat getrocknet und einrotiert; Ausbeute: 640 mg 6-[3-(4-Methyl-piperazin-1-yl)-propionyl]-3H-benzoxazol-2-on.

5.3 148 mg (0.45 mmol) 6-[3-(4-Methyl-piperazin-1-yl)-propionyl]-3H-benzoxazol-2-on und 243 mg (1.76 mmol) Kaliumcarbonat werden in 10 ml Acetonitril suspendiert und mit 126 mg (0.50 mmol) 3-[3-(Brommethyl)-phenyl]-5-methyl-1,2,4-oxadiazol versetzt. Das Reaktionsgemisch wird 5 Tage bei 80° gerührt. Nach dem Abkühlen wird filtriert und das Filtrat eingeengt. Der Rückstand wird mittels präparativer HPLC aufgereinigt. Ausbeute: 42,2 mg (16%) "A43" TFA-Salz; HPLC: Rt= 2,902 min. (Methode B); LC-MS: M+H= 462 bei Rt= 1,251 min.

Entsprechend dem vorher beschriebenen Reaktionsschema erhält man

| Nr. | Struktur | ESI (M+H) | Rt. in min |
|---|---|---|---|
| "A43a" | 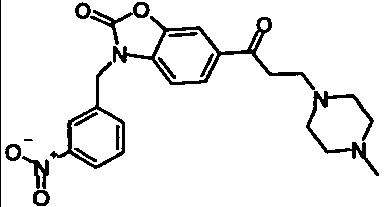 | 425 | 1.81 (Methode B) |
| | Trifluoracetat | | |

### Beispiel 6

Herstellung von N,N-Dimethyl-N'-{2-[3-(5-methyl-2-oxo-benzooxazol-3-ylmethyl)-phenyl]-pyrimidin-5-yl}-formamidin ("A44"): 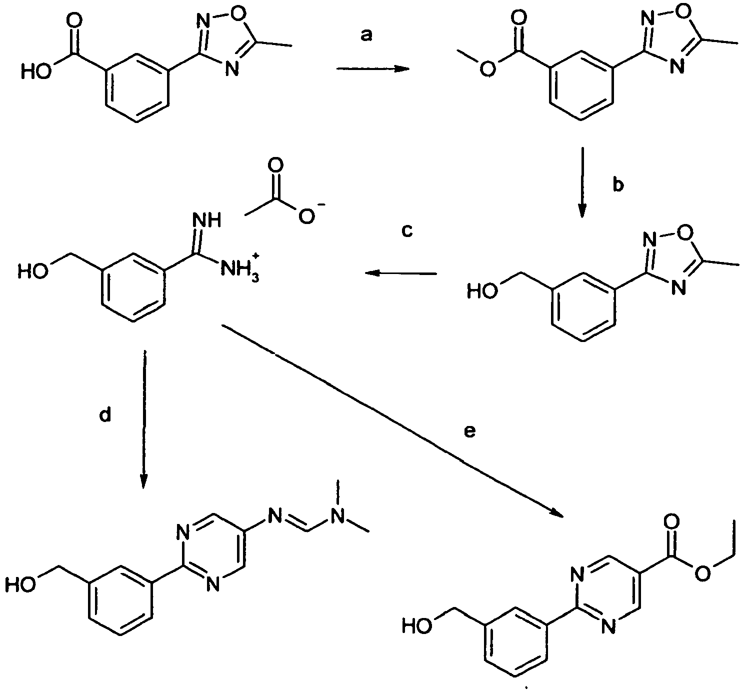 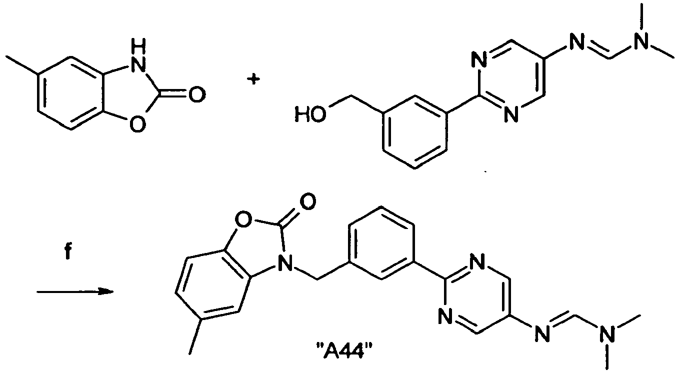
a) 3,781 g 3-(5-Methyl-[1,2,4]oxadiazol-3-yl)-benzoic acid (18,58 mmol) werden in 100 ml absolutem Methanol suspendiert, unter Eis / H₂O-Kühlung und Rühren 2,694 ml Thionylchlorid (37,13 mmol) zugetropft und 72 h ohne Kühlung nachgerührt, wobei eine klare Lsg. entsteht. Das Lösungsmittel wird entfernt, der Rückstand in 100 ml Dichlormethan gelöst, mit 50 ml gesättigter NaHCO₃-Lsg geschüttelt, über Natriumsulfat getrocknet, zum Rückstand abgezogen und aus Diethylether / Petrolether kristallisiert.
   Ausbeute: 3,46 g (15,86 mmol) = 85 % 3-(5-Methyl-[1,2,4]oxadiazol-3-yl)-benzoesäure-methylester; F. 81-82°; ESI 219 (M+H), HPLC: Rt. = 2.65 min (Methode B).
b) In einem 250 ml Dreihalskolben weurden 3,46 g 3-(5-Methyl-[1,2,4]oxadiazol-3-yl)-benzoesäure-methylester (15,86 mmol) in absolutem THF gelöst, unter Eis / H₂O-Kühlung und Rühren portionsweise 0,691 g LiBH₄ (31,71 mmol) eingetragen und 20 h ohne Kühlung nachgerührt. Aufarbeitung: Es wird unter Rühren durch langsames Zutropfen von 1 N HCl (starkes Schäumen) auf pH 7 eingestellt, mit 100 ml H₂O verdünnt, mit 3 x 50 ml Dichlormethan geschüttelt, die vereinigten Extrakte mit 100 ml H₂O gewaschen, über Natriumsulfat getrocknet, zum Rückstand abgezogen und durch Chromatographie gereinigt.
   Der rohe Chromatographierückstand wird aus Diethylether/Petroether umkristallisiert.
   Ausbeute: 1,643 g (8,64 mmol) = 54 % [3-(5-Methyl-[1,2,4]oxadiazol-3-yl)-phenyl]-methanol; F. 57 - 58°; ESI 191 (M+H); HPLC: Rt. = 2.88 min (Methode C).
c) 800 mg [3-(5-Methyl-[1,2,4]oxadiazol-3-yl)-phenyl]-methanol (4,21 mmol) werden in einer Mischung aus 10 ml Methanol, 1 ml Eisessig und 1 ml Wasser mit 1 g Raney-Nickel (wassernass) versetzt und bei Raumtemperatur und Normaldruck bis zu einer Wasserstoffaufnahme von 91 ml hydriert. Zur Aufarbeitung wird der Katalysator abfiltiert und die verbleibende Lösung zum Rückstand eingeengt. Aufreinigung erfolgt durch Kristallisation aus Methanol/Diethylether; Ausbeute: 716 mg (3,41 mmol) = 81 % 3-Hydroxymethyl-benzamidiniumacetat; F.188°; ESI 151 (M+H); HPLC: Rt. = 0.51 min (Methode C).
d) In einem 100 ml Dreihalskolben werden unter Stickstoffatmosphäre 716 mg 3-Hydroxymethyl-benzamidiniumacetat (3,41 mol) und 1662 mg Aminoreductone precursor (Acros Best. Nr. 292440050) in 15 ml absolutem Methanol suspendiert, unter Rühren eine frisch hergestellte Lösung von 0,235 g Natrium in 5 ml absolutem Methanol zugetropft und anschließend 30 min bei 60 °C gerührt, wobei eine klare Lösung entsteht. Zur Aufarbeitung wird das Reaktionsgemisch mit 50 ml Dichlormethan verdünnt, zweimal mit 20 ml H₂O gewaschen, über Natriumsulfat getrocknet, zum Rückstand abgezogen und durch Chromatographie gereinigt (FlashMaster II Gradient 0 - 5 % Methanol in Dichlormethan in 40 min); Ausbeute 597 mg (2,33 mmol) = 68 % N'-[2-(3-Hydroxymethyl-phenyl)-pyrimidin-5-yl]-N,N-dimethyl-formamidin; F. 105 - 106°; ESI 257 (M+H), HPLC: Rt. = 2.24 min (Methode C).
e) 190 mg 2-Formyl-3-oxo-propionsäure-ethylester (1,32 mmol) werden in 5 ml absolutem Pyridin gelöst und mit 252 mg 3-Hydroxymethyl-benzamidiniumacetat (1,2 mmol) versetzt. Diese Suspension wird 2 Stunden bei 90° im Heizblock erhitzt, wobei sich alles löst. Das Reaktionsgemisch wird in 30 ml Wasser eingerührt. Die ausgefallenen Kristalle werden abegsaugt, gut mit Wasser gewaschen und über Nacht bei 80° unter Vakuum im Trockenschrank getrocknet; Ausbeute: 279 mg beige Kristalle = 90% d.Th; ESI 301 (M+H), HPLC: Rt. = 3.06 min (Methode B).
f) In einem 25 ml Einhalskolben werden 149 mg (1,00 mmol) 5-Methylbenzoxazolon unter Schutzgasatmosphäre in 5 ml absolutem THF suspendiert und anschließend 249 mg (1,15 mmol) N'-[2-(3-Hydroxymethyl-phenyl)-pyrimidin-5-yl]-N,N-dimethyl-formamidin und 397 mg (1,50 mmol) Triphenylphosphin bei Raumtemperatur zugegeben. Das Reaktionsgemisch wird 30 min bei RT gerührt. Anschließend wird der Reaktionsansatz im Eisbad eingekühlt und bei 0° 310 µl (1,50 mmol) Diisopropylazodiarboxylat zugetropft und nach beendeter Zugabe 2 h bei RT nachgerührt. Der Reaktionsansatz wird mit 30 ml Diethylether verdünnt, das entstandene Kristallisat abgesaugt, mit Diethylether gewaschen und im Vakuumtrockenschrank bei 50° getrocknet.

Ausbeute: 263 mg (0,68 mmol) = 68 % N,N-Dimethyl-N'-{2-[3-(5-methyl-2-oxo-benzooxazol-3-ylmethyl)-phenyl]-pyrimidin-5-yl}-formamidin ("A44"); ESI 387 (M+H); HPLC: Rt. = 3.71 min (Methode C);
¹H NMR (250 MHz, DMSO-d₆) δ [ppm] 8.486 (s, 2H), 8.309 (s, 1 H), 8.242 (m, 1H), 8.007 (s, 1 H), 7.473 (m, 2H), 7.208 (s, 1 H), 7.097 (d, 1 H), 6.987 (d, 1H), 5.114 (s, 2H), 3.070 (s, 3H), 2.983 (s, 3H), 2.313 (s, 3H).

### Beispiel 7

### Analog nachstehendem Schema erhält man 5-Methyl-3-[3-(5-methyl-pyrimidin-2-yl)-benzyl]-3H-benzooxazol-2-on ("A45")

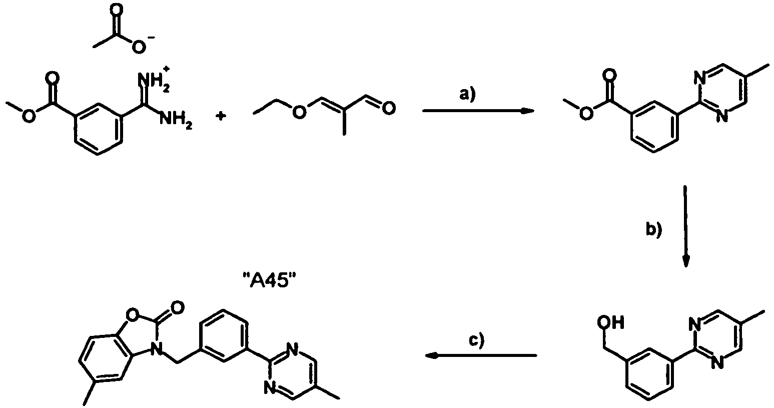

### Stufe a:

Herstellung von 3-(5-Methyl-pyrimidin-2-yl)-benzoesäuremethylester: 2.41 g (10 mmol) 3-Carbamimidoyl-benzoesäuremethylester Acetat werden in 40 ml Methanol suspendiert, mit 1.31 ml (11 mmol) 3-Ethoxymethacrolein und 2.04 ml 30% Natriummethanolat in Methanol versetzt und 15 h bei 50 °C gerührt. Das Reaktionsgemisch wird zum Rückstand eingeengt und mit 100 ml Wasser versetzt. Der gebildete Niederschlag wird abgesaugt und im Vakuum getrocknet. Das Rohprodukt wird ohne weitere Aufreinigung weiter umgesetzt; Produkt: 1.65 g; ESI: 229 (M+H).

### Stufe b:

### Herstellung von [3-(5-Methyl-pyrimidin-2-yl)-phenyl]-methanol:

1.65 g (7.16 mmol) 3-(5-Methyl-pyrimidin-2-yl)-benzoesäuremethylester gelöst in 7 ml THF werden unter Stickstoffatmosphäre zu einer Suspension aus 272 mg (7.16 mmol) Lithiumaluminiumhydrid in 7 ml THF getropft und 24 h bei Raumtemperatur gerührt. Anschließend werden 4 ml eines THF/ Wasser Gemisches (1:1) zugetropft. Nun wird eine Lösung von 1.5 g Na₂C0₃ in 4 ml Wasser zugegeben, der Niederschlag abgesaugt und der Rückstand mit 2 x THF/Ethylacetat aufgekocht und wiederum abgesaugt. Die vereinten Mutterlaugen werden zum Rückstand eingeengt, in Dichlormethan gelöst, über Natriumsulfat getrocknet, filtriert und wieder zum Rückstand eingeengt. Das Rohprodukt wird mittels Säulenchromatographie an Kieselgel aufgereinigt; Produkt: 500 mg; ESI: 201 (M+H).

### Stufe c:

### Herstellung von 5-Methyl-3-[3-(5-methyl-pyrimidin-2-yl)-benzyl]-3H-benzoxazol-2-on:

112 mg (0.75 mmol) 5-Methyl-3H-oxazolo[4,5-b]pyridin-2-on, 180 mg (0.90 mmol) [3-(5-Methyl-pyrimidin-2-yl)-phenyl]-methanol und 238 mg (0.90 mmol) Triphenylphosphin werden in 5 ml THF suspendiert und 30 min gerührt. Anschließend wurde auf 0°C gekühlt und 186 µl (0.90 mmol) Diisopropylazodicarboxylat zugegeben. Das Reaktionsgemisch wird 2 h bei Raumtemperatur gerührt. Das Reaktionsgemisch wird mit 20 ml Dichlormethan verdünnt, mit 2 x 20 ml Wasser gewaschen, über Natriumsulfat getrocknet und eingeengt. Der Rückstand wird mittels Säulenchromatographie an Kieselgel aufgereinigt; Ausbeute: 101 mg "A45"; ESI: 332 (M+H); Rt. = 4.91 min (Methode C);
¹H NMR (250 MHz, OMSO-d₆) δ [ppm] 8.742 (s, 1 H), 8.370 (s, 1 H), 8.300 (m, 1 H), 7.497 (m, 2H), 7.206 (s, 1H), 7.103 (d, 1H), 6.975 (d, 1H), 5.129 (s, 2H), 2.311 (s, 3H), 2.304 (s, 3H).

### Beispiel 8

Die Herstellung von 3-{3-[6-(3-Dimethylamino-propoxy)-pyridazin-3-yl]-benzyl}-2-oxo-2,3-dihydro-benzoxazol-5-carbonsäure-propylamid ("A54") erfolgt analog nachstehendem Schema

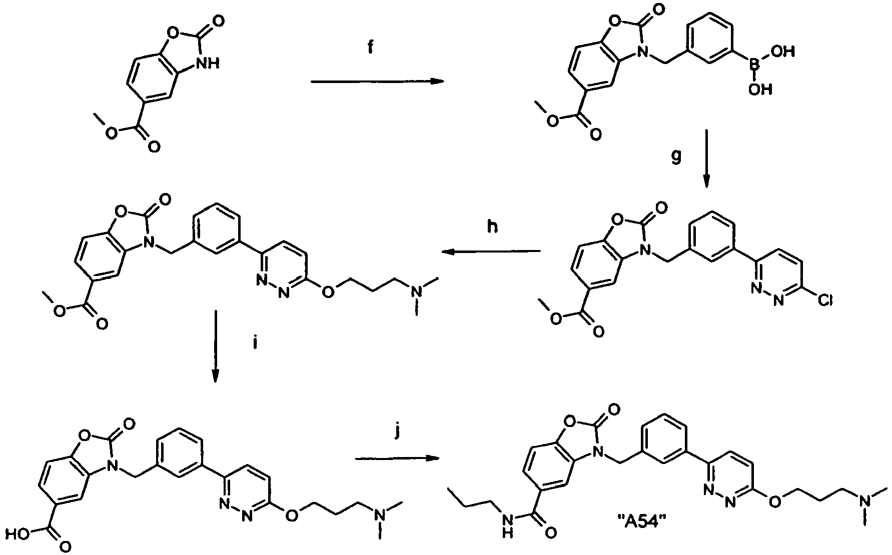

### Schritt f)

Die Herstellung des Edukts 2-Oxo-2,3-dihydro-benzoxazol-5-carbonsäuremethylester erfolgt analog
1) Varma; Kapoor; CUSCAM; Curr. Sci.; 46; 1977; 779
2) Einhorn; Ruppert; JLACBF; Justus Liebigs Ann. Chem.; 325; 1902; 320.

Die Kupplung mit 3-Hydroxymethylphenylboronsäure erfolgt nach Standardverfahren.

### Schritt g)

### Herstellung von 3-[3-(6-Chlor-pyridazin-3-yl)-benzyl]-2-oxo-2,3-dihydro-benzoxazol-5-carbonsäure-methylester

Die Umsetzung der in Schritt g) erhaltenen Boronsäure mit 3-Chlor-6-iodo-pyridazin (Herstellung analog Goodman, Allan J.; Stanforth, Stephen P.; Tarbit, Brian; TETRAB; Tetrahedron; EN; 55; 52; 1999; 15067 - 15070) erfolgt analog Goodman, Allan J.; Stanforth, Stephen P.; Tarbit, Brian; Tetrahedron; 55; 52; 1999; 15067 - 15070.

### Schritt h)

Die Herstellung von 3-{3-[6-(3-Dimethylamino-propoxy)-pyridazin-3-yl]-benzyl}-2-oxo-2,3-dihydro-benzoxazol-5-carbonsäure-methylester erfolgt analog Heinisch, Gottfried; Langer, Thierry; J. Heterocycl. Chem.; 30; 6; 1993; 1685-1690.

### Schritt i)

Saure Esterspaltung nach Standardmethode

### Schritt i)

Bildung des Amids nach Standardmethode (Reagenzien TBTU/HOBt)

Analog werden nachstehende Verbindungen erhalten

| Nr. | Name und/oder Struktur | LCMS- Retentionszeit [min] 1 LCMS-Masse [M+H]⁺/ F. [°C] |
|---|---|---|
| "A55" | 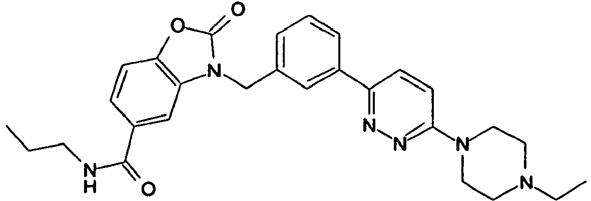 | |
| "A56" | 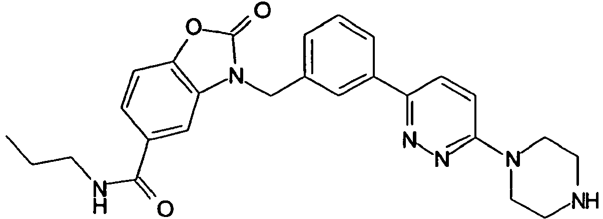 | |
| "A57" | 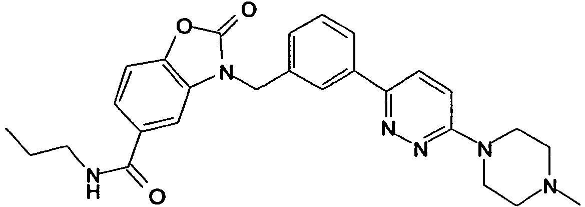 | |
| "A58" | 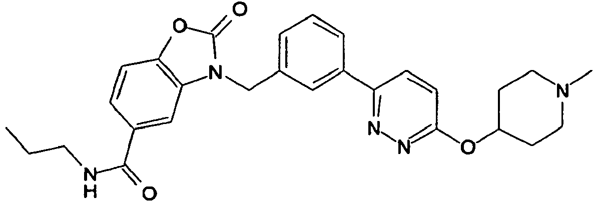 | |
| "A59" | 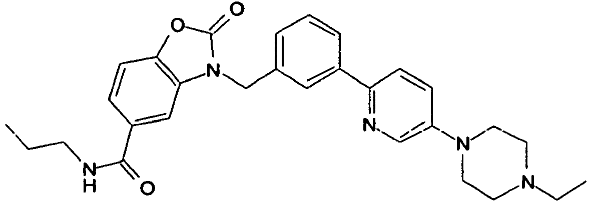 | |
| "A60" | 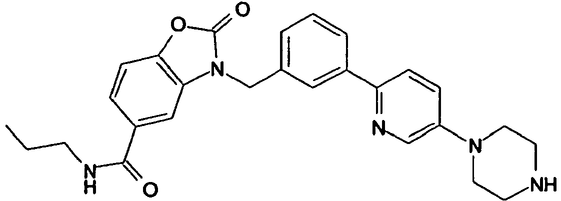 | |
| "A61" | 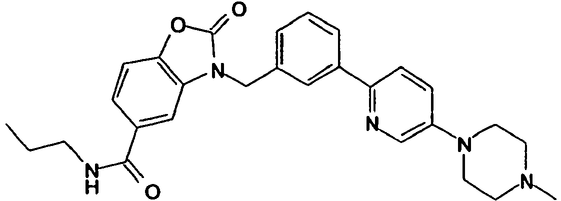 | |
| "A62" | 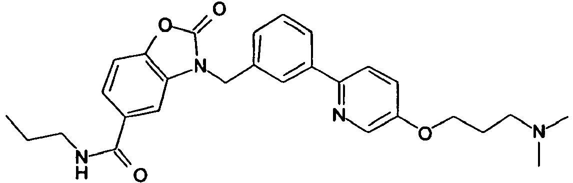 | |
| "A63" | 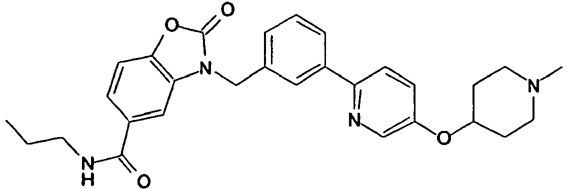 | |

### Beispiel 9

Die Herstellung von {3-[6-(1-Hydroxy-ethyl)-2-oxo-benzooxazol-3-ylmethyl]-phenyl}-carbaminsäure-3-(4-methyl-piperazin-1-yl)-propylester ("A9") erfolgt analog nachstehendem Schema

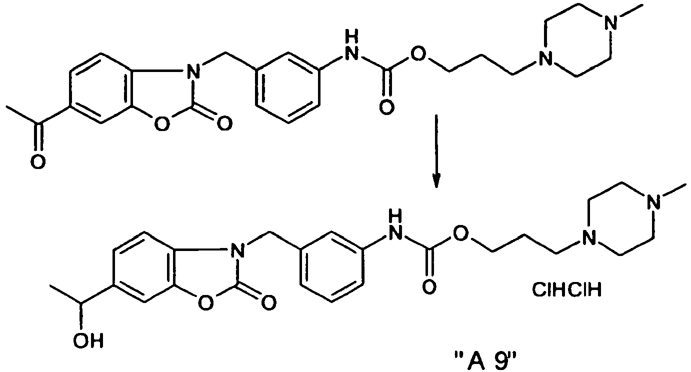

477 mg (1.02 mmol) [3-(6-Acetyl-2-oxo-benzooxazol-3-ylmethyl)-phenyl]-carbaminsäure-3-(4-methyl-piperazin-1-yl)-propylester werden in 20 ml Ethanol gelöst und unter Kühlung werden portionsweise 38.7 mg (1.02 mmol) Natriumborhydrid zugegeben. Es wird 1 h bei Raumtemperatur nachgerührt, die klare Reaktionslösung wird mit Wasser verdünnt und mit Dichlormethan extrahiert. Die organische Phase wird getrocknet, filtriert und zum Rückstand abgezogen. Der Rückstand wird mittels Säulenchromatographie an Kieselgel aufgereinigt. Das Produkt wird mit 4N Dioxan/HCl und Ether als Dihydrochlorid gefällt. Man erhält 322 mg "A9"; F. 215°C; ESI 469 (M+H); HPLC: Rt. = 3,63 min (Methode A).

Analog erhält man die Verbindung

| | | |
|---|---|---|
| "A28" | {3-[5-(1-Hydroxy-ethyl)-2-oxo-benzoxazol-3- ylmethyl]-phenyl}-carbaminsäure-3-(4-methyl- piperazin-1-yl)-propylester | 1,439 / 469.2 /140 (Zersetzung) ESI: 469 (M+H), HPLC: Rt. = 3.65 min (Methode A) |
| | 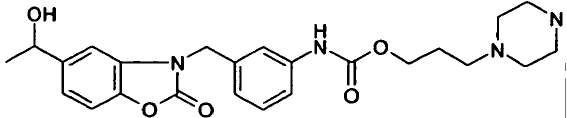 | |
| | Dihydrochlorid [erhältlich aus "A8" durch NaBH₄-Reduktion] | |

### Beispiel 10

Die Herstellung von 3-{3-[5-(3-Dimethylamino-propoxy)-pyrimidin-2-yl]-benzyl}-2-oxo-2,3-dihydro-benzoxazol-5-carbonsäuremethylester ("A64") erfolgt analog nachstehendem Schema

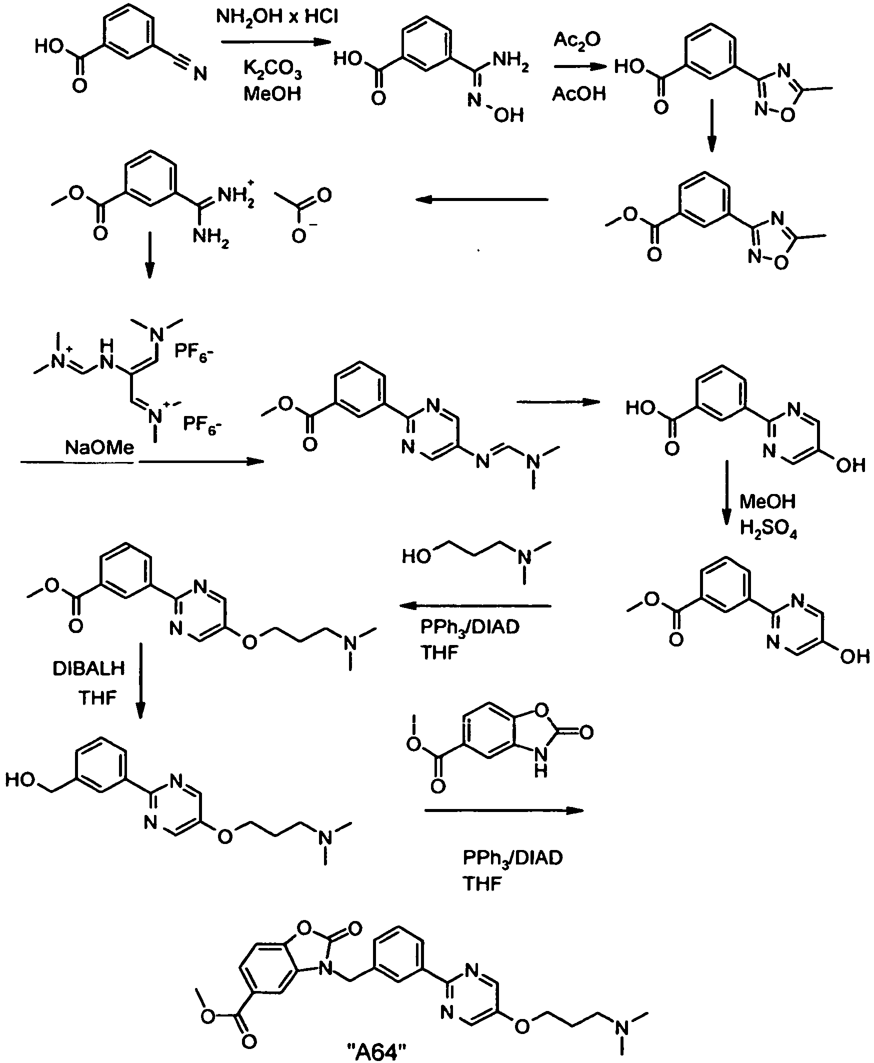

### Stufe 1:

### Herstellung von 3-(N-Hydroxycarbamimidoyl)-benzoesäure:

Zu einer auf 30° C gehaltenen Suspension von 500 g (3.40 mol) 3-Cyanbenzoesäure in 8 I Methanol werden unter Rühren portionsweise 1382 g (10.0 mol) Kaliumcarbonat gegeben. Anschließend werden bei einer Innentemperatur von 40 - 45° C 695 g (10.0 mol) Hydroxylammoniumchlorid in kleinen Portionen zugegeben. Dann wird das Reaktionsgemisch 15 Stunden zum Sieden erhitzt. Das Reaktionsgemisch wird im Vakuum eingeengt, der Rückstand in Wasser gelöst und mit 37%iger wässriger Salzsäure angesäuert. Der entstandene Niederschlag wird abgesaugt, mit Wasser gewaschen und im Vakuum getrocknet: 3-(N-Hydroxy-carbamimidoyl)-benzoesäure als farblose Kristalle; F. 208°C; ESI 181 (M+H).

### Stufe 2:

### Herstellung von 3-(5-Methyl-[1,2,4]oxadiazol-3-yl)-benzoesäure:

Ein Gemisch von 614 g (3.41 mol) 3-(N-Hydroxycarbamimidoyl)-benzoesäure, 756 ml (8.0 mol) Essigsäureanhydrid und 2 I Essigsäure wird 14 Stunden auf eine Temperatur von 118° C erhitzt. Das Reaktionsgemisch wird auf 6° C gekühlt und abgesaugt. Der Rückstand wird in 2 I Wasser aufgenommen, abgesaugt und gut mit Wasser gewaschen. Der Rückstand wird aus Ethanol/Wasser umkristallisiert: 3-(5-Methyl-[1,2,4]oxadiazol-3-yl)-benzoesäure als farblose Kristalle; F. 225° C; ESI 205 (M+H).

### Stufe 3:

### Herstellung von 3-(5-Methyl-[1,2,4]oxadiazol-3-yl)-benzoesäuremethylester:

Eine Suspension von 30.0 g (147 mmol) 3-(5-Methyl-[1,2,4]oxadiazol-3-yl)-benzoesäure in 150 ml Methanol wird mit 7.83 ml (147 mmol) konzentrierter Schwefelsäure versetzt und 18 Stunden zum Sieden erhitzt. Das Reaktionsgemisch wird im Eisbad gekühlt, mit Wasser versetzt, abgesaugt und gut mit Wasser gewaschen: 3-(5-Methyl-[1,2,4]oxadiazol-3-yl)-benzoesäuremethylester als farblose Kristalle; F. 81°C; ESI 219 (M+H); HPLC: Rt. = 2.65 min (Methode B).

### Stufe 4:

### Herstellung von 3-Carbamimidoyl-benzoesäuremethylester Acetat:

Eine Lösung von 327 g (1.47 mol) 3-(5-Methyl-[1,2,4]oxadiazol-3-yl)-benzoesäuremethylester in 3 I Methanol wird mit 150 ml Essigsäure, 150 ml Wasser und 50 g wasserfeuchtem Raney-Nickel versetzt und 18 Stunden bei Raumtemperatur und Normaldruck hydriert. Der Katalysator wird abfiltriert und das Filtrat eingedampft. Der Rückstand wird in *tert.-*Butylmethylether aufgenommen, zum Sieden erhitzt und abgesaugt. Der Rückstand wird im Vakuum getrocknet: 3-Methoxycarbonylbenzamidinium-Acetat als farblose Kristalle; F. 222°C; ESI 179 (M+H); HPLC: Rt. = 1.40 min (Methode B).

### Stufe 5:

### Herstellung von 3-[5-(Dimethylamino-methylenamino)-pyrimidin-2-yl]-benzoesäuremethylester:

Zu einer Suspension von 259 g (1.09 mol) 3-Methoxycarbonylbenzamidinium-Acetat und 528 g (1.08 mol) ({2-Dimethylamino-1-[dimethylimmoniomethyl]-vinylamino}-methylen)-dimethyl-ammonium-di-hexafluorophosphat ("aminoreductone precursor", hergestellt gemäß C. B. Dousson et al., Synthesis 2005, 1817) in 1 l methanol werden unter Rühren 2.2 l einer frisch bereiteten 1.5 M Natriummethanolat-Lösung zugetropft. Dann wird das Reaktionsgemisch innerhalb von 40 min auf 60° C erwärmt und 30 min bei dieser Temperatur gehalten. Dann wird das Reaktionsgemisch auf Raumtemperatur abgekühlt, mit 10 I Dichlormethan verdünnt und dreimal mit je 5 I Wasser gewaschen. Die organische Phase wird über Natriumsulfat getrocknet und eingedampft. Der Rückstand wird aus Ethylacetat umkristallisiert: 3-[5-(Dimethylamino-methylenamino)-pyrimidin-2-yl]-benzoesäuremethylester als beige Kristalle; F. 146° C; ESI 285 (M+H); HPLC: Rt. = 2.03 min (Methode B).

### Stufe 6:

### Herstellung von 3-(5-Hydroxy-pyrimidin-2-yl)-benzoesäure:

Eine Suspension von 103.5 g (364 mmol) 3-[5-(Dimethylamino-methylenamino)-pyrimidin-2-yl]-benzoesäuremethylester in 1.3 I Wasser wird mit 160 ml (2.88 mol) konzentrierter Schwefelsäure versetzt und 4 Stunden zum Sieden erhitzt. Das Reaktionsgemisch wird auf Raumtemperatur abgekühlt, mit Wasser verdünnt und abgesaugt. Der Rückstand wird mit Wasser gewaschen und im Vakuum getrocknet: 3-(5-Hydroxypyrimidin-2-yl)-benzoesäure als bräunliche Kristalle; F. 293-295°C; ESI 217 (M+H); HPLC: Rt. = 3.25 min (Methode C).

### Stufe 7:

### Herstellung von 3-(5-Hydroxy-pyrimidin-2-yl)-benzoesäuremethylester:

Eine Suspension von 88.0 g (366 mmol) 3-(5-Hydroxypyrimidin-2-yl)-benzoesäure in 1.4 l Methanol wird mit 32.7 ml (445 mmol) Thionylchlorid versetzt und 2 Stunden auf 80° C erhitzt. Dann werden 20 ml (276 mmol) Thionylchlorid und nach 2 Stunden noch mal 10 ml (138 mmol) Thionylchlorid zugegeben. Nach jeder Zugabe wird das Reaktionsgemisch 2 Stunden bei 80° C gerührt. Das Reaktionsgemisch wird im Vakuum auf ein Volumen von ca. 300 ml eingeengt. Der entstandene Niederschlag wird abfiltriert und im Vakuum getrocknet: 3-(5-Hydroxypyrimidin-2-yl)-benzoesäuremethylester als bräunliche Kristalle; F. 219-223°C, ESI 231 (M+H); HPLC: Rt. = 3.87 min (Methode C).

### Stufe 8:

### Herstellung von 3-[5-(3-Dimethylamino-propoxy)-pyrimidin-2-yl]-benzoesäuremethylester:

Eine unter Stickstoff gehaltene Lösung von 6.1 g (26.5 mmol) 3-(5-Hydroxypyrimidin-2-yl)-benzoesäuremethylester, 10.5 g (39.8 mmol) Triphenylphosphin und 4.76 ml (39.8 mmol) 3-(Dimethylamino)-1-propanol in 200 ml THF wird im Eisbad gekühlt und es werden anschließend langsam unter Rühren 8.21 ml (39.8 mmol) Diisopropylazodicarboxylat zugetropft. Nach 2 Stunden Rühren bei Raumtemperatur wird das Reaktionsgemisch im Vakuum eingedampft. Der Rückstand wird zwischen Dichlormethan und gesättigter wässriger Kaliumhydrogensulfat-Lösung verteilt. Die wässrige Phase wird abgetrennt, mit gesättigter wässriger Natronlauge auf einen pH-Wert von 12 gebracht und zweimal mit Dichlormethan extrahiert. Die organische Phase wird über Natriumsulfat getrocknet und eingedampft. Der Rückstand wird an einer Kieselgelsäule mit Dichlormethan/Methanol als Laufmittel chromatographiert: 3-[5-(3-Dimethylamino-propoxy)-pyrimidin-2-yl]-benzoesäuremethylester als farblose Kristalle; F. 66°C; ESI 316 (M+H); HPLC: 2.18 min (Methode B).

### Stufe 9:

### Herstellung von {3-[5-(3-Dimethylamino-propoxy)-pyrimidin-2 -yl]-phenyl}-methanol:

Zu einer unter Stickstoff gehaltenen Lösung von 12.6 g (40.0 mmol) 3-[5-(3-Dimethylamino-propoxy)-pyrimidin-2-yl]-benzoesäuremethylester in 200 ml THF werden unter Rühren 200 ml einer 1 M Lösung von Diisobutylaluminiumhydrid in THF zugetropft. Nach 1 Stunde Rühren bei Raumtemperatur werden 10 ml einer gesättigten wässrigen NatriumsulfatLösung zugetropft. Der entstandene Niederschlag wird abgesaugt und mit Dichlormethan gewaschen. Das Filtrat wird über Natriumsulfat getrocknet und eingedampft. Der Rückstand wird in einem Gemisch von Diethylether und Petrolether aufgenommen. Der entstandene Niederschlag wird abgesaugt, mit Petrolether gewaschen und im Vakuum getrocknet: {3-[5-(3-Dimethylamino-propoxy)-pyrimidin-2-yl]-phenyl}-methanol als farblose Kristalle; F. 95-97 °C; ESI 288 (M+H); HPLC: Rt. = 2.35 min (Methode B).

### Stufe 10:

### Herstellung von 3-{3-[5-(3-Dimethylamino-propoxy)-pyrimidin-2-yl]-benzyl}-2-oxo-2,3-dihydro-benzoxazol-5-carbonsäuremethylester:

Eine Lösung von 3.03 g (10.45 mmol) {3-[5-(3-Dimethylamino-propoxy)-pyrimidin-2-yl]-phenyl}-methanol in 40 ml THF wird mit 1,84 g (9.5 mmol) 2-Oxo-2,3-dihydro-benzoxazol-5-carbonsäuremethylester und 4.75 g (14.25 mmol) polymergebundenem Triphenylphosphin (3mmol/g) versetzt. Die Suspension wird 30 min bei Raumtemperatur geschüttelt. Die Suspension wird im Eisbad gekühlt und 3.35 g (14.25 mmol) Di-tert.-butylazodicarboxylat werden portionsweise zugegeben. Nach 24 h Rühren bei Raumtemperatur werden nochmals 4.75 g (14.25 mmol) polymergebundenes Triphenylphosphin (3mmol/g) und 3.35 g (14.25 mmol) Di-tert.-butylazodicarboxylat zugegen und weitere 24 h bei Raumtemperatur geschüttelt. Das Reaktionsgemisch wird filtriert, das Filtrat wird zum Rückstand eingedampft und der Rückstand mittels Säulenchromatographie Kieselgel aufgereinigt.
Man erhält 947 mg "A64"; F. 125°C, ESI: 463 (M+H); Rt. = 3.07 min (Methode C);
¹H-NMR (DMSO-d₆, δ in ppm): .636 (S, 2H), 8.331 (SB, 1H), 8.250 (D, 1 H), 7.806 (M, 2H), 7.514 (M, 3H), 5.251 (S, 2H), 4.217 (T, 2H), 3.829 (S, 3H), 2.370 (T, 2H), 2.153 (S, 6H), 1.895 (T, 2H).

Analog werden folgende Verbindungen hergestellt. In einigen Fällen wird zur besseren Lösung der Edukte in Stufe 10 DMF als Lösungsmittel verwendet. In einigen Fällen werden die Rohprodukte mittels präparativer HPLC aufgereinigt. In einigen Fällen werden die Zielverbindungen in Aceton gelöst und mit 4N HCI in Dioxan als Hydrochlorid gefällt.

Analog werden nachstehende Verbindungen erhalten

| Nr. | Name und/oder Struktur | ESI (M+H) | Rt. in min |
|---|---|---|---|
| "A65" | 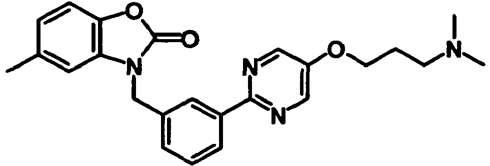 | 419 | 2.52 (Methode B) |
| | Hydrochlorid | | |
| "A66" | 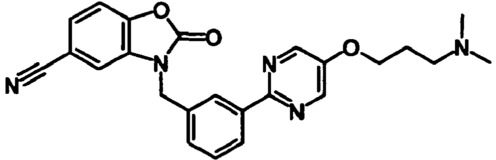 | 430 | 2.36 (Methode B) |
| | Hydrochlorid | | |
| "A67" | 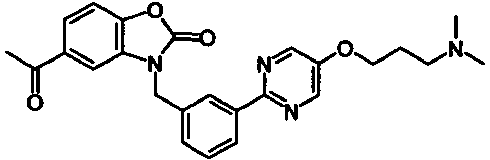 | 447 | 2.33 (Methode B) |
| "A68" | 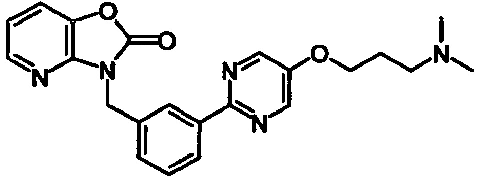 | 446 | 2.25 (Methode B) |
| "A69" | 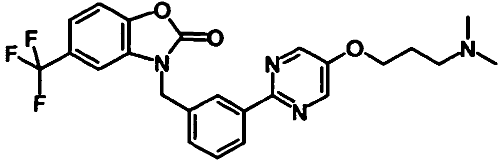 | 473 | 2.65 (Methode B) |
| | Trifluoracetat | | |
| "A70" | 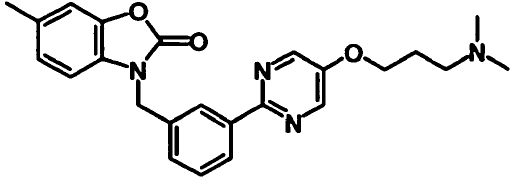 | 419 | 2.52 (Methode B) |
| | Hydrochlorid | | |
| "A71" | 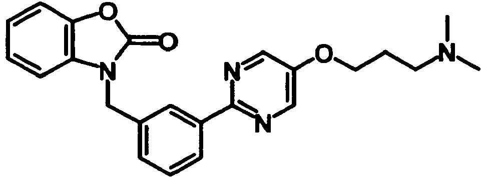 | 405 | 2.39 (Methode B) |
| | Hydrochlorid | | |
| "A72" | 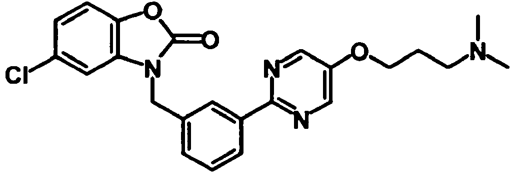 | 439 | 2.54 (Methode B) |
| | Trifluoracetat | | |
| "A73" | 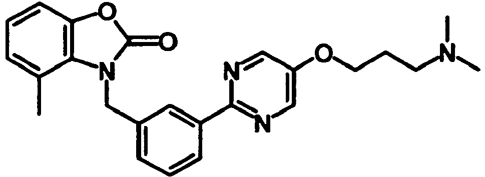 | 419 | 2.48 (Methode B) |
| "A74" | 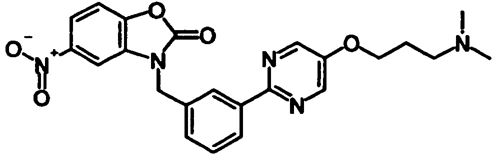 | 450 | 2.41 (Methode B) |
| "A75" | 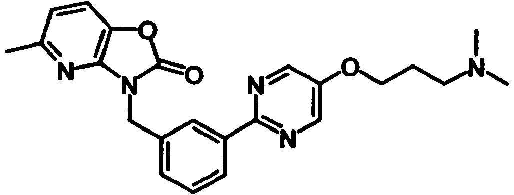 | 420 | 2.36 (Methode B) |
| "A76" | 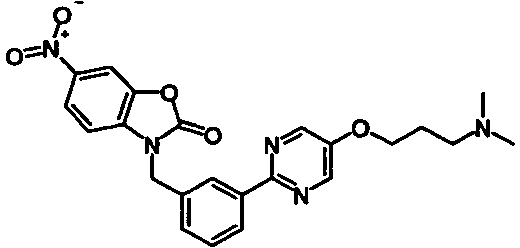 | 450 | 2.43 (Methode B) |
| | Trifluoracetat | | |
| "A77" | 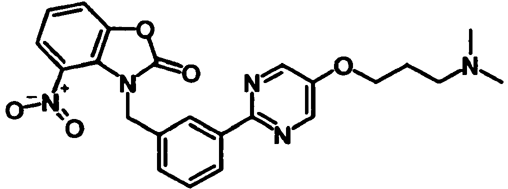 | 450 | 2.43 (Methode B) |
| | Trifluoracetat | | |
| "A78" | 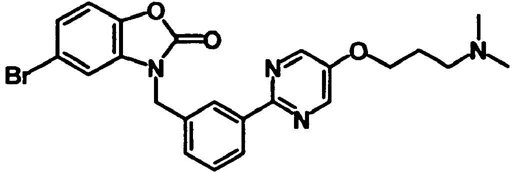 | 484 | 2.58 (Methode B) |
| "A79" | 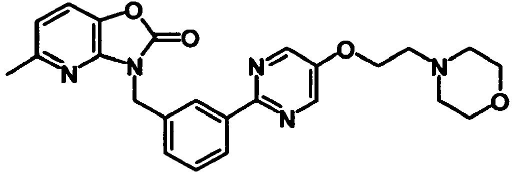 | 448 | 2.28 (Methode B) |
| "A80" | 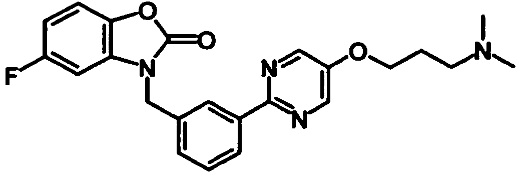 | 423 | 2.43 (Methode B) |
| "A81" | 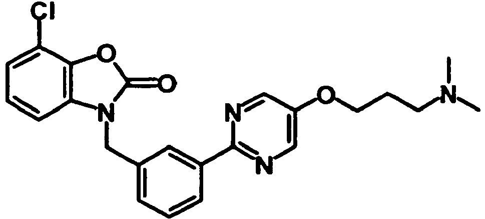 | 439 | 2.56 (Methode B) |
| "A82" | 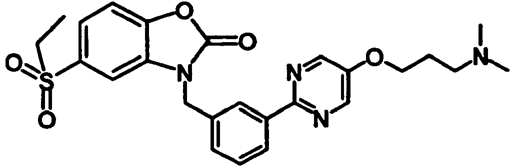 | 497 | 2.26 (Methode B) |
| "A83" | 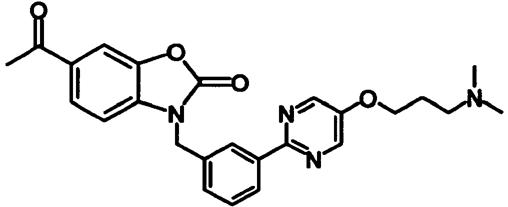 | 447 | 2.31 (Methode B) |
| "A84" | 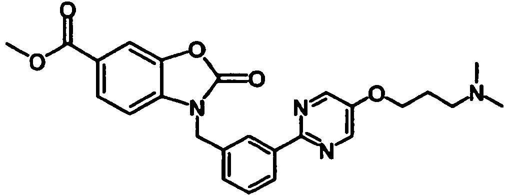 | 463 | 2.42 (Methode B) |
| "A85" | 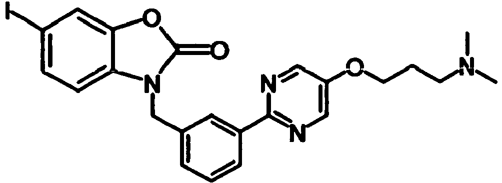 | 530 | 2.66 (Methode B) |
| "A86" | 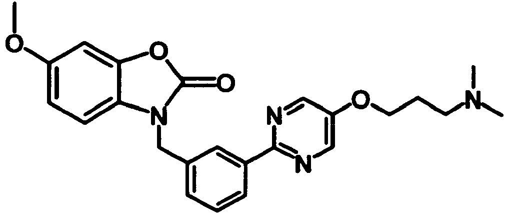 | 435 | 2.39 (Methode B) |
| "A87" | 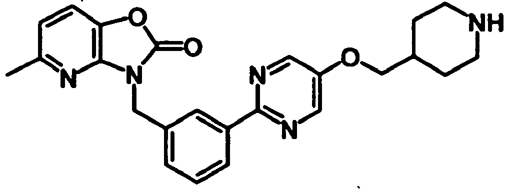 | 432 | 2.41 (M+H) |
| | Trifluoracetat | | |
| "A88" | 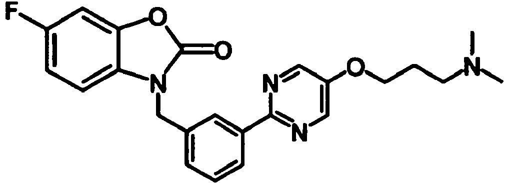 | 423 | 2.43 (Methode B) |
| | Hydrochlorid | | |
| "A89" | 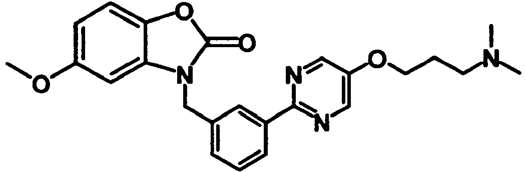 | 435 | 2.42 (Methode B) |
| "A90" | 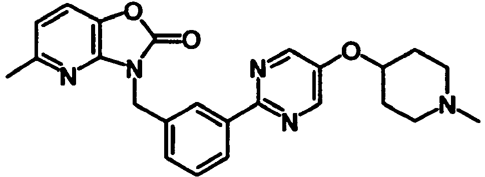 | 432 | 2.37 (Methode B) |
| | Trifluoracetat | | |
| "A91" | 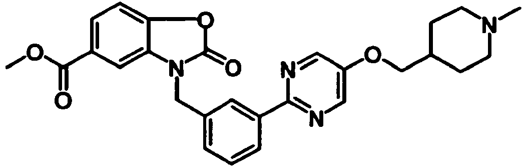 | 489 | 4.27 (Methode C) |
| "A92" | 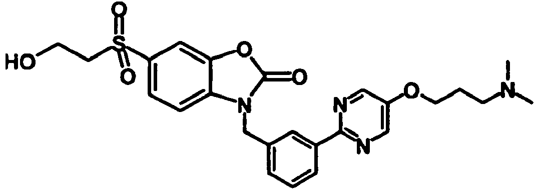 | 514 | 2.13 (Methode B) |
| "A93" | 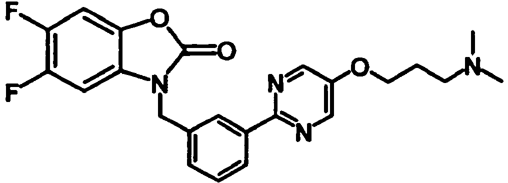 | 441 | 2.47 (Methode B) |
| | Hydrochlorid | | |
| "A94" | 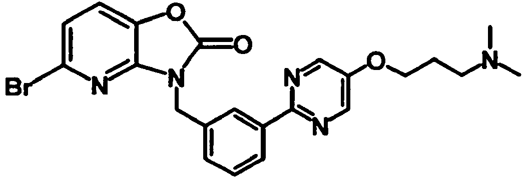 | 484/486 | 2.57 (Methode B) |
| | Trifluoracetat | | |
| "A95" | 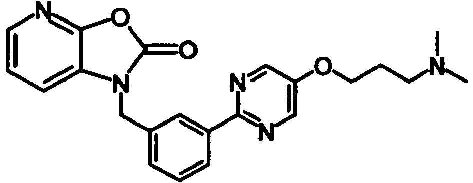 | 406 | 2.17 (Methode B) |
| | Trifluoracetat | | |
| "A96" | 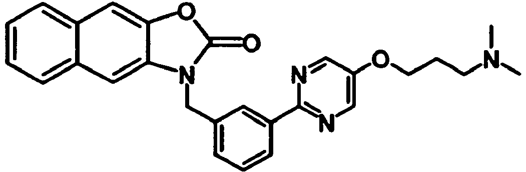 | 455 | 2.73 (Methode B) |
| "A97" | 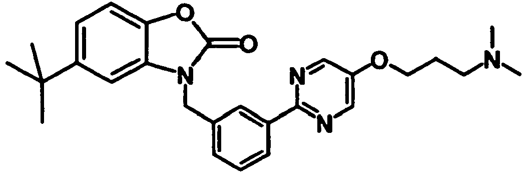 | 461 | 2.86 (Methode B) |
| | Trifluoracetat | | |
| "A98" | 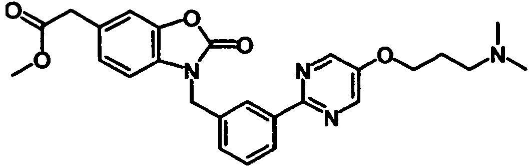 | 477 | 2.43 (Methode B) |
| | Trifluoracetat | | |
| "A99" | 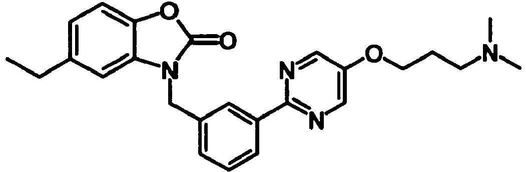 | 433 | 2.68 (Methode B) |
| "A100" | 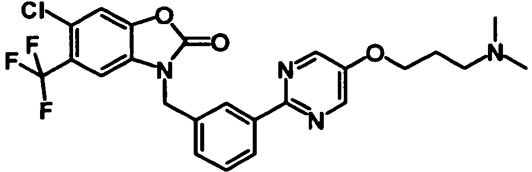 | 507 | 2.84 (Methode B) |
| | Trifluoracetat | | |
| "A101" | 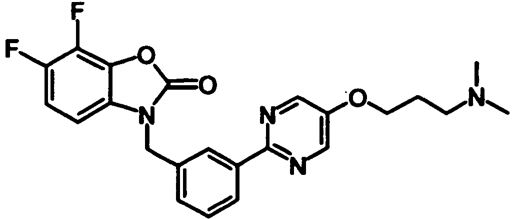 | 441 | 2.60 (Methode B) |
| | Hydrochlorid | | |
| "A102" | 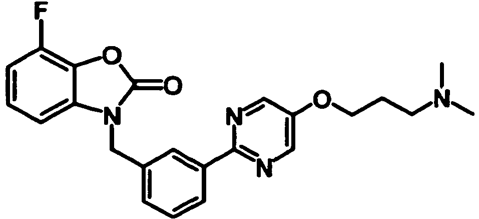 | 423 | 2.52 (Methode B) |
| | Hydrochlorid | | |
| "A103" | 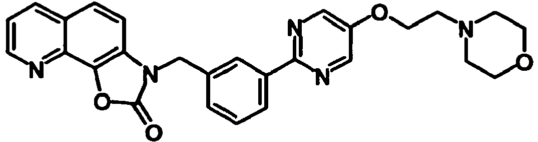 | 484 | 2.07 (Methode B) |
| "A104" | 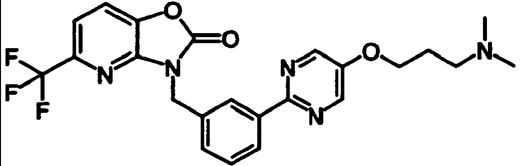 | 474 | |
| "A105" | 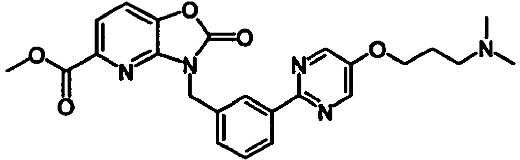 | 464 | |
| "A106" | 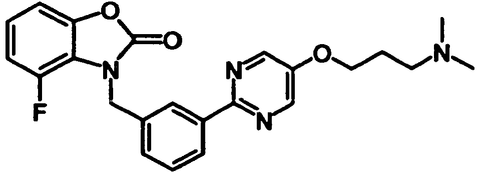 | 423 | 2.49 (Methode B) |
| | Trifluoracetat | | |
| "A107" | 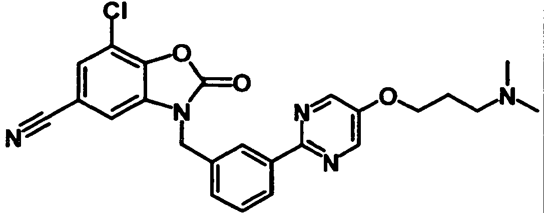 | 464 | 2.59 (Methode B) |
| | Trifluoracetat | | |
| "A108" | 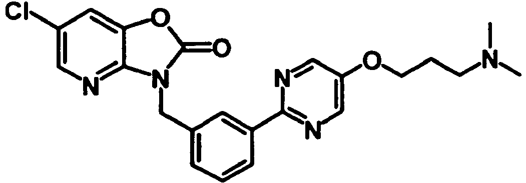 | 440 | |

### Beispiel 11

Die Herstellung von 3-{3-[5-(2-Dimethylamino-ethoxy)-pyrimidin-2-yl]-benzyl}-5-methyl-3H-oxazolo[4,5-b]pyridin-2-on ("A109") erfolgt analog nachstehendem Schema

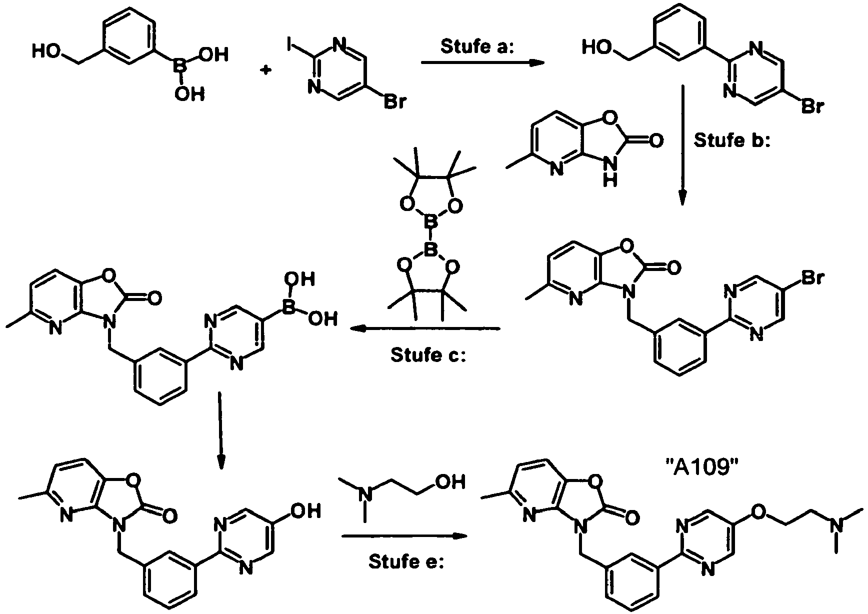

### Stufe a:

### Herstellung von [3-(5-Brompyrimidin-2-yl)-phenyl]-methanol:

Eine unter Stickstoff gehaltene Lösung von 6.11 g (21.5 mmol) 5-Brom-2-lodpyrimidin, 3.91 g (25.7 mmol) 3-(Hydroxymethyl)-benzolboronsäure und 9.11 g (42.9 mmol) Trikaliumphosphat-Trihydrat in 120 ml Dioxan und 14 ml Wasser wird mit 750 mg (0.65 mmol) Tetrakis(triphenylphosphin)-palladium versetzt und 18 Stunden bei 90° C gerührt. Das Reaktionsgemisch wird auf Raumtemperatur abgekühlt, mit t*ert*.-Butylmethylether und Wasser versetzt und über Kieselgur filtriert. Die organische Phase des Filtrats wird abgetrennt, über Natriumsulfat getrocknet und eingedampft. Der Rückstand wird an einer Kieselgelsäule mit Dichlormethan/Methanol als Laufmittel chromatographiert:
Produkt: 2.49 g; F. 114-117°C; ESI: 265, 267 (M+H); HPLC: Rt. = 2.51 min (Methode B).

### Stufe b:

### Herstellung von 3-[3-(5-Bromo-pyrimidin-2-yl)-benzyl]-5-methyl-3H-oxazolo[4,5-b]pyridin-2-on:

283 mg (1.87 mmol) 5-Methyl-3H-oxazolo[4,5-b]pyridin-2-on, 500 mg (1.87 mmol) (3-Hydroxymethyl-phenyl)-carbaminsäure-3-(4-methyl-piperazin-1-yl)-propylester und 943 mg (2.83 mmol) polymergebundenes Triphenylphosphin (3mmol/g) werden in 15 ml DMF suspendiert und 30 min geschüttelt. Anschließend werden 665 mg (2.83 mmol) Di-tert.-butylazodi-carboxylat zugegeben. Das Reaktionsgemisch wird bei Raumtemperatur geschüttelt. Das Reaktionsgemisch wird filtriert, der Rückstand mit THF gewaschen und das Filtrat eingedampft. Das Rohprodukt wird mittels präparativer HPLC aufgereinigt; ESI: 399 (M+H); Rt. = 3.12 min (Methode B);
¹H-NMR (DMSO-d₆, δ in ppm): 9.08 (2H, s); 8.40 (1 H, b); 8.29 (1 H, m); 7.65 (1H, d); 7.51-7.60 (2H, m); 7.05 (1 H, d); 5.11 (2H, s); 2.46 (3H, s).

### Stufe c:

### Herstellung von 2-(3-((5-methyl-2-oxoxazolo[4,5-b]pyridin-3(2H)-yl)methyl)phenyl)pyrimidin-5-yl-5-boronsäure:

Eine Suspension von 500 mg (1.13 mmol) 3-[3-(5-Bromo-pyrimidin-2-yl)-benzyl]-5-methyl-3H-oxazolo[4,5-b]pyridin-2-on in 25 ml DMF werden mit 374 mg (1.47 mmol) Bis(pinacolato)dibor und 334 mg (3.40 mmol) Kaliumacetat versetzt und unter Stickstoff auf 70° C erhitzt. Nach 15minütigem Rühren bei dieser Temperatur werden 82 mg (0.12 mmol) Bis(triphenylhosphin)-palladium(II)-chlorid zugegeben und das Reaktionsgemisch 18 Stunden bei 70°C unter Stickstoff gerührt. Man lässt das Reaktionsgemisch auf Raumtemperatur abkühlen und gibt das Reaktionsgemisch in Eiswasser und rührt 30 min. Der gebildete Feststoff wird abgesaugt und im Vakuum getrocknet. Das Produkt wird ohne weitere Aufreinigung weiter umgesetzt, ESI: 363 (M+H); Rt. = 2.45 min (Methode B).

### Stufe d:

### Herstellung von 3-[3-(5-Hydroxy-pyrimidin-2-yl)-benzyl]-5-methyl-3H-oxazolo[4,5-b]pyridin-2-on:

Zu 500 mg (1.40 mmol) 2-(3-((5-methyl-2-oxoxazolo[4,5-b]pyridin-3(2H)-yl)methyl)phenyl)pyrimidin-5-yl-5-boronsäure in 10 ml THF und 10 ml Wasser werden unter Eiskühlung 419 mg (4.2 mmol) Natriumperborat gegeben und 2 Stunden bei Raumtemperatur gerührt. Das Reaktionsgemisch wird über Kieselgur abgesaugt. Das Filtrat wird mehrfach mit Dichlormethan extrahiert, über Natriumsulfat getrocknet und zum Rückstand eingedampft. Das Rohprodukt wird ohne weitere Aufreinigung weiter umgesetzt; ESI: 335 (M+H); Rt. = 2.71 min (Methode B).

### Stufe e:

### Herstellung von 3-{3-[5-(2-Dimethylamino-ethoxy)-pyrimidin-2-yl]-benzyl}-5-methyl-3H-oxazolo[4,5-b]pyridin-2-on:

Zu einer Suspension von 67 mg (0.2 mmol) 3-[3-(5-Hydroxy-pyrimidin-2-yl)-benzyl]-5-methyl-3H-oxazolo[4,5-b]pyridin-2-on in 3 ml DMF werden nacheinander 100 mg (0.3 mmol) polymergebundenes Triphenylphosphin (3mmol/g) und 30 µl (0.3 mmol) 2-Dimethylaminoethanol gegeben. Anschließend werden 69 mg (0.30 mmol) Di-tert.-butylazodicarboxylat zugegeben. Das Reaktionsgemisch wird 18 Stunden bei Raumtemperatur geschüttelt. Es werden noch weitere 100 mg (0.3 mmol) polymergebundenes Triphenylphosphin (3mmol/g) und 69 mg (0.30 mmol) Di-tert.-butylazodicarboxylat zugegeben und 18 h bei Raumtemperatur geschüttelt. Anschließend werden weitere 100 mg (0.3 mmol) polymergebundenes Triphenylphosphin (3mmol/g), 69 mg (0.30 mmol) Di-tert.-butylazodicarboxylat und 30 µl (0.3 mmol) 2-Dimethylaminoethanol zugegeben und 18 h bei Raumtemperatur geschüttelt. Das Reaktionsgemisch wird filtriert, das Filtrat im Vakuum eingedampft und der Rückstand mittels Säulenchromatographie an Kieselgel aufgereinigt. Man erhält "A109". Das Rohprodukt wird mittels präparativer HPLC aufgereinigt; ESI: 406; HPLC: Rt. = 2.31 min (Methode B);
¹H-NMR (DMSO-d₆, δ in ppm): 8.69 (2H, b), 8.34 (1 H, b), 8.23-8.27 (1 H, m); 7.65 (1H, d); 7.48-7.52 (2H, m); 7.05 (1H, d); 5.102 (2H, s); 4.56 (2H, t); 2.89 (6H, b), 2.51 (überlagert 2H, b); 2.49 (3H, s).

Analog werden folgende Verbindungen hergestellt. In einigen Fällen werden die Zielverbindungen in Aceton gelöst und mit 4N HCl in Dioxan als Hydrochlorid gefällt.

| Nr. | Name und/oder Struktur | ESI (M+H) | Rt. in min |
|---|---|---|---|
| "A110" | 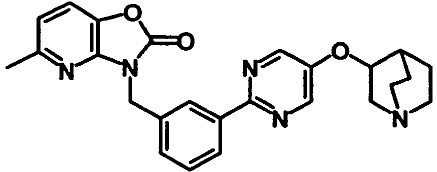 | 444 | 2.45 (Methode B) |
| "A111" | 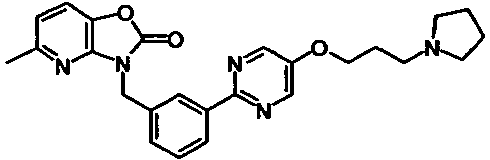 | 446 | 2.45 (Methode B) |
| "A112" | 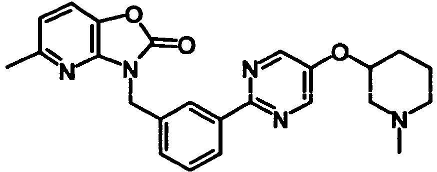 | 431 | 2.37 (Methode B) |
| "A113" | 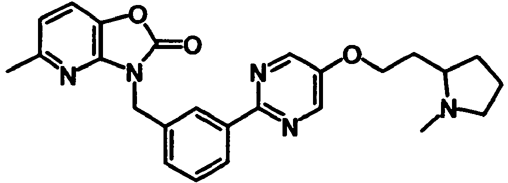 | 446 | 2.44 (Methode B) |
| "A114" | 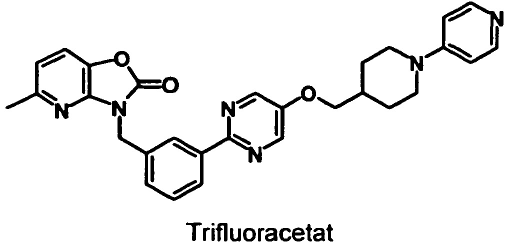 | 509 | 2.68 (Methode B) |
| "A115" | 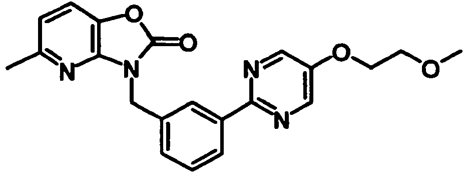 | 393 | |
| "A116" | 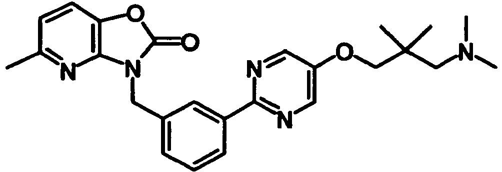 | 448 | |
| "A117" | 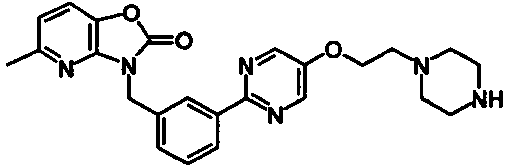 | 447 | |
| "A118" | 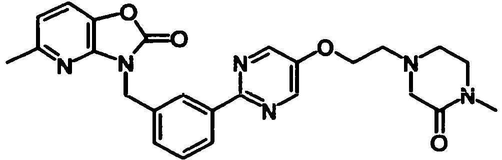 | 475 | |
| "A119" | 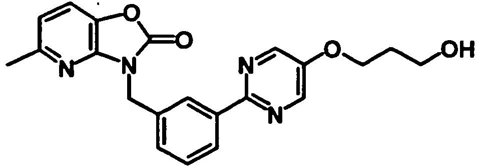 | 393 | |

### Beispiel 12

Die Herstellung von 3-{3-[5-(2,3-Dihydroxy-propoxy)-pyrimidin-2-yl]-benzyl}-5-methyl-3H-oxazolo[4,5-b]pyridin-2-on ("A120") erfolgt analog nachstehendem Schema

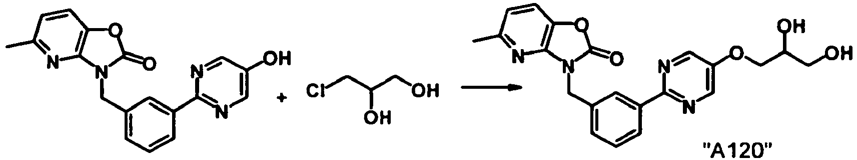

67 mg (0.2 mmol) 3-[3-(5-Hydroxy-pyrimidin-2-yl)-benzyl]-5-methyl-3H-oxazolo[4,5-b]pyridin-2-on in 3 ml Aceton werden mit 37 mg (0.34 mmol) 3-chlor-1,2-Propandiol und 156 mg (0.48 mmol) Cäsiumcarbonat gegeben. Das Reaktionsgemisch wird 16 h bei Raumtemperatur gerührt und anschließend filtriert, der Rückstand mit Aceton nachgewaschen und das Filtrat im Vakuum eingedampft. Der Rückstand wird mittels präparativer HPLC aufgereinigt; ESI: 409, HPLC: Rt. = 2.50 min (Methode B).

Analog werden folgende Verbindungen hergestellt. In einigen Fällen werden die Rohprodukte mittels Säulenchromatographie an Kieselgel aufgereinigt. In einigen Fällen werden die Zielverbindungen in Aceton gelöst und mit 4N HCl in Dioxan als Hydrochlorid gefällt.

| Nr. | Name und/oder Struktur | ESI (M+H) | Rt. in min |
|---|---|---|---|
| "A121" | 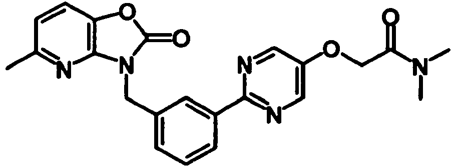 | 420 | |
| "A122" | 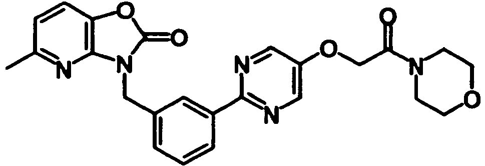 | 462 | |
| "A123" | 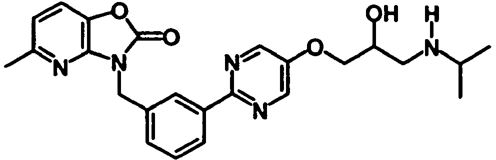 | 450 | |
| "A124" | 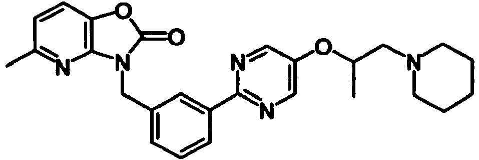 | 460 | |

### Beispiel 13

### Herstellung von 5-Methyl-3-{3-[5-(4-methyl-piperazin-1-yl)-pyrimidin-2-yl]-benzyl}-3H-oxazolo[4,5-b]pyridin-2-on ("A125")

### 13.1 Herstellung von 3-(5-Amino-pyrimidin-2-yl)-benzoesäuremethylester

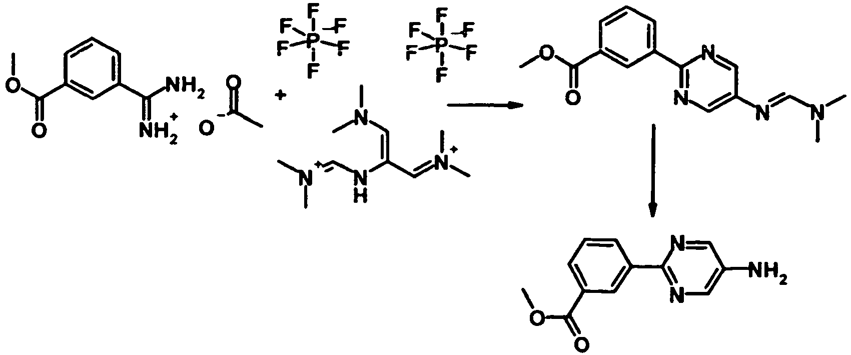

65.4 g (274 mmol) 3-Carbamimidoyl-benzoesäuremethylester werden in 800 ml Methanol suspendiert und mit 134 g (274 mmol) aminoreductone precursor versetzt. Zu dieser Suspension werden 102 ml (548 mmol) 30%ige Natriummethanolatlösung in Methanol zugetropft. Es entsteht eine Lösung. Diese wird 1 Stunde bei 60°C Innentemperatur gerührt. Nach dem Abkühlen auf Raumtemperatur werden weitere 20 ml 30%ige Natriummethanolatlösung in Methanol zugetropft und 1 Stunde bei 60°C gerührt. Nach dem Abkühlen auf Raumtemperatur wird der entstandene Niederschlag abgesaugt, der Rückstand in 1 l Wasser suspendiert und 30 min bei Raumtemperatur gerührt. Der Niederschlag wird abgesaugt und im Vakuumtrockenschrank bei 80°C getrocknet; Ausbeute: 68.5 g; HPLC: Rt. = 2.03 min (Methode B); LC-MS: 285 (M+H).

10.2 g (35.9 mmol) 3-[5-(Dimethylamino-methylenamino)-pyrimidin-2-yl]-benzoesäuremethylester werden in 1 I Methanol suspendiert. Unter leichter Kühlung (ca. 5-10°C) werden 5.3 ml (107.3 mmol) rauchende Schwefelsäure tropfenweise zugetropft (Achtung, stark exotherme Reaktion). Nach beendeter Zugabe wird zunächst 30 min bei Raumtemperatur und anschließend bei 88° Ölbadtemperatur gerührt. Die Reaktion wird mittels HPLC verfolgt. Nach 20 h wird die klare, dunkelgelbe Lösung zum Rückstand abgezogen. Der Rückstand wird in 600 ml Ethylacetat gelöst und mit 2 x 150 ml 1 N NaOH und 2 x 1 N HCl gewaschen, über Natriumsulfat getrocknet und eingedampft; Ausbeute: 3g; HPLC: Rt. = 2.17 min (Methode B); LC-MS: 300 (M+H).

### 13.2 Herstellung von {3-[5-(4-Methyl-piperazin-1-yl)-pyrimidin-2-yl]-phenyl}-methanol:

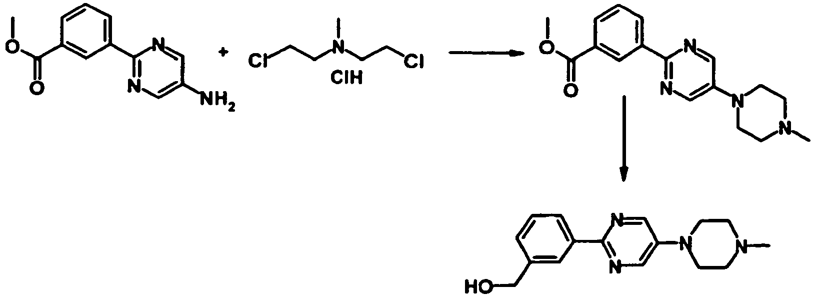

2.5 g (10.9 mmol) 3-(5-Amino-pyrimidin-2-yl)-benzoesäuremethylester werden in 10 ml NMP gelöst, mit 2.59 g (18.5 mmol) Kaliumcarbonat und 3.6 g (18.5 mmol) Bis-(2-chlor-ethyl)-ethyl-amin Hydrochlorid versetzt. Die Suspension wird unter Argonatmosphäre 15 h bei 120°C gerührt. Anschließend wird weitere 12 h bei 140°C gerührt. Nach dem Abkühlen auf Raumtemperatur wird das Reaktionsgemisch in 150 ml Wasser eingerührt. Der entstandene Niederschlag wird über Kieselgur abgesaugt und verworfen. Das Filtrat wird mit 32%-iger NaOH auf pH=14 eingestellt. Die leicht getrübte Lösung wird mit 2 x 200 ml Ethylacetat extrahiert. Die vereinigten organischen Phasen werden mit gesättigter Natriumchloridlösung gewaschen, über Natriumsulfat getrocknet und zum Rückstand eingeengt und im Vakuum getrocknet. Das Produkt wird ohne weitere Aufreinigung weiter umgesetzt;
Ausbeute: 860 mg; HPLC: Rt. = 2.11 min (Methode B); ESI: 313 (M+H).

860 mg (2.75 mmol) 3-[5-(4-Methyl-piperazin-1-yl)-pyrimidin-2-yl]-benzoesäuremethylester werden in 16 ml THF gelöst und bei Raumtemperatur werden 13.8 ml (13.8 mmol) 1 M Diisobutylaluminiumhydrid in THF zugetropft und das Reaktionsgemisch wird 1 h bei Raumtemperatur gerührt. Es werden weitere 13.8 ml (13.8 mmol) 1 M Diisobutylaluminiumhydrid in THF zugetropft und das Reaktionsgemisch wird 1 h bei Raumtemperatur gerührt. Das Reaktionsgemisch wird unter Eiskühlung mit 3 ml gesättigter Natriumsulfatlösung versetzt. Das gelartige Gemisch wird mit Dichlormethan versetzt, 30 min gerührt und filtriert. Das Filtrat wird über Natriumsulfat getrocknet und eingedampft. Ausbeute: 300 mg, gelber Feststoff. Das Produkt wird ohne weitere Aufreinigung weiter umgesetzt; HPLC: 1.68 min (Methode B); ESI: 285 (M+H).

### 13.3 Herstellung von 5-Methyl-3-{3-[5-(4-methyl-piperazin-1-yl)-pyrimidin-2-yl]-benzyl}-3H-oxazolo[4,5-b]pyridin-2-on:

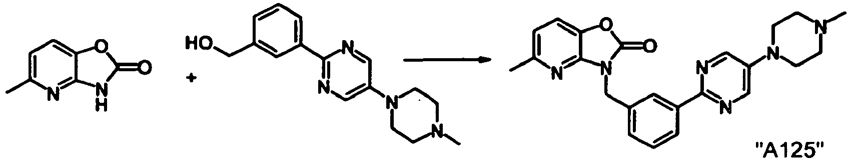

67 mg (0.44 mmol) 5-Methyl-3H-oxazolo[4,5-b]pyridin-2-on und 125 mg (0.44 mmol) {3-[5-(4-Methyl-piperazin-1-yl)-pyrimidin-2-yl]-phenyl}-methanol werden mit 222 mg (0.67 mmol) polymergebundenem Triphenylphosphin (ca. 3 mmol Triphenylphosphin pro g) in 5 ml DMF suspendiert und 30 min bei Raumtemperatur geschüttelt. Es werden 156 mg (0.67 mmol) Di-*tert*.-butylazodicarboxylat zugegeben. Das Reaktionsgemisch wird 15 h bei Raumtemperatur geschüttelt. Das Reaktionsgemisch wird filtriert, der Rückstand eingedampft und der Rückstand mittels Säulenchromatographie an Kieselgel aufgereinigt.
Man erhält 67 mg "A125"; HPLC: Rt. = 2.26 min (Methode B); ESI: 418 (M+H);
¹H-NMR (DMSO-d₆, δ in ppm): 8.58 (2H, s); 8.30 (1H, s); 8.21 (1 H, d); 7.65 (1 H, d); 7.42-7.49 (2H, m); 7.06 (1 H, d); 5.09 (2H, s); 2,48-2.52 (überlagert 8H, m); 2.47 (3H, s); 2.25 (3H, s).

### Beispiel 14

### Herstellung von 5-Methyl-3-{3-[5-(4-methyl-piperazin-1-yl)-pyrimidin-2-yl]-benzyl}-3H-oxazolo[4,5-b]pyridin-2-on ("A126")

### 14.1 Herstellung von 4-[2-(3-Hydroxymethyl-phenyl)-pyrimidin-5-yl]-piperazin-1-carbonsäure-tert.-butylester:

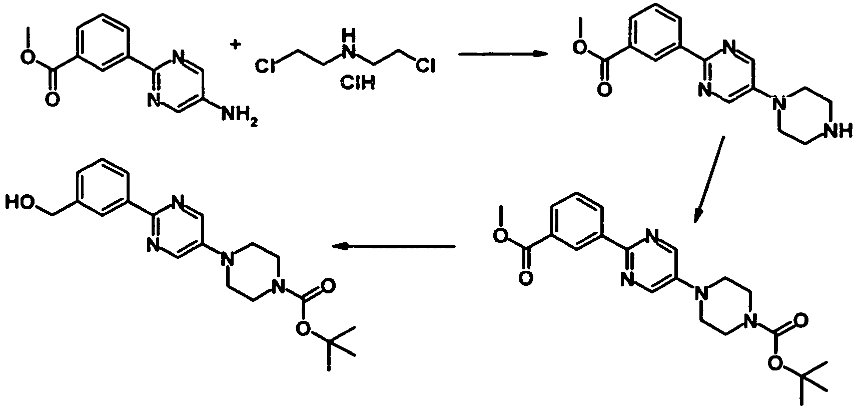

3.2 g (13.95 mmol) 3-(5-Amino-pyrimidin-2-yl)-benzoesäuremethylester werden in 80 ml NMP gelöst und mit 4.73 g (25.96 mmol) Bis(2-chlorethyl)-ammoniumchlorid und 3.13 g (23.73 mmol) Kaliumcarbonat versetzt. Die Suspension wird unter Argonatmosphäre 7 Tage bei 130°C gerührt. Das Reaktionsgemisch wird filtriert, das Filtrat wird in 1 I Diethylether eingerührt. Dabei ölt ein Rückstand aus. Die organische Phase wird abgetrennt und verworfen. Der Rückstand wird mit 500 ml Ethylacetat und 200 ml gesättigter Natriumhydrogencarbonatlösung versetzt, die organische Phase abgetrennt und die wässrige Phase nochmals mit 500 ml Ethylacetat extrahiert. Die organischen Phasen werden vereinigt, über Natriumsulfat getrocknet und eingedampft. Der Rückstand wird ohne weitere Aufarbeitung weiter umgesetzt; Ausbeute: 2.4 g; HPLC: Rt. = 2.07 min (Methode B); ESI: 299 (M+H).

2.4 g (5.4 mmol) 3-(5-Piperazin-1-yl-pyrimidin-2-yl)-benzoesäuremethylester werden in 15 ml DMF gelöst, mit 2.98 g (21.6 mmol) Kaliumcarbonat und 1.5 ml (7.0 mmol) Di-*tert.*-butyldicarbonat versetzt und 30 min bei Raumtemperatur gerührt. Das Reaktionsgemisch wird filtriert und das Filtrat eingeengt. Der Rückstand wird in 200 ml Ethylacetat und 50 ml gesättigter Natriumhydrogencarbonatlösung aufgenommen. Die organische Phase wird abgetrennt und mit 50 ml 1 N HCl gewaschen, über Natriumsulfat getrocknet und eingeengt. Das Produkt wird ohne weitere Aufreinigung weiter umgesetzt; Ausbeute: 1.1 g; HPLC: 3.18 min (Methode B); ESI: 399 (M+H).

862 mg (2.16 mmol) 4-[2-(3-Methoxycarbonyl-phenyl)-pyrimidin-5-yl]-piperazin-1-carbonsäure-tert.-butylester werden in 15 ml THF gelöst und bei Raumtemperatur mit 10.8 ml (10.8 mmol) 1 M Diisobutylaluminiumhydrid in THF versetzt. Das Reaktionsgemisch wird 1 h bei Raumtemperatur gerührt. Das Reaktionsgemisch wird unter Eiskühlung mit 3 ml gesättigter Natriumsulfatlösung versetzt. Das gelartige Gemisch wird mit 30 ml Dichlormethan und 5 ml Methanol versetzt, 10 min gerührt und über Kieselgur abgesaugt. Das Filtrat wird über Natriumsulfat getrocknet und eingedampft. Der Rückstand wird in Dichlormethan gelöst, filtriert und das Filtrat eingedampft. Das Produkt wird ohne weitere Aufreinigung weiter umgesetzt; Ausbeute: 677 mg 4-[2-(3-Hydroxymethyl-phenyl)-pyrimidin-5-yl]-piperazin-1-carbonsäure-*tert*.-butylester; HPLC: 2.66 min (Methode B); ESI: 371 (M+H).

### 14.2

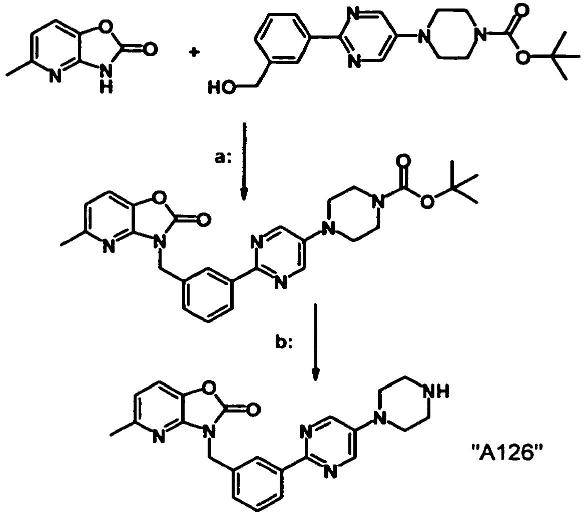

### Herstellung von 5-Methyl-3-[3-(5-piperazin-1-yl-pyrimidin-2-yl)-benzyl]-3H-oxazolo[4,5-b]pyridin-2-on:

### Stufe a:

67 mg (0.44 mmol) 5-Methyl-3H-oxazolo[4,5-b]pyridin-2-on und 163 mg (0.44 mmol) 4-[2-(3-Hydroxymethyl-phenyl)-pyrimidin-5-yl]-piperazin-1-carbonsäure-tert.-butylester werden mit 222 mg (0.67 mmol) polymergebundenem Triphenylphosphin (ca. 3 mmol Triphenylphosphin pro g) in 5 ml DMF suspendiert und 30 min bei Raumtemperatur geschüttelt. Es werden 156 mg (0.67 mmol) Di-tert.-butylazodicarboxylat zugegeben. Das Reaktionsgemisch wird 15 h bei Raumtemperatur geschüttelt. Das Reaktionsgemisch wird filtriert, der Rückstand eingedampft und der Rückstand mittels Säulenchromatographie an Kieselgel aufgereinigt.
Produkt: 80 mg; HPLC: Rt. = 3.10 min (Methode B);
ESI: 503 (M+H), 403 (M-Boc+H).

### Stufe b:

80 mg (0.16 mmol) 4-{2-[3-(5-Methyl-2-oxo-oxazolo[4,5-b]pyridin-3-ylmethyl)-phenyl]-pyrimidin-5-yl}piperazin-1-carbonsäure-*tert*.-butylester werden in 6 ml Acetonitril gelöst und mit 6 ml 4 M HCl in Dioxan versetzt. Das Reaktionsgemisch wird 1 h bei Raumtemperatur gerührt und eingeengt. Der Rückstand wird in Wasser und Ethylacetat aufgenommen, die Wasserphase mit NaOH auf pH 12 gebracht und mit Ethylacetat und Dichlormethan extrahiert. Die organischen Phasen werden vereinigt, über Natriumsulfat getrocknet und mittels Säulenchromatographie aufgereinigt. Ausbeute: 44 mg "A126"; HPLC: Rt. = 2.23 min (Methode B); ESI: 403 (M+H).

Analog werden nachstehende Verbindungen erhalten

| Nr. | Name und/oder Struktur | LCMS-Retentions zeit [min] /LCMS-Masse [M+H]⁺ / F. [°C] |
|---|---|---|
| "A46" | 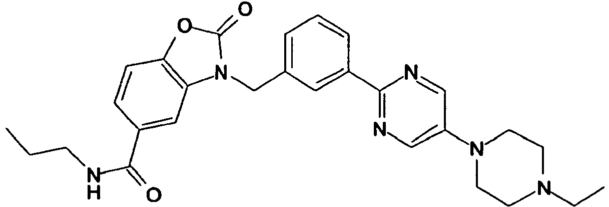 | |
| "A47" | 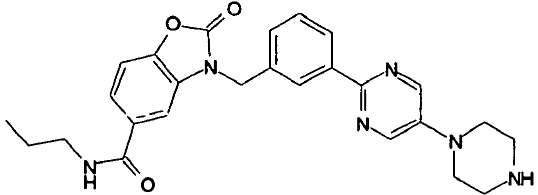 | |
| "A48" | 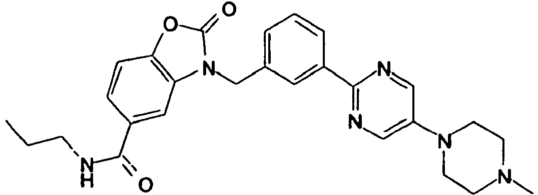 | |

### Beispiel 15

Die Herstellung von 3-{3-[5-(3-Dimethylamino-propoxy)-pyrimidin-2-yl]-benzyl}-2-oxo-2,3-dihydro-benzoxazol-5-carbonsäurepropylamid Hydrochlorid ("A49") erfolgt analog nachstehendem Schema

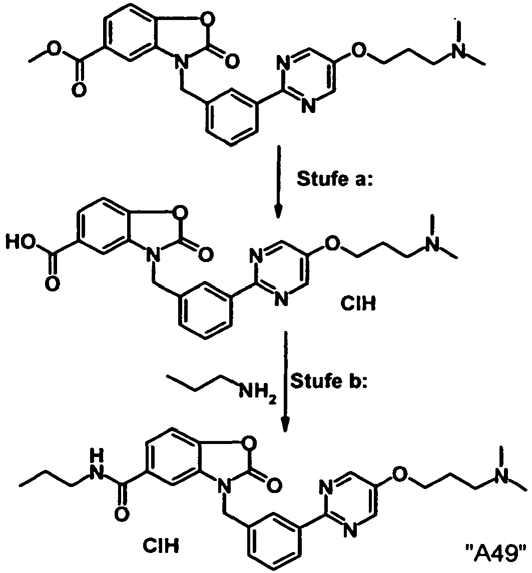

### Stufe a:

### Herstellung von 3-{3-[5-(3-Dimethylamino-propoxy)-pyrimidin-2-yl]-benzyl}-2-oxo-2,3-dihydro-benzoxazol-5-carbonsäure Hydrochlorid:

886 mg (1.92 mmol) 3-{3-[5-(3-Dimethylamino-propoxy)-pyrimidin-2-yl]-benzyl}-2-oxo-2,3-dihydro-benzoxazol-5-carbonsäure werden in 18 ml Wasser suspendiert und mit 18 ml konz. HCl versetzt. Das Reaktionsgemisch wird 2 h bei 130°C gerührt. Das Reaktionsgemisch wird zum Rückstand eingeengt, im Vakuum getrocknet und ohne weitere Aufreinigung weiter umgesetzt:
Produkt: 1.0 g. Das Produkt liegt als Hydrochlorid vor.
ESI: 449 (M+H), Rt. = 2.77 min (Methode C).

### Stufe b:

### Herstellung von 3-{3-[5-(3-Dimethylamino-propoxy)-pyrimidin-2-yl]-benzyl}-2-oxo-2,3-dihydro-benzoxazol-5-carbonsäurepropylamid Hydrochlorid:

485 mg (1 mmol) 3-{3-[5-(3-Dimethy)amino-propoxy)-pyrimidin-2-yl]-benzyl}-2-oxo-2,3-dihydro-benzoxazol-5-carbonsäure Hydrochlorid werden in 4 ml DMF gelöst und mit 387 mg (2 mmol) EDCI, 139 mg (1 mmol) HOBt und 516 µL (5 mmol) N-Methylmorpholin versetzt. Anschließend werden 71 mg (1.2 mmol) Propylamin zugegeben und die Reaktionslösung wird 3 Tage bei Raumtemperatur gerührt. Anschließend wird das Reaktionsgemisch auf Wasser gegeben und mit Dichlormethan extrahiert. Die organische Phase wird über Natriumsulfat getrocknet, zum Rückstand eingedampft und mittels Säulenchromatographie an Kieselgel aufgereinigt. Das Produkt wird in Methanol aufgenommen, mit etherischer HCl versetzt und zum Rückstand eingeengt. Man erhält 349 mg "A49" Hydrochlorid;
ESI 490 (M+H); HPLC: Rt.= 2,67 min (Methode C);
¹H-NMR (DMSO-d₆, δ in ppm): 8.658 (S, 2H), 8.487 (SB, 1H), 8.320 (S, 1 H), 8.260 (M, 1H), 7.746 (D, 1 H), 7.701 (DD, 1 H), 7.520 (D, 2H), 7.465 (D, 1 H), 5.197 (S, 2H), 4.309 (T, 2H), 3.210 (M, 4H), 2.782 (D, 6H), 2.211 (M, 2H), 1.517 (M, 2H), 0.867 (T, 3H).

Analog werden folgende Verbindungen hergestellt. In einigen Fällen werden die Rohprodukte mittels präparativer HPLC aufgereinigt.

| Nr. | Struktur | ESI (M+H) | Rt. in min |
|---|---|---|---|
| "A127" | 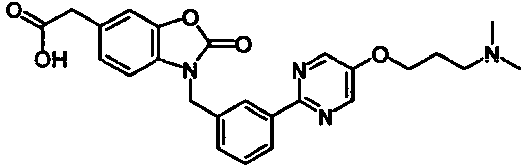 | 463 | 2.21 (Methode B) |
| "A128" | 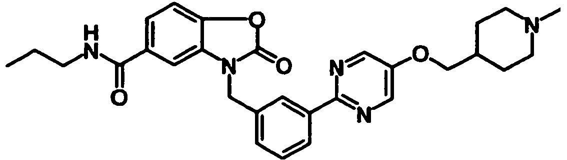 | 516 | 4.05 (Methode C) |
| | Hydrochlorid | | |
| "A129" | 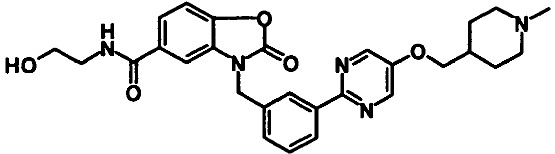 | 518 | 3.63 (Methode C) |
| | Hydrochlorid | | |
| "A130" | 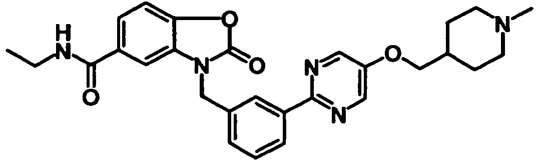 | 502 | 3.89 (Methode C) |
| | Hydrochlorid | | |
| "A131" | 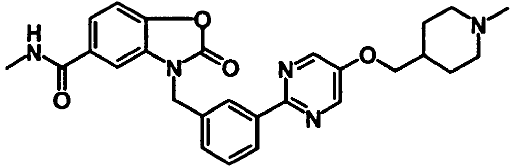 | 489 | 3.81 (Methode C) |
| | Hydrochlorid | | |
| "A132" | 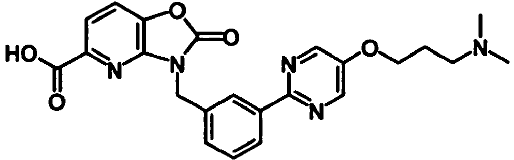 | 450 | |
| "A133" | 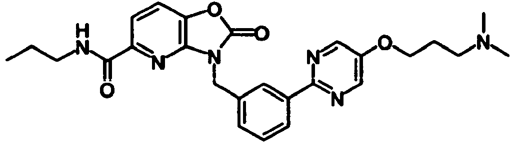 | 491 | |
| "A134" | 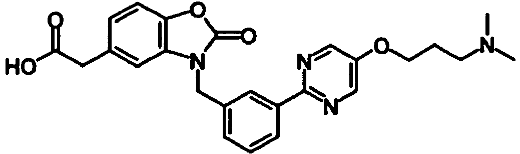 | 463 | |
| "A135" | 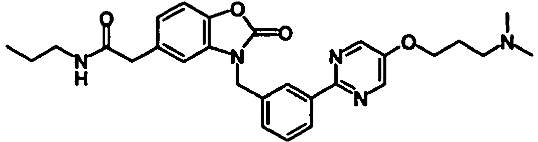 | 504 | |
| "A50" | 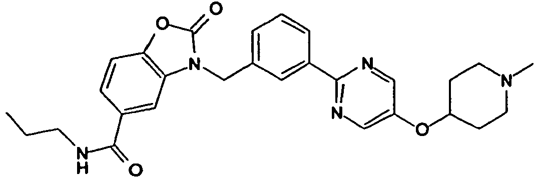 | | |
| "A51" | 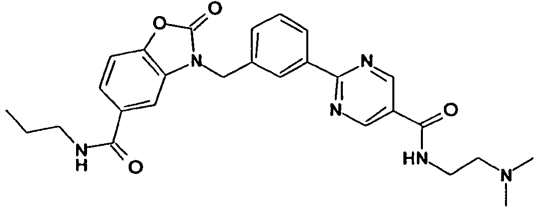 | | |
| "A52" | 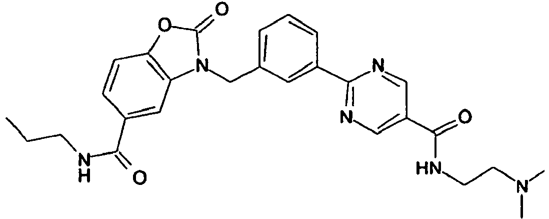 | | |

### Beispiel 16

Die Herstellung von 3-{3-[5-(3-Dimethylamino-propoxy)-pyrimidin-2-yl]-benzyl}-6-(1-hydroxy-ethyl)-3H-benzooxazol-2-on ("A136") erfolgt analog nachstehendem Schema

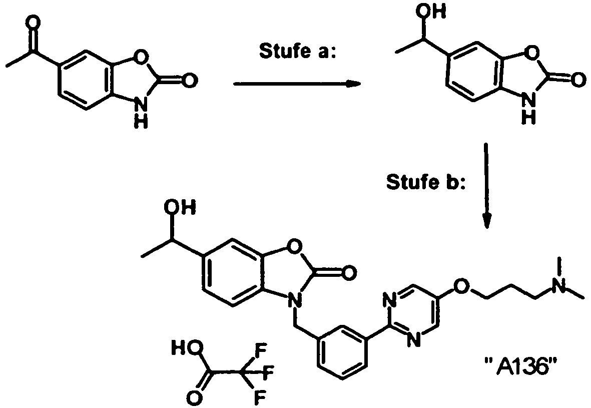

### Stufe a:

### Herstellung von 6-(1-Hydroxy-ethyl)-3H-benzooxazol-2-on:

2.5 g (14.1 mmol) 6-Acetyl-3H-benzoxazol-2-on werden in 150 ml Methanol gelöst, mit 2.5 g Palladium auf Aktivkohle (5%) und unter Wasserstoffatmosphäre 5 h gerührt. Der Katalysator wird abgesaugt und mit Methanol nachgewaschen. Das Filtrat wird zum Rückstand eingeengt und im Vakuum getrocknet.

### Stufe b:

### Herstellung von 3-{3-[5-(3-Dimethylamino-propoxy)-pyrimidin-2-yl]-benzyl}-6-(1-hydroxy-ethyl)-3H-benzoxazol-2-on:

Eine Lösung von 150 mg (0.52 mmol) {3-[5-(3-Dimethylamino-propoxy)-pyrimidin-2-yl]-phenyl}-methanol in 6 ml THF wird mit 94 mg (0.52 mmol) 6-(1-Hydroxy-ethyl)-3H-benzoxazol-2-on und 261 mg (0.78 mmol) polymergebundenem Triphenylphosphin (3mmol/g) versetzt. Die Suspension wird 30 min bei Raumtemperatur geschüttelt. Zu der Suspension werden 212 mg (0.90 mmol) Di-tert.-butylazodicarboxylat zugegeben. Nach 24 h Schütteln bei Raumtemperatur werden nochmals 261 mg (0.78 mmol) polymergebundenes Triphenylphosphin (3mmol/g) und 212 mg (0.90 mmol) Di-tert.-butylazodicarboxylat zugegeben und weitere 24 h bei Raumtemperatur geschüttelt. Das Reaktionsgemisch wird filtriert, das Filtrat zum Rückstand eingedampft und der Rückstand mittels präparativer HPLC aufgereinigt.
Man erhält 33 mg "A136" Trifluoracetat; ESI: 449 (M+H); Rt. = 2.20 min (Methode B);
¹H-NMR (DMSO-d₆, δ in ppm): 9.42 (1H, b); 8.65 (2H, s); 8.30 (1H, b); 8.26 (1H, m); 7.48-7.53 (2H, m); 7.33 (1H, s); 7.15 (2H, d); 5.17 (1H, b), 5.13 (2H, s); 4.72 (1 H, m); 4.27 (2H, t); 3.26 (2H, t); 2.83 (6H, s); 2.15 (2H, m), 1.30 (3H, d).

Analog wird folgende Verbindung hergestellt

| Nr. | Name und/oder Struktur | ESI (M+H) | Rt. in min |
|---|---|---|---|
| "A137" | 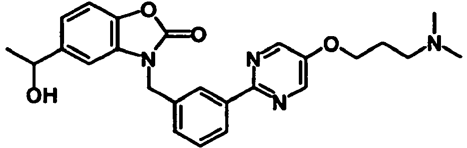 | 449 | 2.25 (Methode B) |
| | Trifluoracetat | | |

### Beispiel 17

Die Herstellung von N-[3-(6-Chlor-2-oxo-benzooxazol-3-ylmethyl)-phenyl]-acetamid ("A138") erfolgt analog nachstehendem Schema

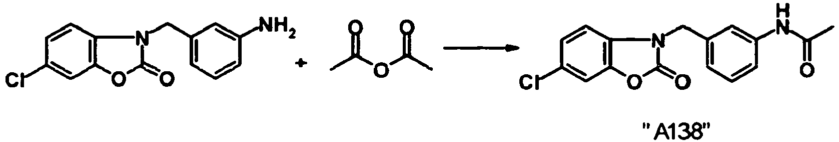

300 mg (1.09 mmol) 3-(3-Aminobenzyl)-6-chlor-3H-benzoxazol-2-on werden in 3 ml Tetrahydrofuran gelöst, mit 134 µl (1.42 mmol) Essigsäureanhydrid und 303 µl (2.18 mmol) Triethylamin versetzt und 2 h bei Raumtemperatur stehen gelassen, wobei sich ein Niederschlag bildet. Das Reaktionsgemisch wird mit Wasser verdünnt, der Niederschlag abgesaugt und mit Wasser gewaschen. Der Rückstand wird mit Ether verrieben, abgesaugt und getrocknet.
Man erhält 272 mg "A138"; F. 209-210 °C; ESI: 317 (M+H); HPLC: Rt. = 4,43 min (Methode C);
¹H-NMR (DMSO-d₆, δ in ppm): 9.920 (SB, 1H), 7.573 (M, 2H), 7.483 (SB, 1 H), 7.272 (M, 2H), 7.173 (D, 1 H), 7.051 (D, 1 H), 5.015 (S, 2H), 2.001 (S, 3H).

### Beispiel 18

Die Herstellung von 1-[3-(6-Chlor-2-oxo-benzoxazol-3-ylmethyl)-phenyl]-3-[3-(4-methyl-piperazin-1-yl)-propyl]-harnstoff ("A139") erfolgt analog nachstehendem Schema

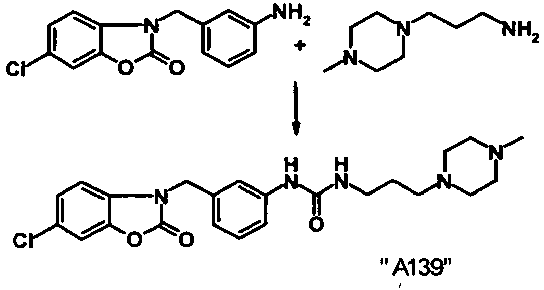

300 mg (1.09 mmol) 3-(3-Aminobenzyl)-6-chlor-3H-benzoxazol-2-on werden in 6 ml Acetonitril gelöst, mit 227 mg (1.09 mmol) 4-Nitrophenylchlorformiat und 88 µl (1.09 mmol) Pyridin versetzt und 40 Minuten bei Raumtemperatur gerührt. Anschließend werden 381 mg (2.73 mmol) Kaliumcarbonat und 174 µl (1.64 mmol) 1-(3-Aminopropyl)-4-methylpiperazin zugegeben und 24 h bei 70°C gerührt. Das Reaktionsgemisch wird in 50 ml Wasser gegossen, mit 3 x 100 ml Dichlormethan extrahiert, die vereinigten Dichlormethanphasen über Natriumsulfat getrocknet, zum Rückstand eingeengt. Das Rohprodukt wird mittels Säulenchromatographie an Kieselgel aufgereinigt. Das Produkt wird in Methanol gelöst, mit etherischer Salzsäure versetzt und zum Rückstand eingeengt. Das Salz wird aus Methanol/Ether kristallisiert, abgesaugt und getrocknet.
Man erhält 256 mg "A139" Dihydrochlorid; F. 246°C; ESI: 458 (M+H);
HPLC: Rt. = 2,80 min (Methode C);
¹H-NMR (DMSO-d₆, δ in ppm): 8.868 (SB, 1H), 7.594 (D, 1H), 7.361 (M, 2H), 7.265 (M, 1 H), 7.188 (M, 2H), 6.906 (D, 1H), 6.499 (SB, 1 H), 4.981 (S, 2H) , 3.888 - 3.218 (M, 6H), 3.153 (D, 4H), 2.821 (SB, 3H), 1.857 (SB, 2H).

Analog wird folgende Verbindung hergestellt

| Nr. | Name und/oder Struktur | ESI (M+H) | Rt. in min |
|---|---|---|---|
| "A140" | 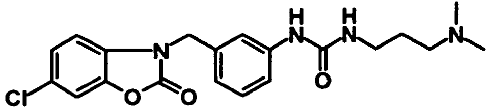 | 403 | 2.91 (Methode C) F. 182 °C |
| | Trifluoracetat | | |

### Beispiel 19

Die Herstellung von N-[3-(6-Chlor-2-oxo-benzoxazol-3-ylmethyl)-phenyl]-N'-(2-dimethylamino-ethyl)-oxalamid ("A141") erfolgt analog nachstehendem Schema

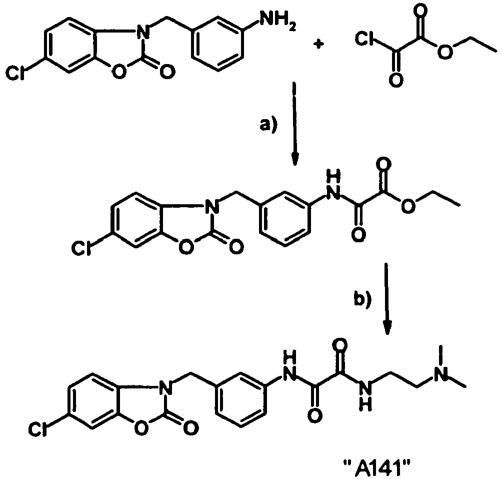

### Stufe a:

### Herstellung von N-[3-(6-Chlor-2-oxo-benzoxazol-3-ylmethyl)-phenyl]-oxalaminsäureethylester:

300 mg (1.09 mmol) 3-(3-Aminobenzyl)-6-chlor-3H-benzoxazol-2-on werden in 3 ml Dichlormethan und 115 µl (1.42 mmol) Pyridin suspendiert. Anschließend werden 124 µl (1.09 mmol) Ethylchlorformylformiat zugegeben und 30 Minuten bei Raumtemperatur gerührt, wobei eine klare Lösung entsteht. Es wird mit Dichlormethan verdünnt, mit 1 N Salzsäure und dann mit Wasser gewaschen, über Natriumsulfat getrocknet und zum Rückstand abgezogen. Das Produkt wird ohne weitere Aufreinigung direkt weiter umgesetzt; Produkt: 408 mg; ESI: 375 (M+H); HPLC: 4.32 min (Methode C).

### Stufe b:

### Herstellung von N-[3-(6-Chlor-2-oxo-benzoxazol-3-ylmethyl)-phenyl]-N'-(2-dimethylamino-ethyl)-oxalamid:

408 mg (1.09 mmol) N-[3-(6-Chlor-2-oxo-benzoxazol-3-ylmethyl)-phenyl]-oxalaminsäureethylester werden in 20 ml Ethanol suspendiert, mit 133 µl (1.20 mmol) N,N-Dimethylethylendiamin versetzt und 48 h bei Raumtemperatur gerührt. Der Niederschlag wird abgesaugt, mit Ethanol und dann mit Ether gewaschen und getrocknet. Das Rohprodukt wird in Methanol suspendiert, mit etherischer Salzsäure versetzt, wobei kurzzeitig eine fast klare Lösung entsteht, aus der das Salz sofort wieder auskristallisiert. Der Niederschlag wird abgesaugt, mit wenig Methanol und dann mit Ether gewaschen und getrocknet.
Man erhält 292 mg "A141" Hydrochlorid; F. 273 °C; ESI 417 (M+H); HPLC:
Rt. = 3.09 min (Methode C);
¹H-NMR (DMSO-d₆, δ in ppm): 10.667 (S, 1 H), 9.990 (SB, 1 H), 7.797 (M, 2H), 7.617 D, 1 H), 7.373 (T, 1 H), 7.266 (M, 1 H), 7.196 (M, 2H), 5.061 (S, 2H), 3.557 (M, 2H), 3.233 (T, 2H), 2.798 (S, 6H).

### Beispiel 20

Die Herstellung von 2-[3-(5-Methyl-2-oxo-oxazolo[4,5-b]pyridin-3-ylmethyl)-phenyl]-pyrimidin-5-carbonsäure(4-dimethylamino-butyl)-amid ("A142") erfolgt analog nachstehendem Schema

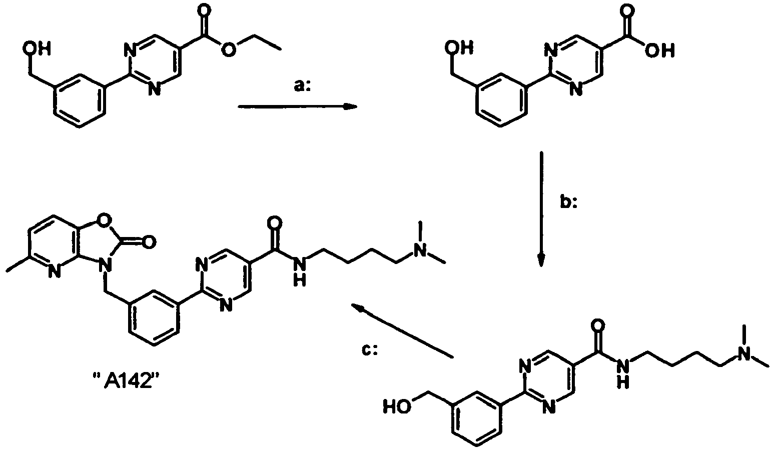

### Stufe a:

1 g (3.88 mmol) 2-(3-Hydroxymethyl-phenyl)-pyrimidine-5-carbonsäure-ethylester werden in 40 ml THF und 4 ml Wasser gelöst und mit 372 mg (15.5 mmol) Lithiumhydroxid versetzt. Das Reaktionsgemisch wird 4 h refluxiert. Anschließend wird das THF abdestilliert, die Lösung mit 1 N HCl auf pH 5 gebracht und der Feststoff wird abgesaugt, im Vakuum getrocknet und ohne weitere Aufreinigung direkt weiter umgesetzt.
ESI: 231 (M+H); Rt. = 1.98 min (Methode B).

### Stufe b:

1.4 g (6.08 mmol) 2-(3-Hydroxymethyl-phenyl)-pyrimidine-5-carbonsäure werden in 8 ml THF und 2 ml DMF gelöst und mit 1.36 ml (12.2 mmol) 4-Methylmorpholin, 1.77 g (9.12 mmol) EDCI und 1.10 g (7.91 mmol) HOBt versetzt. Es werden 919 mg (7.91 mmol) N,N-Dimethylaminobutylamin zugegeben und das Reaktionsgemisch wird 18 h bei Raumtemperatur gerührt. Die Reaktionslösung wird eingedampft, der Rückstand in Ethylacetat aufgenommen und mit 1 N NaOH und gesättigter NaCl-Lösung gewaschen. Die organische Phase wird über Natriumsulfat getrocknet und eingedampft. Das Rohprodukt wird ohne weitere Aufreinigung weiter umgesetzt.
ESI: 329; HPLC: Rt. = 1.81 min (Methode B).

### Stufe c:

Umsetzung der Edukte in einer Mitsunobu Reaktion wie oben beschrieben. Man erhält "A142"; ESI: 461 (M+H); Rt. = 2.32 min (Methode B);
¹H-NMR (DMSO-d₆, δ in ppm): 9.24 (2H, s); 8.81 (1H, t); 8.51 (1H, s); 8.38 (1 H, d); 7.66 (1H, d); 7.54-7.61 (2H, m); 7.06 (1 H, d); 5.14 (2H, s); 2.51 (überlagert, 6H, b); 2.48 (3H, s); 2.23 (2H, t); 1.57 (2H, m); 1.47 (2H, m).

### Beispiel 21

Die Herstellung von 5-Methyl-3-[3-(3-methyl-6-oxo-6H-pyridazin-1-yl)-benzyl]-3H-oxazolo[4,5-b]pyridin-2-on ("A143") erfolgt analog nachstehendem Schema

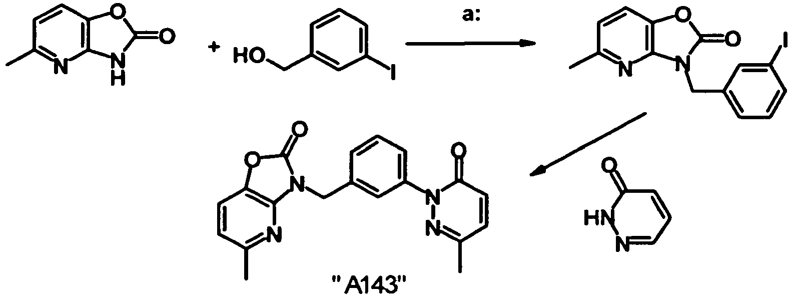

### Stufe a:

Umsetzung des 5-Methyl-3H-oxazolo[4,5-b]pyridin-2-ons mit (3-lod-phenyl)-methanol unter Mitsunobu-Bedingungen liefert 3-(3-lod-benzyl)-5-methyl-3H-oxazolo[4,5-b]pyridin-2-on; ESI: 367 (M+H).

### Stufe b:

Eine Lösung von 184 mg (0.50 mmol) 3-(3-Iod-benzyl)-5-methyl-3H-oxazolo[4,5-b]pyridin-2-on und 55.1 mg (0.5 mmol) 6-Methylpyridazin-3(2H)-on in 2 ml DMF wird mit 14.3 mg (0.08 mmol) Kupfer(I)-iodid, 76 mg (0.55 mmol) Kaliumcarbonat und 11 mg (0.08 mmol) 8-Hydroxychinolin versetzt und 24 Stunden auf 120° C erhitzt. Man lässt das Reaktionsgemisch abkühlen, versetzt es mit 10%iger wässriger Ammoniaklösung und Ethylacetat. Der entstandene Niederschlag wird abgesaugt, mit Wasser gewaschen und getrocknet. Der Rückstand wird in Ethylacetat aufgekocht, abgesaugt und mit Ethylacetat gewaschen. Der Rückstand wird im Vakuum getrocknet. Man erhält "A143", ESI 349 (M+H).

### Beispiel 22

Die Herstellung von 5-Methyl-3-[3-(5-methyl-pyridin-2-yl)-benzyl]-3H-oxazolo[4,5-b]pyridin-2-on ("A144") erfolgt analog nachstehendem Schema

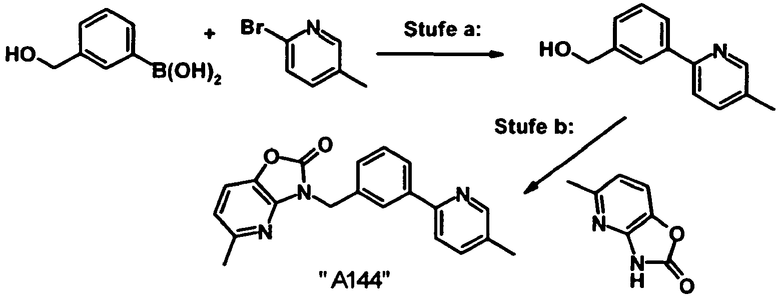

### Stufe a:

Zu einer unter Stickstoff gehaltenen Suspension von 849 mg (4.0 mmol) Trikaliumphosphat, 344 mg (2.0 mmol) 2-Brom-5-methylpyridin und 304 mg (2.0 mmol) 3-Hydroxymethylbenzolboronsäure in 12 ml Dioxan und 1 ml Wasser werden 92 mg (0.08 mmol) Tetrakis(triphenylphosphin)-palladium gegeben und das Gemisch 18 Stunden unter Rühren zum Sieden erhitzt. Das Reaktionsgemisch wird auf Raumtemperatur abgekühlt und zwischen Wasser und Ethylacetat verteilt. Die organische Phase wird über Natriumsulfat getrocknet, eingedampft und der Rückstand an einer Kieselgelsäule mit Dichlormethan/Methanol als Laufmittel chromatographiert: [3-(5-Methyl-pyridin-2-yl)-phenyl]-methanol als gelbliches Öl; ESI 200.

### Stufe b:

Umsetzung von [3-(5-Methyl-pyridin-2-yl)-phenyl]-methanol mit 5-Methyl-3H-oxazolo[4,5-b]pyridin-2-on unter Mitsunobu-Bedingungen liefert das gewünschte 5-Methyl-3-[3-(5-methyl-pyridin-2-yl)-benzyl]-3H-oxazolo[4,5-b]pyridin-2-on, ESI 332 (M+H).

Analog wird 5-Methyl-3-(3-pyrimidin-5-yl-benzyl)-3H-oxazolo[4,5-b]pyridin-2-on ("A145") hergestellt:

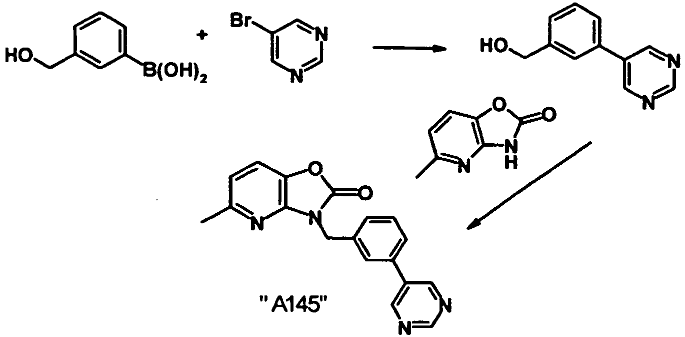

### Beispiel 23

Die Herstellung von 5-Methyl-3-[3-(4-piperazin-1-yl-pyrimidin-2-yl)-benzyl]-3H-oxazolo[4,5-b]pyridin-2-on ("A146") erfolgt analog nachstehendem Schema

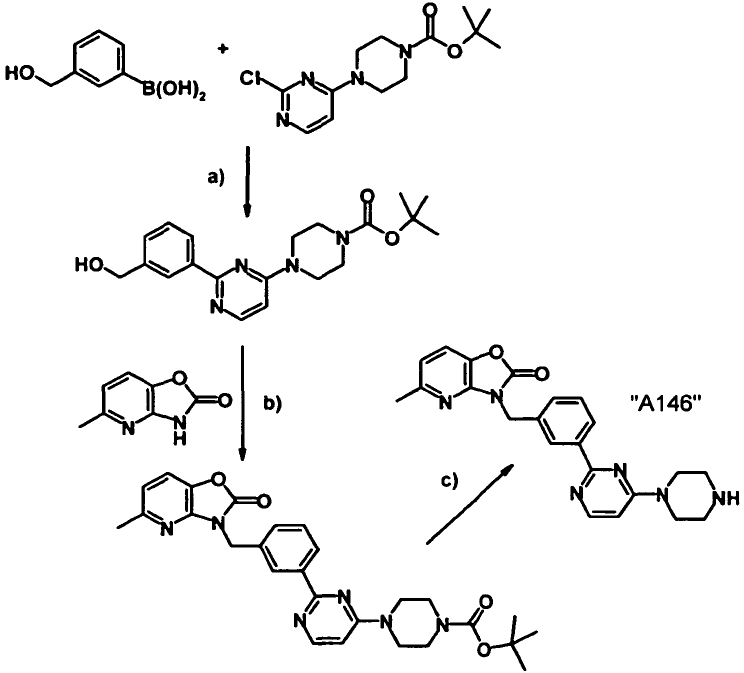

### Stufe a:

Zu einer unter Stickstoff gehaltenen Suspension von 849 mg (4.0 mmol) Trikaliumphosphat, 598 mg (2.0 mmol) 4-(2-Chlorpyrimidin-4-yl)-piperazin-1-carbonsäure-tert.-butylester (hergestellt nach WO03/104225 und 304 mg (2.0 mmol) 3-Hydroxymethylbenzolboronsäure in 12 ml Dioxan und 1 ml Wasser wird eine Katalysatorlösung, die durch Umsetzung von von 56 mg (0.08 mmol) Bis(triphenylphosphin)-palladium(II)-chlorid und 3.0 mg (0.08 mmol) Natriumborhydrid in 0.4 ml THF bei 55° C hergestellt wurde, zugegeben. Das Reaktionsgemisch wird 18 Stunden bei 97° C gerührt. Das Reaktionsgemisch wird abgekühlt und zwischen Wasser und Ethylacetat verteilt. Die organische Phase wird über Natriumsulfat getrocknet, eingedampft und der Rückstand an einer Kieselgelsäule mit Dichlormethan /Methanol als Laufmittel chromatographiert: 4-[2-(3-Hydroxymethylphenyl)-pyrimidin-4-yl]-piperazin-1-carbonsäure-tert-butylester als gelblicher Feststoff; ESI 371.

### Stufe b:

Eine Lösung von 144 mg (0.388 mmol) 4-[2-(3-Hydroxymethylphenyl)-pyrimidin-4-yl]-piperazin-1-carbonsäure-tert.-butylester, 87 mg (0.582 mmol) 5-Methyl-3H-oxazolo[4,5-b]pyridin-2-on und 153 mg (0.582 mmol) Triphenylphosphin in 3 ml THF wird mit 118 mg (0.582 mmol) Diisopropylazodicarboxylat versetzt. Das Reaktionsgemisch wird 18 Stunden bei Raumtemperatur gerührt. Es wird eingedampft und der Rückstand an einer Kieselgelsäule mit Dichlormethan/Methanol als Laufmittel chromatographiert; ESI 503 (M+H).

### Stufe c:

Eine Lösung von 70 mg (0.14 mmol) 4-{2-[3-(5-Methyl-2-oxo-oxazolo[4,5-b]pyridin-3-ylmethyl)-phenyl]-pyrimidin-4-yl}-piperazin-1-carbonsäure-tert.-butylester in 1 ml Dioxan wird mit 1.3 ml 4 N HCl in Dioxan versetzt und 18 Stunden bei Raumtemperatur belassen. Das Reaktionsgemisch wird zwischen Wasser und Ethylacetat verteilt. Die wässrige Phase wird mit 1 N NaOH auf einen pH von 14 gebracht und mit Ethylacetat extrahiert. Die organische Phase wird über Natriumsulfat getrocknet und eingedampft. Man erhält "A146" als Hydrochlorid; ESI 403 (M+H).

### Beispiel 24

Die Herstellung von 5-Methyl-3-{3-[5-(1-methy)-1H-pyrazo)-4-yl)-pyrimidin-2-yl]-benzyl}-3H-oxazolo[4,5-b]pyridin-2-on ("A147") erfolgt analog nachstehendem Schema

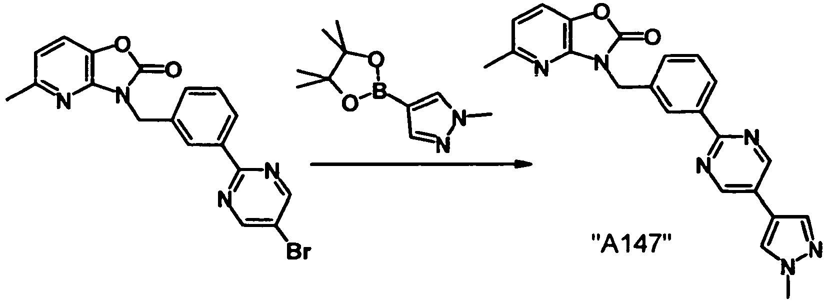

Eine unter Stickstoff gehaltene Lösung von 397 mg (1.00 mmol) 3-[3-(5-Brom-pyrimidin-2-yl)-benzyl]-5-methyl-3H-oxazolo[4,5-b]pyridin-2-on und 229 mg (1.10 mmol) 1-Methyl-4-(4,4,5,5-tetramethyl-[1,3,2]dioxaborolan-2-yl)-1 H-pyrazol in 10 ml 1,2-Dimethoxyethan wird mit 425 mg (2.0 mmol) Trikaliumphosphat-Trihydrat und 56.2 mg (0.08 mmol) Bis(triphenylphosphin)-palladiumchlorid versetzt und 18 Stunden bei 80° C gerührt, wobei sich ein grauer Niederschlag bildet. Das Reaktionsgemisch wird mit Wasser verdünnt und filtriert. Der Rückstand wird an einer Kieselgelsäule mit Dichlormethan / Methanol als Laufmittel chromatographiert. Man erhält "A147", ESI: 399 (M+H).

### Beispiel 25

Die Herstellung von 3-{3-[5-(3-Dimethylamino-propoxy)-pyrimidin-2-yl]-benzyl}-5-(1-methyl-1 H-pyrazol-4-yl)-3H-oxazolo[4,5-b]pyridin-2-on ("A148") erfolgt analog nachstehendem Schema

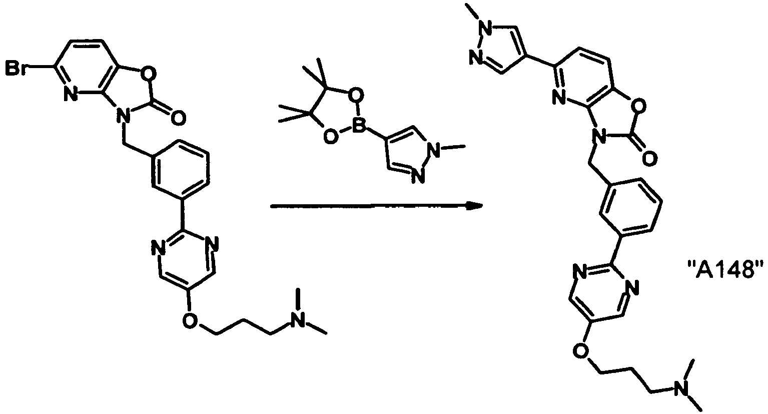

Eine unter Stickstoff gehaltene Lösung von 484 mg (1.00 mmol) 5-Brom-3-{3-[5-(3-dimethylamino-propoxy)-pyrimidin-2-yl]-benzyl}-3H-oxazolo[4,5-b]pyridin-2-on und 229 mg (1.10 mmol) 1-Methyl-4-(4,4,5,5-tetramethyl-[1,3,2]dioxaborolan-2-yl)-1 H-pyrazol in 10 ml 1,2-Dimethoxyethan wird mit 425 mg (2.0 mmol) Trikaliumphosphat-Trihydrat und 56.2 mg (0.08 mmol) Bis(triphenylphosphin)-palladiumchlorid versetzt und 18 Stunden bei 80° C gerührt. Das Reaktionsgemisch wird mit Wasser verdünnt und filtriert. Der Rückstand wird an einer Kieselgelsäule mit Dichlormethan / Methanol als Laufmittel chromatographiert. Man erhält "A148", ESI: 486 (M+H).

### Pharmakologische Daten

### Met-Kinase-Inhibierung

**Tabelle 1**

| Verbindung Nr. | IC₅₀ (Zell-Assay) |
|---|---|
| "A1" | A |
| "A2" | A |
| "A3" | A |
| "A4" | A |
| "A5" | A |
| "A6" | A |
| "A7" | A |
| "A8" | A |
| "A9" | A |
| "A10" | A |
| "A11" | A |
| "A12" | A |
| "A13" | A |
| "A14" | A |
| "A15" | A |
| "A16" | A |
| "A17" | A |
| "A18" | A |
| "A19" | A |
| "A20" | A |
| "A21" | A |
| "A22" | A |
| "A30" | A |
| "A31" | A |
| "A32" | A |
| "A33" | A |
| "A34" | A |
| "A35" | A |
| "A36" | A |
| "A37" | A |
| "A64" | A |
| "A66" | A |
| "A67" | A |
| "A76" | A |
| "A79" | A |
| "A82" | A |
| "A87" | A |
| "A92" | B |
| "A93" | B |
| "A95" | B |
| "A96" | A |
| "A103" | B |

| | |
|---|---|
| IC₅₀: 10 nM - 1 µM = A 1 µM-10 µM=B > 10 mM = C | |

Die nachfolgenden Beispiele betreffen Arzneimittel:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Nr. | Struktur und/oder Name |
|---|---|
| "A1" | [3-(5-Methoxy-2-oxo-benzoxazol-3-ylmethyl)-phenyl]-carbaminsäure-3-(4-methyl-piperazin-1-yl)-propylester |
| | 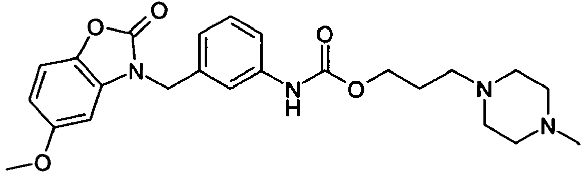 |
| "B1" | [3-(5-Methyl-2-oxo-oxazolo[4,5-b]pyridin-3-ylmethyl)-phenyl]-carbaminsäure-3-(4-methyl-piperazin-1-yl)-propylester |
| "A2" | [3-(5-Chlor-2-oxo-benzoxazol-3-ylmethyl)-phenyl]-carbaminsäure-3-(4-methyl-piperazin-1-yl)-propylester |
| | 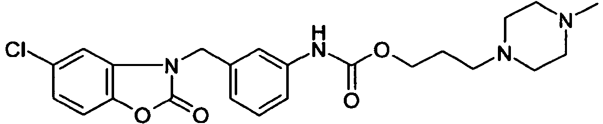 |
| | |
| "A3" | [3-(5-Dimethylcarbamoyl-2-oxo-benzoxazol-3-ylmethyl)-phenyl]-carbaminsäure-3-(4-methyl-piperazin-1-yl)-propylester |
| | 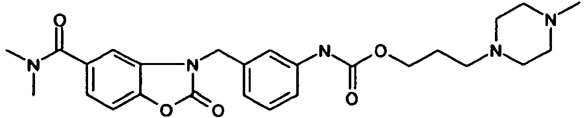 |
| "A4" | [3-(5-Dimethylcarbamoyl-2-oxo-benzoxazol-3-ylmethyl)-phenyl]-carbaminsäure-3-piperazin-1-yl-propylester |
| "A5" | [3-(5-Propylcarbamoyl-2-oxo-benzoxazol-3-ylmethyl)-phenyl]-carbaminsäure-3-(4-methyl-piperazin-1-yl)-propylester |
| "A6" | [3-(6-Chlor-2-oxo-benzoxazol-3-ylmethyl)-phenyl]-carbaminsäure-3-(4-methyl-piperazin-1-yl)-propylester |
| "A7" | [3-(5-Methyl-2-oxo-benzoxazol-3-ylmethyl)-phenyl]-carbaminsäure-3-(4-methyl-piperazin-1-yl)-propylester |
| "A8" | [3-(5-Acetyl-2-oxo-benzoxazol-3-ylmethyl)-phenyl]-carbaminsäure-3-(4-methyl-piperazin-1-yl)-propylester |
| "A9" | {3-[6-(1-Hydroxy-ethyl)-2-oxo-benzoxazol-3-ylmethyl]-phenyl}-carbaminsäure-3-(4-methyl-piperazin-1-yl)-propylester |
| | 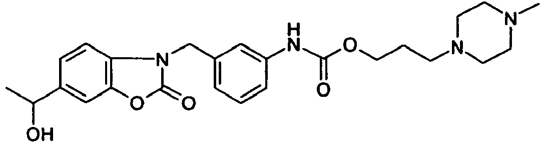 |
| "A10" | [3-(5-Propylcarbamoyl-2-oxo-benzoxazol-3-ylmethyl)-phenyl]-carbaminsäure-3-piperazin-1-yl-propylester |
| "A11" | [3-(2-Oxo-oxazolo[4,5-b]pyridin-3-ylmethyl)-phenyl]-carbaminsäure-3-dimethylamino-propylester |
| | 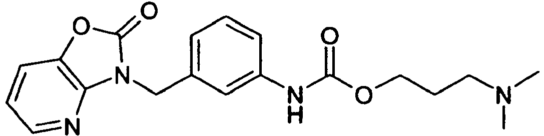 |
| "A12" | [3-(5-Dimethylcarbamoyl-2-oxo-benzoxazol-3-ylmethyl)-phenyl]-carbaminsäure-3-dimethylamino-propylester |
| "A13" | [3-(5-Propylcarbamoyl-2-oxo-benzoxazol-3-ylmethyl)-phenyl]-carbaminsäure-3-dimethylamino-propylester |
| "A14" | [3-(6-Methoxycarbonyl-2-oxo-benzoxazol-3-ylmethyl)-phenyl]-carbaminsäure-3-dimethylamino-propylester |
| "A15" | [3-(5-Methoxycarbonyl-2-oxo-benzoxazol-3-ylmethyl)-phenyl]-carbaminsäure-3-dimethylamino-propylester |
| "A16" | [3-(2-Oxo-benzoxazol-3-ylmethyl)-phenyl]-carbaminsäure-3-dimethylamino-propylester |
| "A17" | [3-(2-Oxo-benzoxazol-3-ylmethyl)-phenyl]-carbaminsäure-2-(4-methyl-piperazin-1-yl)-ethylester |
| | 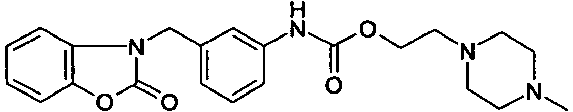 |
| "A18" | [3-(2-Oxo-benzoxazol-3-ylmethyl)-phenyl]-carbaminsäure-3-(4-methyl-piperazin-1-yl)-propylester |
| "A19" | [3-(5,6-Difluor-2-oxo-benzoxazol-3-ylmethyl)-phenyl]-carbaminsäure-3-(4-methyl-piperazin-1-yl)-propylester |
| "A20" | [3-(6-Methoxy-2-oxo-benzoxazol-3-ylmethyl)-phenyl]-carbaminsäure-3-(4-methyl-piperazin-1-yl)-propylester |
| "A21" | [3-(6-Methyl-2-oxo-benzoxazol-3-ylmethyl)-phenyl]-carbaminsäure-3-(4-methyl-piperazin-1-yl)-propylester |
| "A22" | [3-(6-Acetyl-2-oxo-benzoxazol-3-ylmethyl)-phenyl]-carbaminsäure-3-(4-methyl-piperazin-1-yl)-propylester |
| "A23" | {3-[5-(4-Methyl-piperazin-1-yl)-2-oxo-benzoxazol-3-ylmethyl]-phenyl}-carbaminsäure-ethylester |
| | 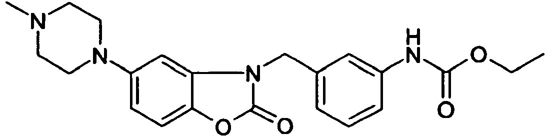 |
| "A24" | [3-(5-Cyan-2-oxo-benzoxazol-3-ylmethyl)-phenyl]-carbaminsäure-3-(4-methyl-piperazin-1-yl)-propylester |
| "A25" | [3-(5-Ethylsulfonyl-2-oxo-benzoxazol-3-ylmethyl)-phenyl]-carbaminsäure-3-(4-methyl-piperazin-1-yl)-propylester |
| "A26" | {3-[1-(5,6-Difluor-2-oxo-benzoxazol-3-yl)-ethyl]-phenyl}-carbaminsäure-3-(4-methyl-piperazin-1-yl)-propylester |
| | 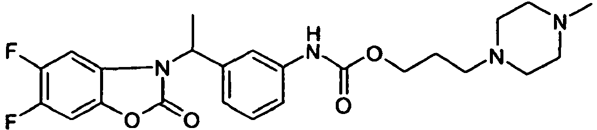 |
| "A27" | {3-[1-(5,6-Difluor-2-oxo-benzoxazol-3-yl)-ethyl]-phenyl}-carbaminsäure-2-(4-methyl-piperazin-1-yl)-ethylester |
| "A28" | {3-[5-(1-Hydroxy-ethyl)-2-oxo-benzoxazol-3-ylmethyl]-phenyl)-carbaminsäure-3-(4-methyl-piperazin-1-yl)-propylester |
| | 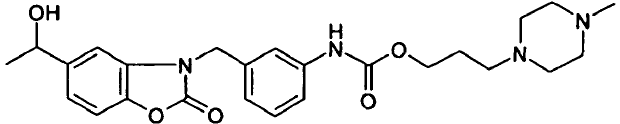 |
| "A28a" | 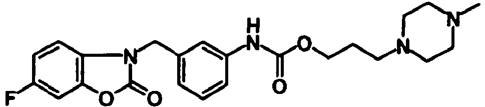 |
| "A28b" | 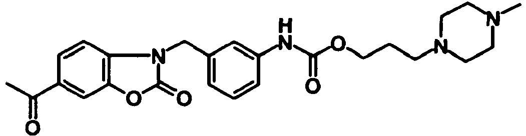 |
| "A29" | {3-[6-(2-Dimethylamino-ethylcarbamoyl)-2-oxo-benzoxazol-3-ylmethyl]-phenyl}-carbaminsäure-ethylester |
| | 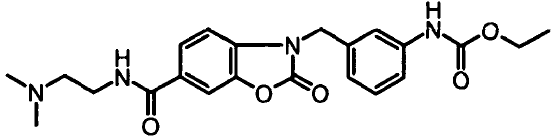 |
| "A30" | {3-[5-(2-Dimethylamino-ethylcarbamoyl)-2-oxo-benzoxazol-3-ylmethyl]-phenyl}-carbaminsäure-ethylester |
| "A31" | {3-[5-(3-Dimethylamino-propylcarbamoyl)-2-oxo-benzoxazol-3-ylmethyl]-phenyl}carbaminsäure-ethylester |
| "A32" | (3-{1-[5-(4-Dimethylamino-butylcarbamoyl)-2-oxo-benzoxazol-3-yl]-ethyl}-phenyl)-carbaminsäure-ethylester |
| | 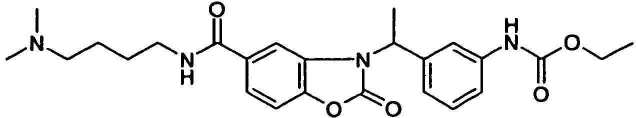 |
| "A33" | (3-{1-[5-(2-Dimethylamino-ethylcarbamoyl)-2-oxo-benzoxazol-3-yl]-ethyl}-phenyl)-carbaminsäure-ethylester |
| "A34" | 2-Oxo-3-(2-oxo-2,3-dihydro-1H-benzimidazol-5-ylmethyl)-2,3-dihydro-benzoxazol-5-carbonsäure-(4-dimethylamino-butyl)-amid |
| | 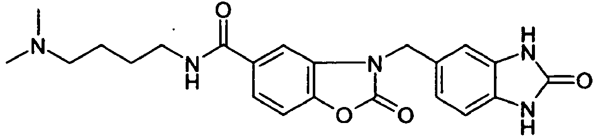 |
| "A35" | 2-Oxo-3-(2-oxo-2,3-dihydro-1H-benzimidazol-5-ylmethyl)-2,3-dihydro-benzoxazol-5-carbonsäure-(2-dimethylaminoethyl)-amid |
| "A36" | (3-{1-[5-(3-Dimethylamino-propylcarbamoyl)-2-oxo-benzoxazol-3-yl]-ethyl}-phenyl)-carbaminsäure-ethylester |
| "A37" | 3-(3-Ethoxycarbonylamino-benzyl)-2-oxo-2,3-dihydro-benzoxazol-6-carbonsäure-methylester |
| | 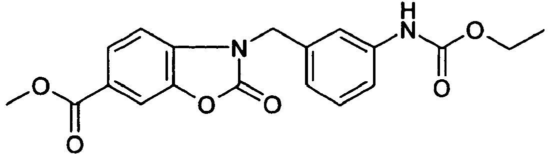 |
| "A38" | {3-[6-(3-Methylamino-propylcarbamoyl)-2-oxo-benzooxazol-3-ylmethyl]-phenyl}-carbaminsäure-ethylester |
| "A39" | {3-[6-(3-Dimethylamino-propylcarbamoyl)-2-oxo-benzoxazol-3-ylmethyl]-phenyl}-carbaminsäure-ethylester |
| "A40" | (3-{6-[3-(tert.-Butoxycarbonyl-methyl-amino)-propylcarbamoyl]-2-oxo-benzoxazol-3-ylmethyl}-phenyl)-carbaminsäure-ethylester |
| | 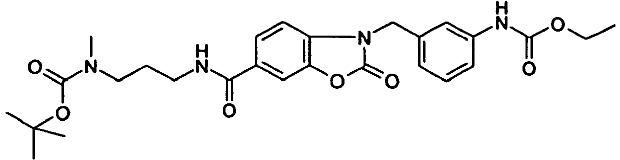 |
| "A41" | 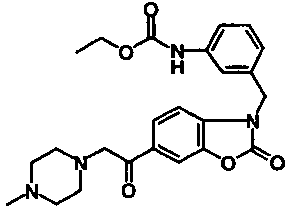 |
| "A42" | 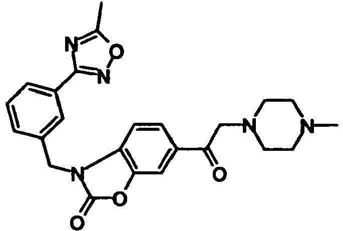 |
| "A43" | 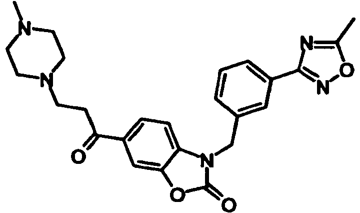 |
| "A43a" | 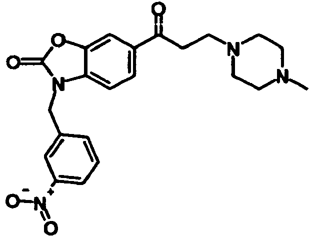 |
| "A44" | 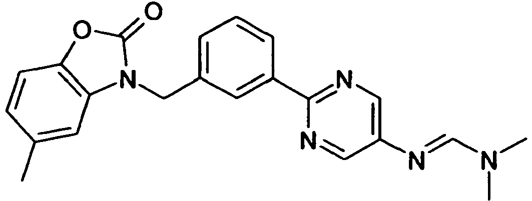 |
| "A45" | 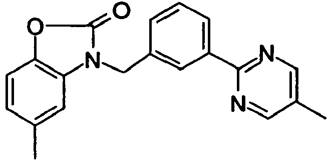 |
| "A46" | 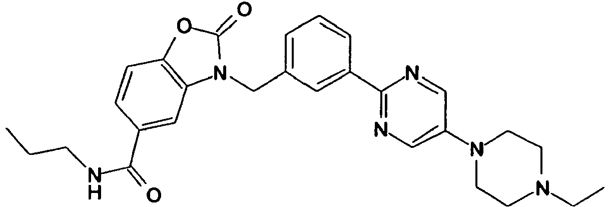 |
| "A47" | 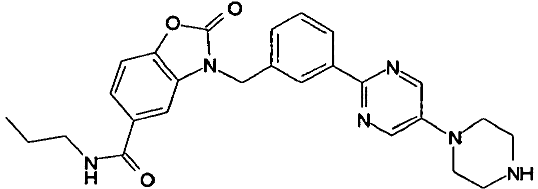 |
| "A48" | 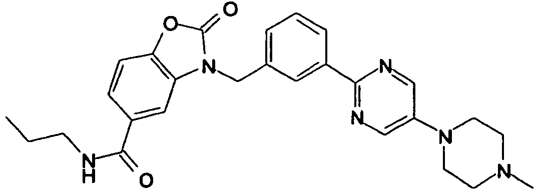 |
| "A49" | 3-{3-[5-(3-Dimethylamino-propoxy)pyrimidin-2-yl]-benzyl}-2-oxo-2,3-dihydro-benzoxazol-5-carbonsäurepropylamid |
| "A50" | 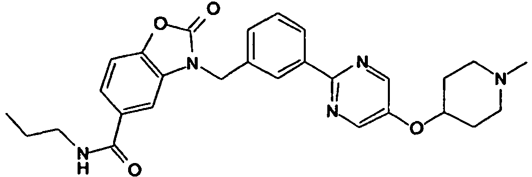 |
| "A51" | 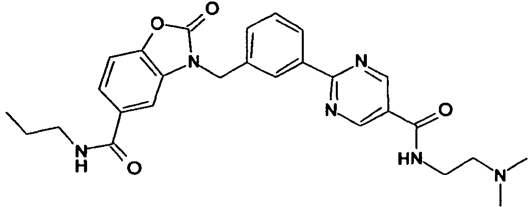 |
| "A52" | 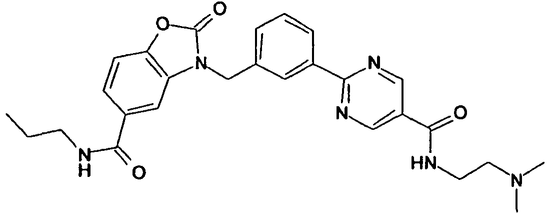 |
| "A53" | 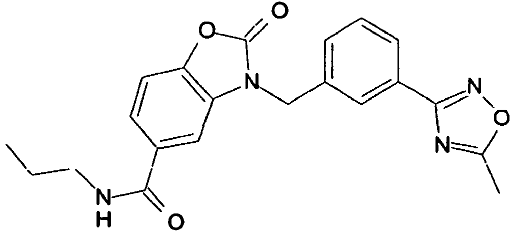 |
| "A54" | 3-{3-[6-(3-Dimethylamino-propoxy)-pyridazin-3-yl]-benzyl}-2-oxo-2,3-dihydro-benzoxazol-5-carbonsäure-propylamid |
| "A55" | 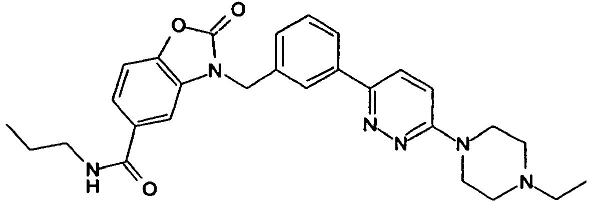 |
| "A56" | 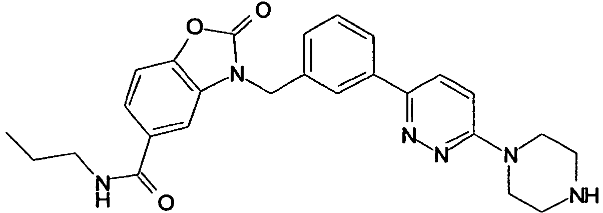 |
| "A57" | 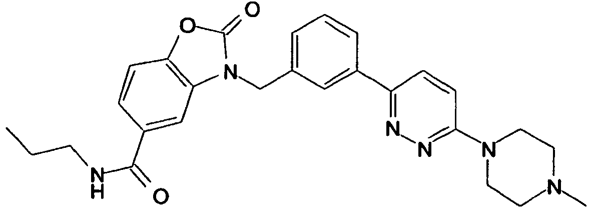 |
| "A58" | 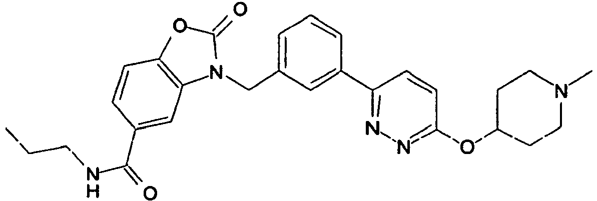 |
| "A59" | 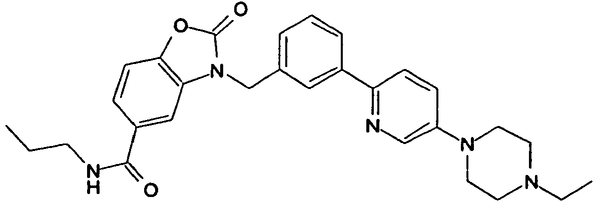 |
| "A60" | 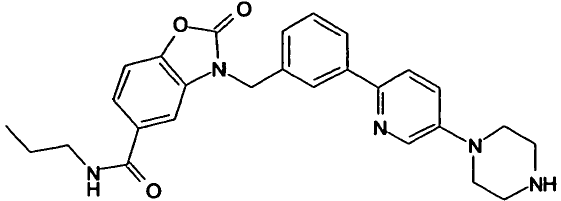 |
| "A61" | 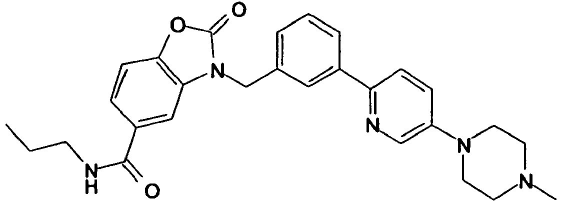 |
| "A62" | 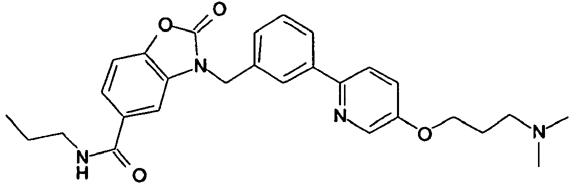 |
| "A63" | 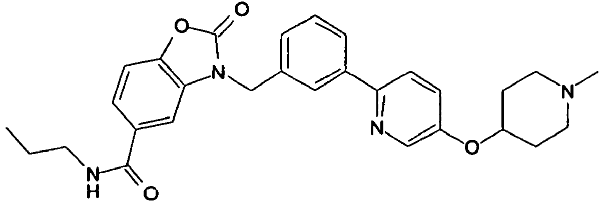 |
| "A64" | 3-{3-[5-(3-Dimethylamino-propoxy)-pyrimidin-2-yl]-benzyl}-2-oxo-2,3-dihydro-benzoxazol-5-carbonsäuremethylester |
| "A65" | 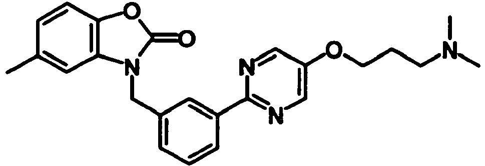 |
| "A66" | 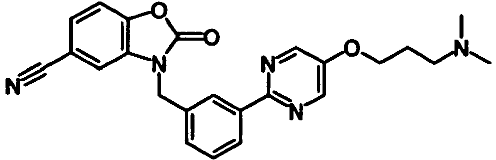 |
| "A67" | 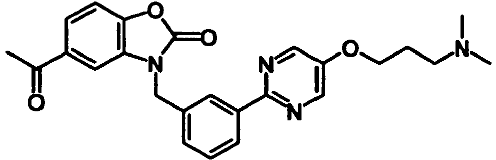 |
| "A68" | 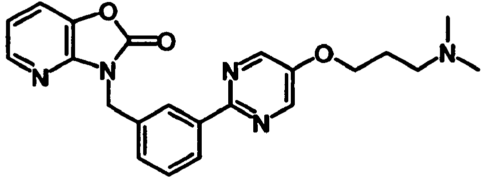 |
| "A69" | 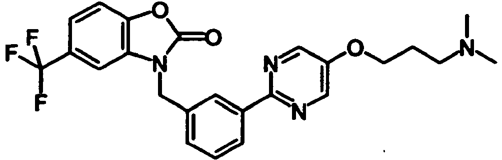 |
| "A70" | 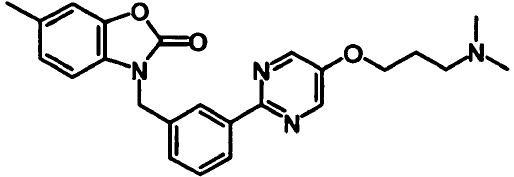 |
| "A71" | 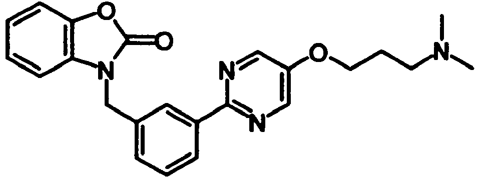 |
| "A72" | 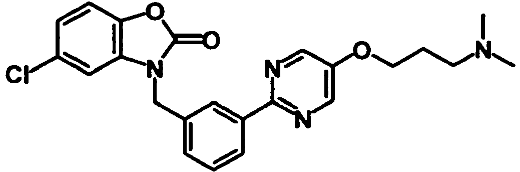 |
| "A73" | 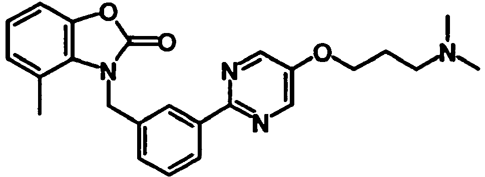 |
| "A74" | 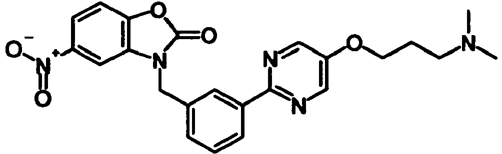 |
| "A75" | 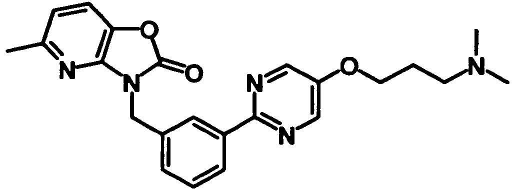 |
| "A76" | 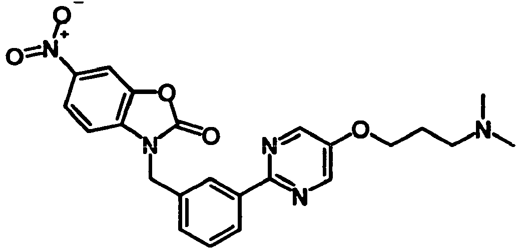 |
| "A77" | 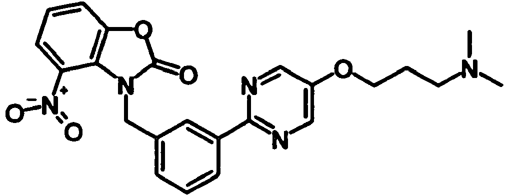 |
| "A78" | 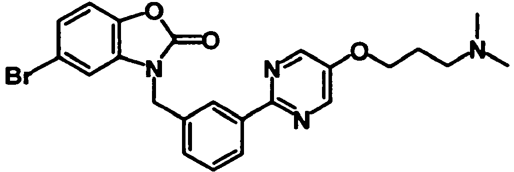 |
| "A79" | 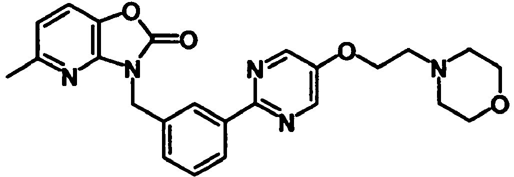 |
| "A80" | 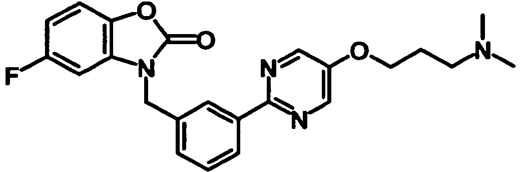 |
| "A81" | 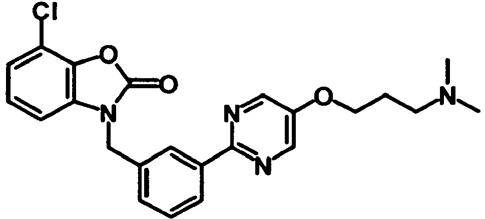 |
| "A82" | 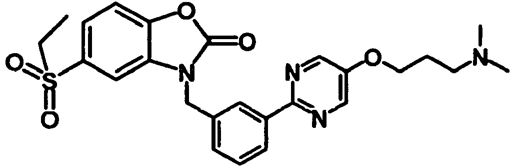 |
| "A83" | 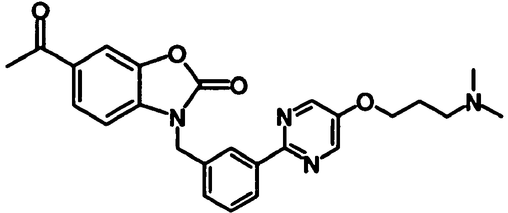 |
| "A84" | 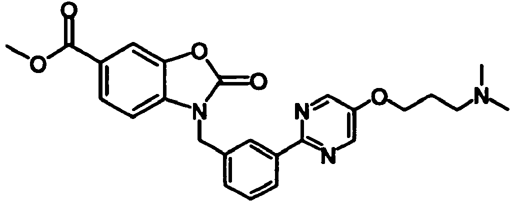 |
| "A85" | 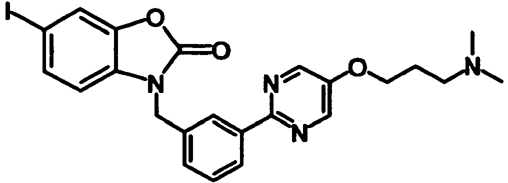 |
| "A86" | 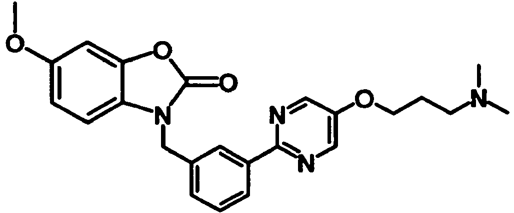 |
| "A87" | 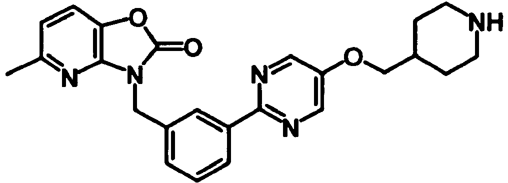 |
| "A88" | 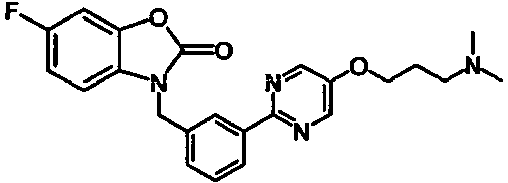 |
| "A89" | 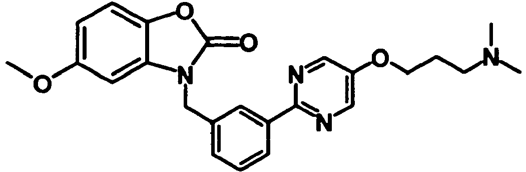 |
| "A90" | 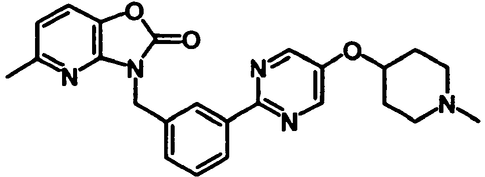 |
| "A91" | 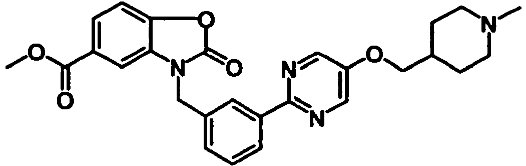 |
| "A92" | 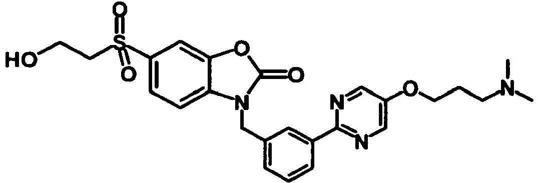 |
| "A93" | 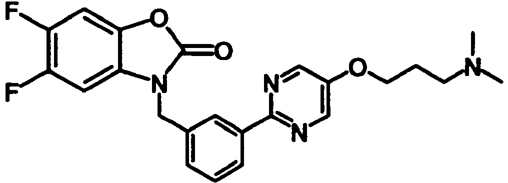 |
| "A94" | 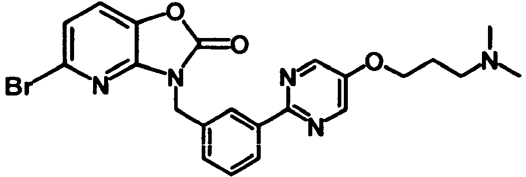 |
| "A95" | 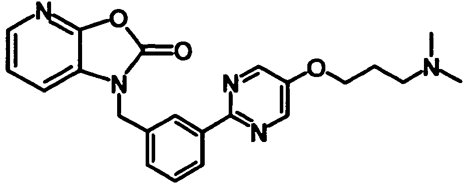 |
| "A96" | 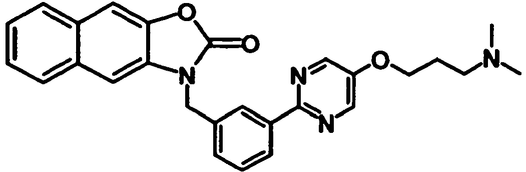 |
| "A97" | 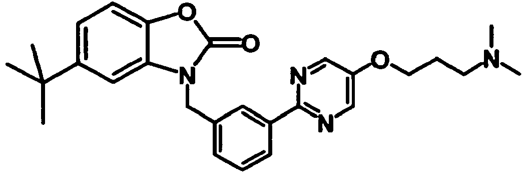 |
| "A98" | 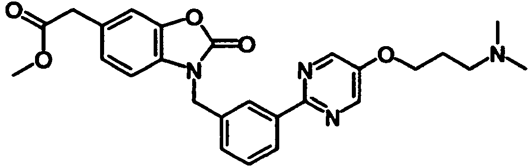 |
| "A99" | 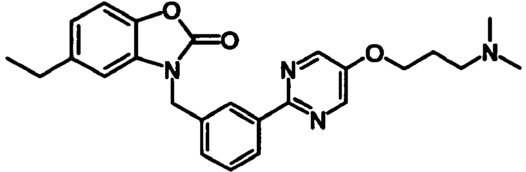 |
| "A100" | 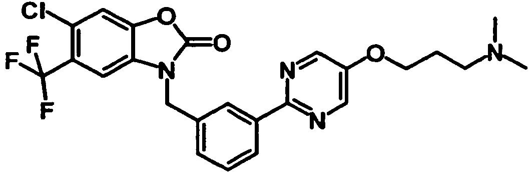 |
| "A101" | 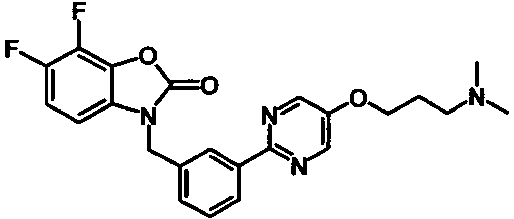 |
| "A102" | 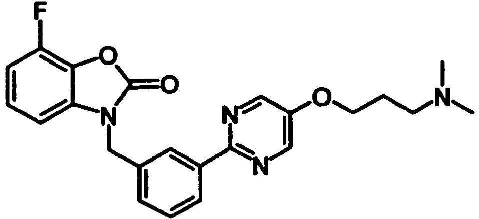 |
| "A103" | 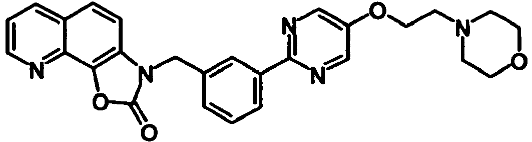 |
| "A104" | 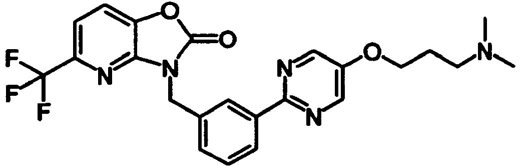 |
| "A105" | 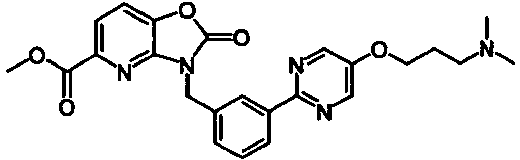 |
| "A106" | 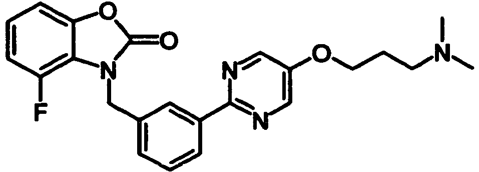 |
| "A107" | 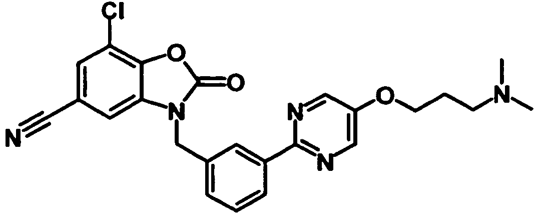 |
| "A108" | 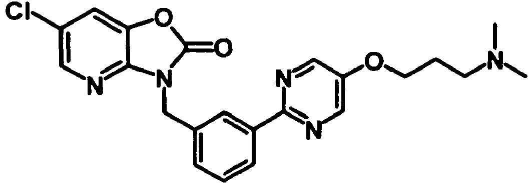 |
| "A109" | 3-{3-[5-(2-Dimethylamino-ethoxy)-pyrimidin-2-yl]-benzyl}-5-methyl-3H-oxazolo[4,5-b]pyridin-2-on |
| "A110" | 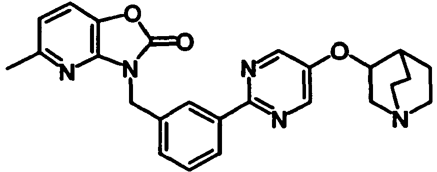 |
| "A111" | 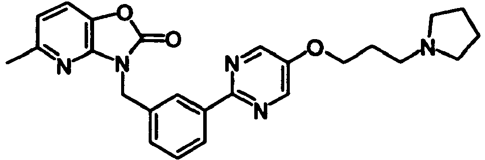 |
| "A112" | 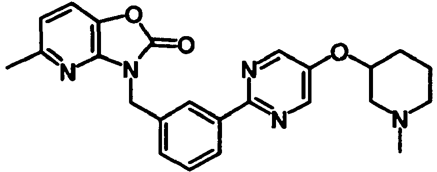 |
| "A113" | 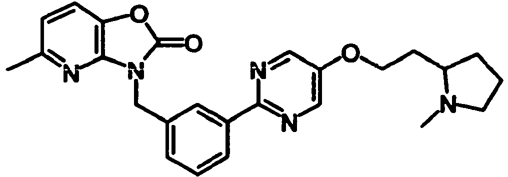 |
| "A114" | 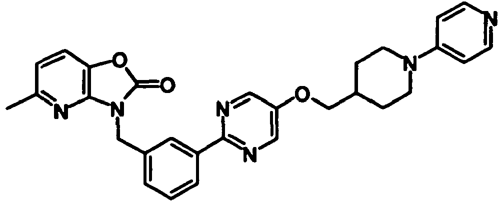 |
| "A115" | 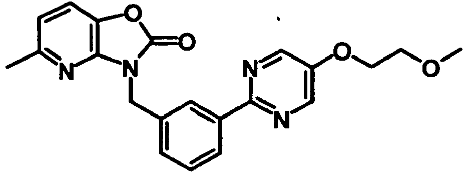 |
| "A116" | 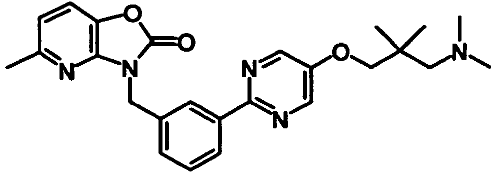 |
| "A117" | 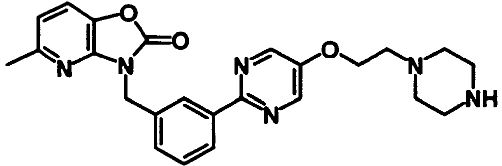 |
| "A118" | 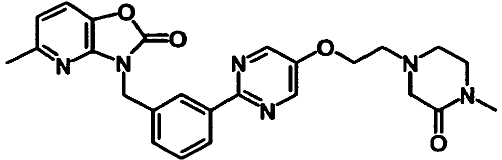 |
| "A119" | 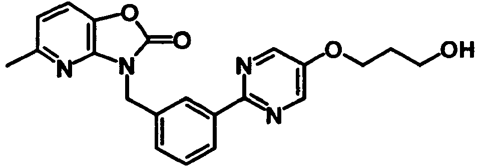 |
| "A120" | 3-{3-[5-(2,3-Dihydroxy-propoxy)-pyrimidin-2-yl]-benzyl}-5-methyl-3H-oxazolo[4,5-b]pyridin-2-on |
| "A121" | 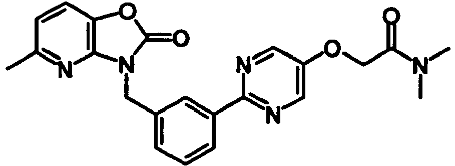 |
| "A122" | 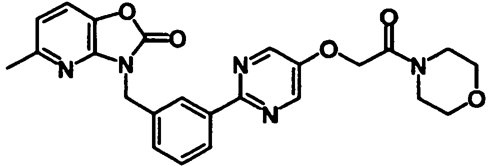 |
| "A123" | 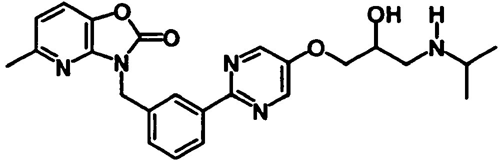 |
| "A124" | 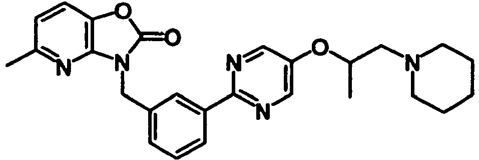 |
| "A125" | 5-Methyl-3-{3-[5-(4-methyl-piperazin-1-yl)-pyrimidin-2-yl]-benzyl}-3H-oxazolo[4,5-b]pyridin-2-on |
| "A126" | 5-Methyl-3-{3-[5-(4-methyl-piperazin-1-yl)-pyrimidin-2-yl]-benzyl}-3H-oxazolo[4,5-b]pyridin-2-on |
| "A127" | 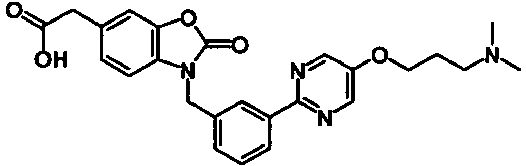 |
| "A128" | 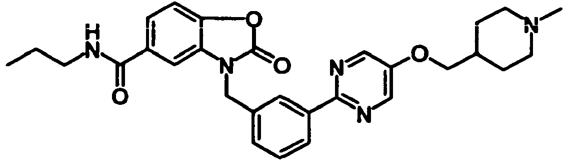 |
| "A129" | 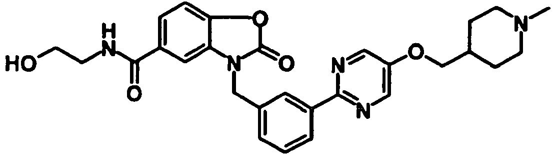 |
| "A130" | 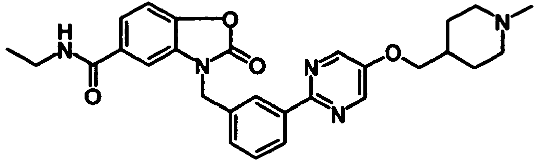 |
| "A131" | 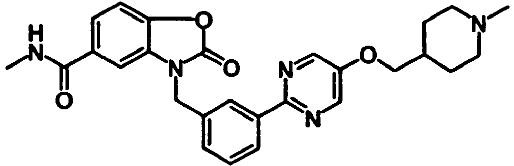 |
| "A132" | 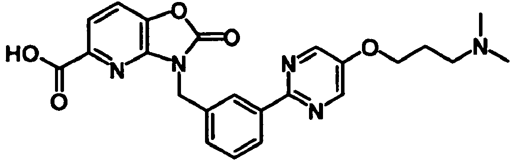 |
| "A133" | 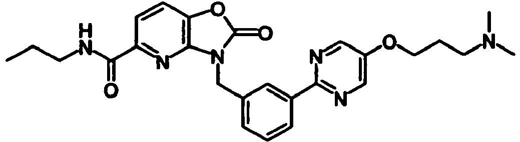 |
| "A134" | 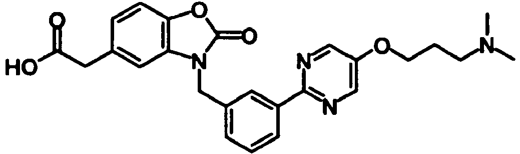 |
| "A135" | 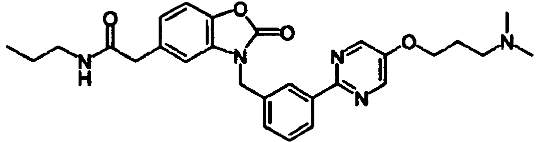 |
| "A136" | 3-{3-[5-(3-Dimethylamino-propoxy)-pyrimidin-2-yl]-benzyl}-6-(1-hydroxy-ethyl)-3H-benzooxazol-2-on |
| "A137" | 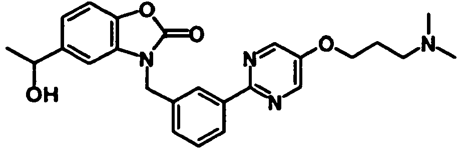 |
| "A138" | N-[3-(6-Chlor-2-oxo-benzooxazol-3-ylmethyl)-phenyl]-acetamid |
| "A139" | 1-[3-(6-Chlor-2-oxo-benzoxazol-3-ylmethyl)-phenyl]-3-[3-(4-methyl-piperazin-1-yl)-propyl]-harnstoff |
| "A140" | 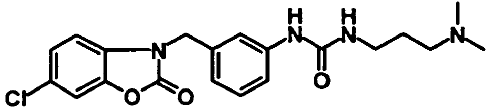 |
| "A141" | N-[3-(6-Chlor-2-oxo-benzoxazol-3-ylmethyl)-phenyl]-N'-(2-dimethylamino-ethyl)-oxalamid |
| "A142" | 2-[3-(5-Methyl-2-oxo-oxazolo[4,5-b]pyridin-3-ylmethyl)-phenyl]-pyrimidin-5-carbonsäure(4-dimethylamino-butyl)-amid |
| "A143" | 5-Methyl-3-[3-(3-methyl-6-oxo-6H-pyridazin-1-yl)-benzyl]-3H-oxazolo[4,5-b]pyridin-2-on |
| "A144" | 5-Methyl-3-[3-(5-methyl-pyridin-2-yl)-benzyl]-3H-oxazolo[4,5-b]pyridin-2-on |
| "A145" | 5-Methyl-3-(3-pyrimidin-5-yl-benzyl)-3H-oxazolo[4,5-b]pyridin-2-on |
| "A146" | 5-Methyl-3-[3-(4-piperazin-1-yl-pyrimidin-2-yl)-benzyl]-3H-oxazolo[4,5-b]pyridin-2-on |
| "A147" | 5-Methyl-3-{3-[5-(1-methyl-1 H-pyrazol-4-yl)-pyrimidin-2-yl]-benzyl}-3H-oxazolo[4,5-b]pyridin-2-on |
| "A148" | 3-{3-[5-(3-Dimethylamino-propoxy)-pyrimidin-2-yl]-benzyl}-5-(1-methyl-1 H-pyrazol-4-yl)-3H-oxazolo[4,5-b]pyridin-2-on |
sowie ihre pharmazeutisch verwendbaren Solvate, Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Solvate, Tautomeren und Stereoisomeren, einschließlich deren Mischungen in allen Verhältnissen, sowie gegebenenfalls Träger- und/oder Hilfsstoffe.

3. Verwendung von Verbindungen nach Anspruch 1
sowie ihrer pharmazeutisch verwendbaren Salze, Solvate, Tautomeren und Stereoisomeren, einschließlich deren Mischungen in allen Verhältnissen,
zur Herstellung eines Arzneimittels zur Behandlung von Krankheiten, bei denen die Hemmung, Regulierung und/oder Modulation der Signaltransduktion von Kinasen eine Rolle spielt.

4. Verwendung nach Anspruch 3 von Verbindungen gemäß Anspruch 1,
sowie ihrer pharmazeutisch verwendbaren Salze, Solvate und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

5. Verwendung nach Anspruch 4, zur Herstellung eines Arzneimittels zur Behandlung von Krankheiten, die durch Inhibierung von Met-Kinase durch die Verbindungen nach Anspruch 1 beeinflußt werden.

6. Verwendung nach Anspruch 4 oder 5, wobei die zu behandelnde Krankheit ein fester Tumor ist.

7. Verwendung nach Anspruch 6, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge stammt.

8. Verwendung nach Anspruch 6, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

9. Verwendung nach Anspruch 6, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

10. Verwendung nach Anspruch 4 oder 5, wobei die zu behandelnde Krankheit ein Tumor des Blut- und Immunsystems ist.

11. Verwendung nach Anspruch 10, wobei der Tumor aus der Gruppe der akuten myelotischen Leukämie, der chronischen myelotischen Leukämie, akuten lymphatischen Leukämie und/oder chronischen lymphatischen Leukämie stammt.

12. Arzneimittel enthaltend mindestens eine Verbindung gemäß Anspruch 1, und/oder ihre pharmazeutisch verwendbaren Salze, Solvate und Stereoisomere, einschließlich deren Mischungen in allen Verhältnissen, und mindestens einen weiteren Arzneimittelwirkstoff.

13. Set (Kit), bestehend aus getrennten Packungen von
(a) einer wirksamen Menge an einer Verbindung gemäß Anspruch 1, und/oder ihrer pharmazeutisch verwendbaren Salze, Solvate, Salze und Stereoisomere, einschließlich deren Mischungen in allen Verhältnissen,
und
(b) einer wirksamen Menge eines weiteren Arzneimittelswirkstoffs.

## Claims

1. Compounds selected from the group
| No. | Structure and/or name |
|---|---|
| "A1" | 3-(4-Methylpiperazin-1-yl)propyl [3-(5-methoxy-2-oxobenzoxazol-3-ylmethyl)phenyl]carbamate |
| | 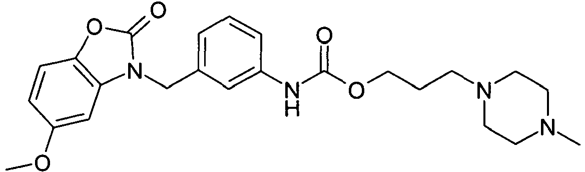 |
| "B1" | 3-(4-Methylpiperazin-1-yl)propyl [3-(5-methyl-2-oxo-oxazolo[4,5-b]pyridin-3-ylmethyl)phenyl]carbamate |
| "A2" | 3-(4-Methylpiperazin-1-yl)propyl [3-(5-chloro-2-oxobenzoxazol-3-ylmethyl)phenyl]carbamate |
| | 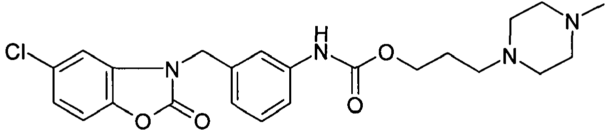 |
| | |
| "A3" | 3-(4-Methylpiperazin-1-yl)propyl [3-(5-dimethylcarbamoyl-2-oxobenzoxazol-3-ylmethyl)phenyl]carbamate |
| | 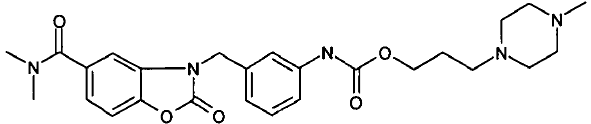 |
| "A4" | 3-Piperazin-1-ylpropyl [3-(5-dimethylcarbamoyl-2-oxobenzoxazol-3-ylmethyl)phenyl]carbamate |
| "A5" | 3-(4-Methylpiperazin-1-yl)propyl [3-(5-propylcarbamoyl-2-oxobenzoxazol-3-ylmethyl)phenyl]carbamate |
| "A6" | 3-(4-Methylpiperazin-1-yl)propyl [3-(6-chloro-2-oxobenzoxazol-3-ylmethyl)phenyl]carbamate |
| "A7" | 3-(4-Methylpiperazin-1-yl)propyl [3-(5-methyl-2-oxobenzoxazol-3-ylmethyl)phenyl]carbamate |
| "A8" | 3-(4-Methylpiperazin-1-yl)propyl [3-(5-acetyl-2-oxobenzoxazol-3-ylmethyl)phenyl]carbamate |
| "A9" | 3-(4-Methylpiperazin-1-yl)propyl {3-[6-(1-hydroxyethyl)-2-oxobenzoxazol-3-ylmethyl]phenyl}carbamate |
| | 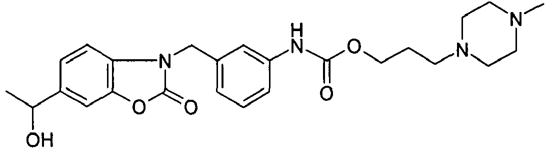 |
| "A10" | 3-Piperazin-1-ylpropyl [3-(5-propylcarbamoyl-2-oxobenzoxazol-3-ylmethyl)phenyl]carbamate |
| "A11" | 3-Dimethylaminopropyl [3-(2-oxooxazolo[4,5-b]pyridin-3-yl-methyl)phenyl]carbamate |
| | 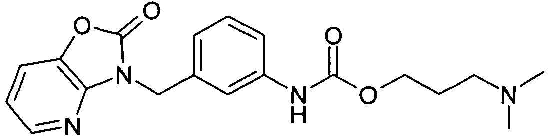 |
| "A12" | 3-Dimethylaminopropyl [3-(5-dimethylcarbamoyl-2-oxobenz-oxazol-3-ylmethyl)phenyl]carbamate |
| "A13" | 3-Dimethylaminopropyl [3-(5-propylcarbamoyl-2-oxobenz-oxazol-3-ylmethyl)phenyl]carbamate |
| "A 14" | 3-Dimethylaminopropyl [3-(6-methoxycarbonyl-2-oxobenz-oxazol-3-ylmethyl)phenyl]carbamate |
| "A15" | 3-Dimethylaminopropyl [3-(5-methoxycarbonyl-2-oxobenz-oxazol-3-ylmethyl)phenyl]carbamate |
| "A16" | 3-Dimethylaminopropyl [3-(2-oxobenzoxazol-3-ylmethyl)-phenyl]carbamate |
| "A17" | 2-(4-Methylpiperazin-1-yl)ethyl [3-(2-oxobenzoxazol-3-yl-methyl)phenyl]carbamate |
| | 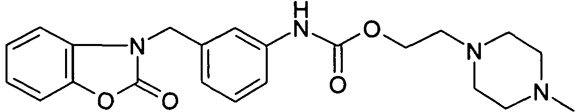 |
| "A18" | 3-(4-Methylpiperazin-1-yl)propyl [3-(2-oxobenzoxazol-3-yl-methyl)phenyl]carbamate |
| "A19" | 3-(4-Methylpiperazin-1-yl)propyl [3-(5,6-difluoro-2-oxobenz-oxazol-3-ylmethyl)phenyl]carbamate |
| "A20" | 3-(4-Methylpiperazin-1-yl)propyl [3-(6-methoxy-2-oxobenz-oxazol-3-ylmethyl)phenyl]carbamate |
| "A21" | 3-(4-Methylpiperazin-1-yl)propyl [3-(6-methyl-2-oxobenzoxa-zol-3-ylmethyl)phenyl]carbamate |
| "A22" | 3-(4-Methylpiperazin-1-yl)propyl [3-(6-acetyl-2-oxobenzoxa-zol-3-ylmethyl)phenyl]carbamate |
| "A23" | Ethyl {3-[5-(4-methylpiperazin-1-yl)-2-oxobenzoxazol-3-yl-methyl]phenyl}carbamate |
| | 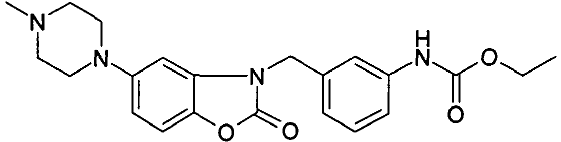 |
| "A24" | 3-(4-Methylpiperazin-1-yl)propyl [3-(5-cyano-2-oxobenzoxa-zol-3-ylmethyl)phenyl]carbamate |
| "A25" | 3-(4-Methylpiperazin-1-yl)propyl [3-(5-ethylsulfonyl-2-oxo-benzoxazol-3-ylmethyl)phenyl]carbamate |
| "A26" | 3-(4-Methylpiperazin-1-yl)propyl {3-[1-(5,6-difluoro-2-oxo-benzoxazol-3-yl)ethyl]phenyl}carbamate |
| | 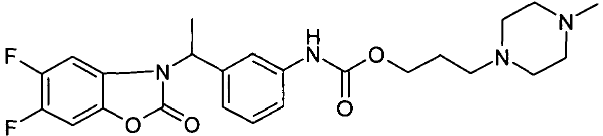 |
| "A27" | 2-(4-Methylpiperazin-1-yl)ethyl {3-[1-(5,6-difluoro-2-oxo-benzoxazol-3-yl)ethyl]phenyl}carbamate |
| "A28" | 3-(4-Methylpiperazin-1-yl)propyl {3-[5-(1-hydroxyethyl)-2-oxobenzoxazol-3-ylmethyl]phenyl}carbamate |
| | 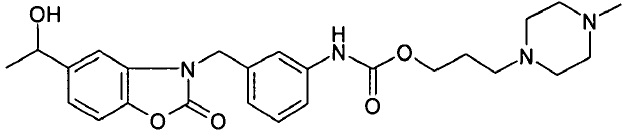 |
| "A28a" | 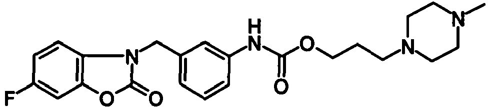 |
| "A28b" | 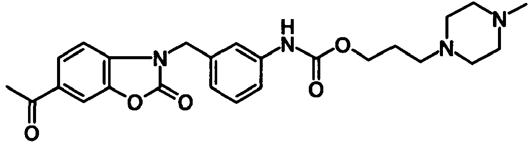 |
| "A29" | Ethyl {3-[6-(2-dimethylaminoethylcarbamoyl)-2-oxobenzoxa-zol-3-ylmethyl]phenyl}carbamate |
| | 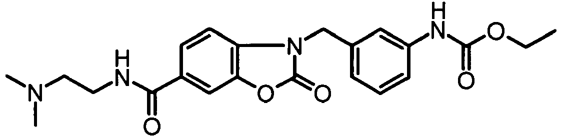 |
| "A30" | Ethyl {3-[5-(2-dimethylaminoethylcarbamoyl)-2-oxobenzoxa-zol-3-ylmethyl]phenyl}carbamate |
| "A31" | Ethyl {3-[5-(3-dimethylaminopropylcarbamoyl)-2-oxobenz-oxazol-3-ylmethyl]phenyl}carbamate |
| "A32" | Ethyl (3-{1-[5-(4-dimethylaminobutylcarbamoyl)-2-oxobenz-oxazol-3-yl]ethyl}phenyl)carbamate |
| | 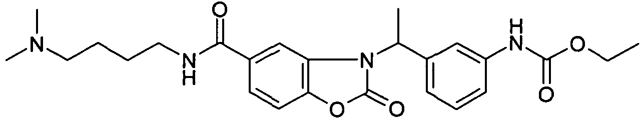 |
| "A33" | Ethyl (3-{1-[5-(2-dimethylaminoethylcarbamoyl)-2-oxobenz-oxazol-3-yl]ethyl}phenyl)carbamate |
| "A34" | N-(4-Dimethylaminobutyl)-2-oxo-3-(2-oxo-2,3-dihydro-1 H-benzimidazol-5-ylmethyl)-2,3-dihydrobenzoxazole-5-carboxamide |
| | 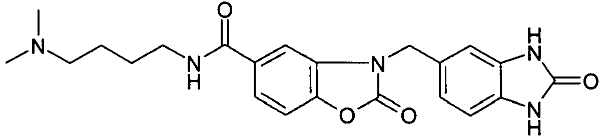 |
| "A35" | N-(2-Dimethylaminoethyl)-2-oxo-3-(2-oxo-2,3-dihydro-1H-benzimidazol-5-ylmethyl)-2,3-dihydrobenzoxazole-5-carboxamide |
| "A36" | Ethyl (3-{1-[5-(3-dimethylaminopropylcarbamoyl)-2-oxo-benzoxazol-3-yl]ethyl}phenyl)carbamate |
| "A37" | Methyl 3-(3-ethoxycarbonylaminobenzyl)-2-oxo-2,3-dihydro-benzoxazole-6-carboxylate |
| | 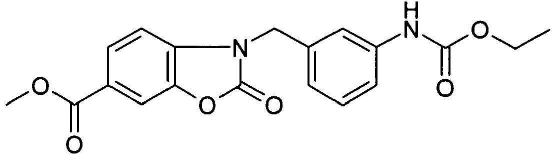 |
| "A38" | Ethyl {3-[6-(3-methylaminopropylcarbamoyl)-2-oxobenzoxa-zol-3-ylmethyl]phenyl}carbamate |
| "A39" | Ethyl {3-[6-(3-dimethylaminopropylcarbamoyl)-2-oxobenz-oxazol-3-ylmethyl]phenyl}carbamate |
| "A40" | Ethyl (3-{6-[3-(tert-butoxycarbonylmethylamino)propylcarbamoyl]-2-oxobenzoxazol-3-ylmethyl}phenyl)carbamate |
| | 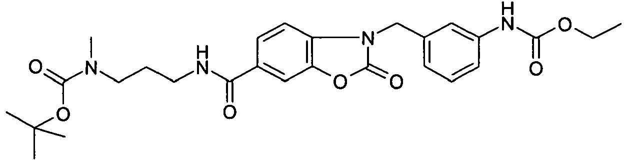 |
| "A41" | 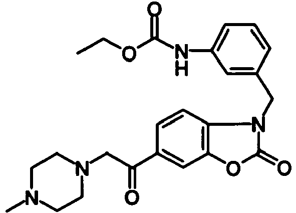 |
| "A42" | 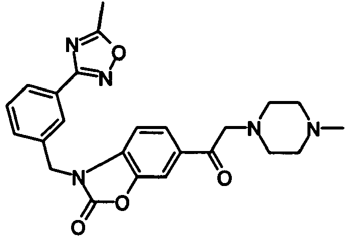 |
| "A43" | 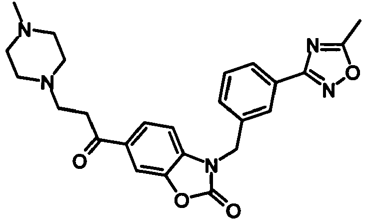 |
| "A43a" | 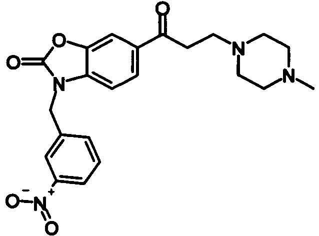 |
| "A44" | 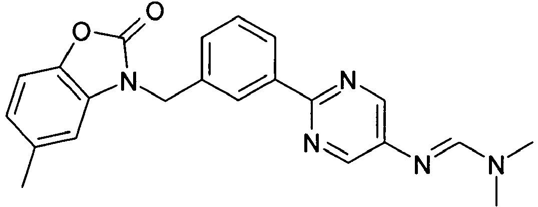 |
| "A45" | 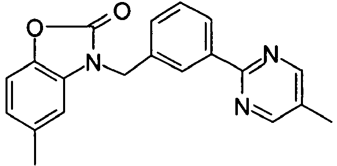 |
| "A46" | 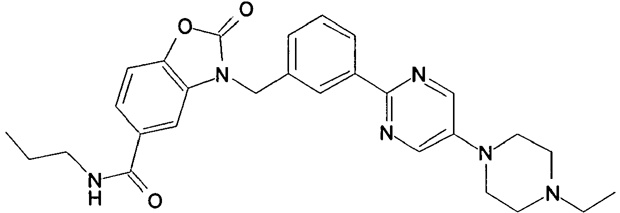 |
| "A47" | 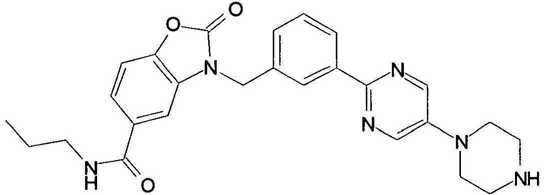 |
| "A48" | 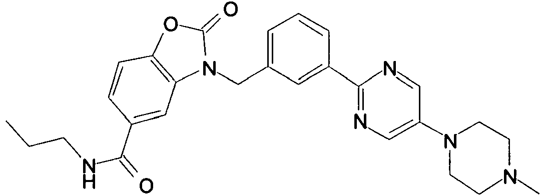 |
| "A49" | N-Propyl-3-{3-[5-(3-dimethylaminopropoxy)pyrimidin-2-yl]-benzyl}-2-oxo-2,3-dihydrobenzoxazole-5-carboxamide |
| "A50" | 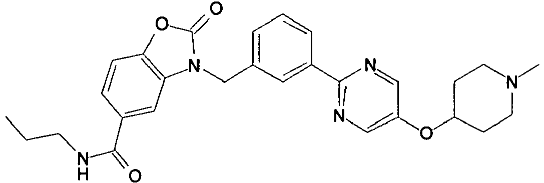 |
| "A51" | 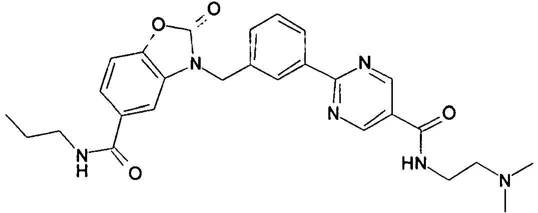 |
| "A52" | 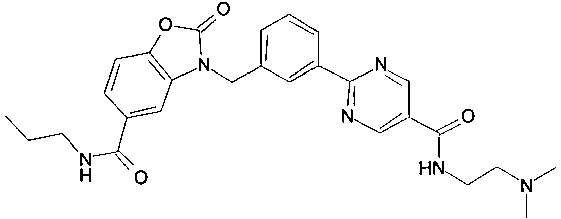 |
| "A53" | 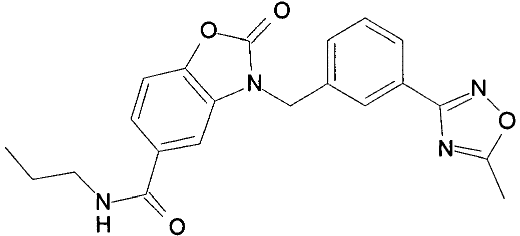 |
| "A54" | N-Propyl-3-{3-[6-(3-dimethylaminopropoxy)pyridazin-3-yl]-benzyl}-2-oxo-2,3-dihydrobenzoxazole-5-carboxamide |
| "A55" | 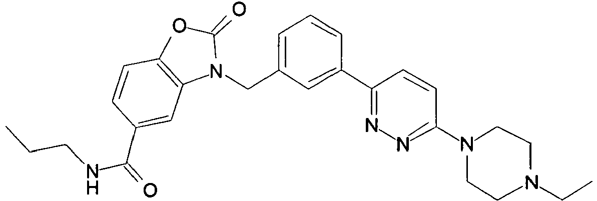 |
| "A56" | 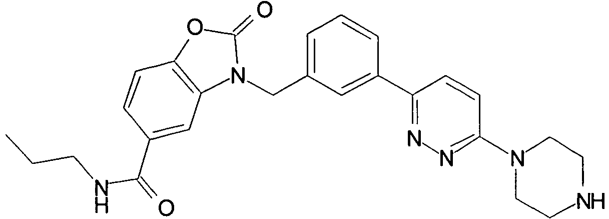 |
| "A57" | 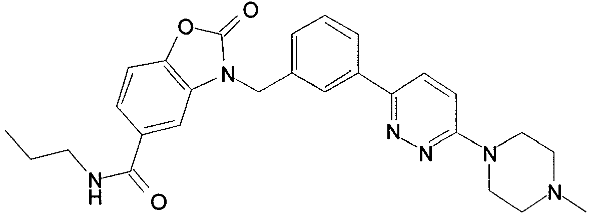 |
| "A58" | 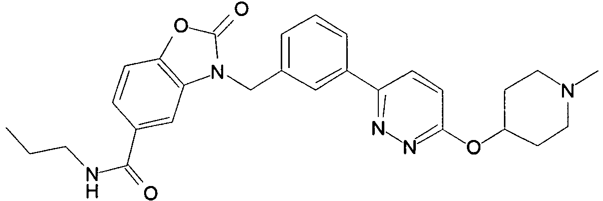 |
| "A59" | 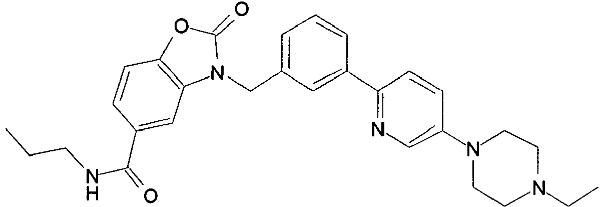 |
| "A60" | 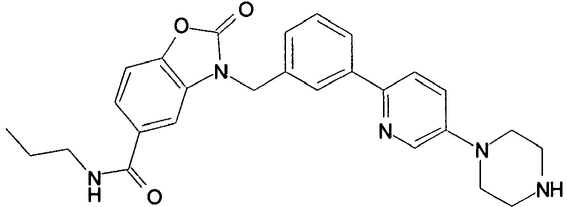 |
| "A61" | 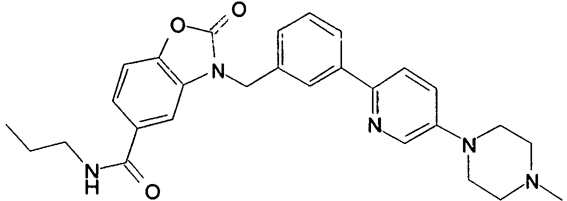 |
| "A62" | 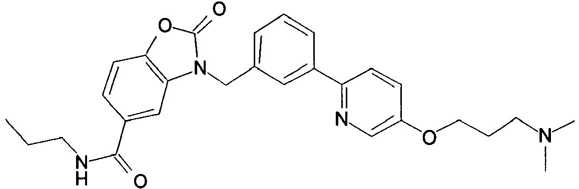 |
| "A63" | 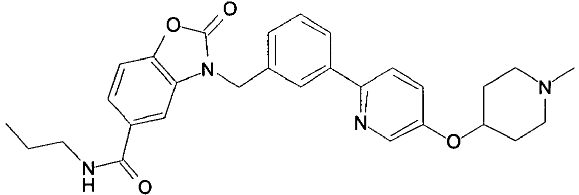 |
| "A64" | Methyl 3-{3-[5-(3-dimethylaminopropoxy)pyrimidin-2-yl]-benzyl}-2-oxo-2,3-dihydrobenzoxazole-5-carboxylate |
| "A65" | 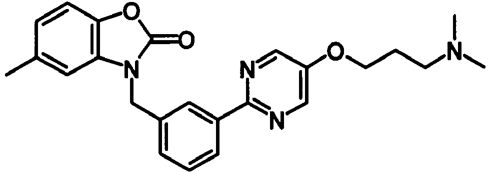 |
| "A66" | 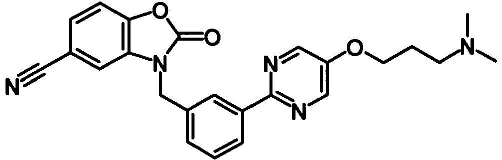 |
| "A67" | 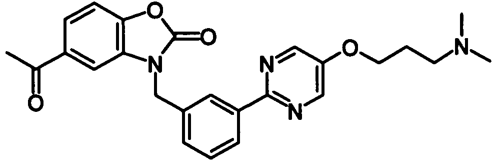 |
| "A68" | 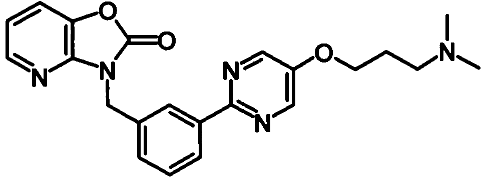 |
| "A69" | 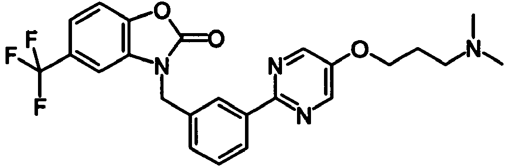 |
| "A70" | 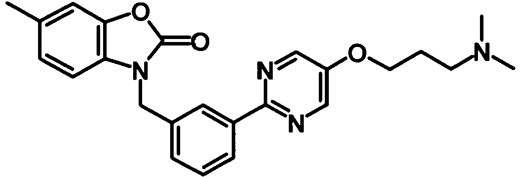 |
| "A71" | 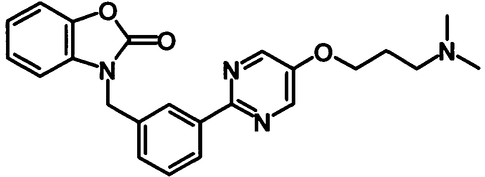 |
| "A72" | 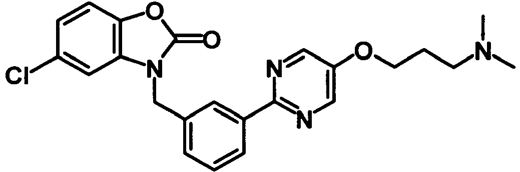 |
| "A73" | 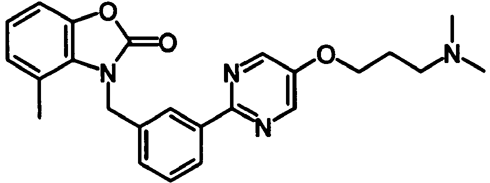 |
| "A74" | 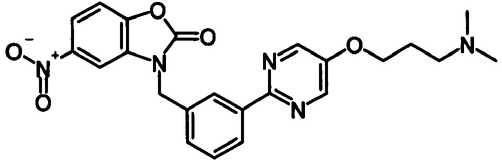 |
| "A75" | 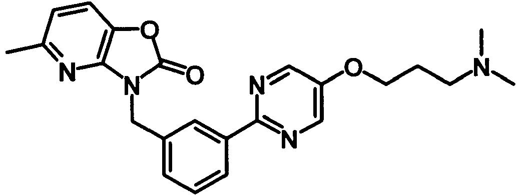 |
| "A76" | 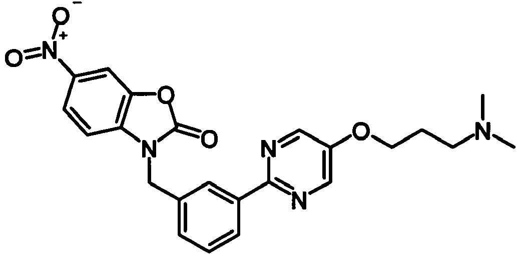 |
| "A77" | 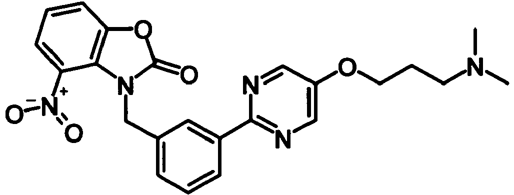 |
| "A78" | 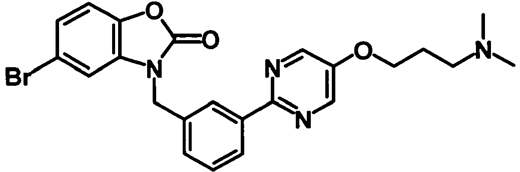 |
| "A79" | 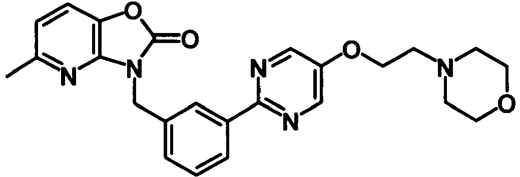 |
| "A80" | 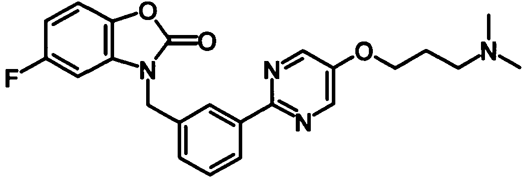 |
| "A81" | 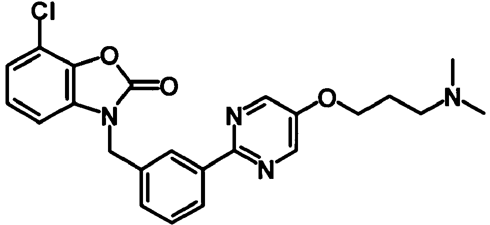 |
| "A82" | 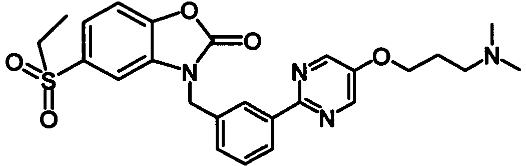 |
| "A83" | 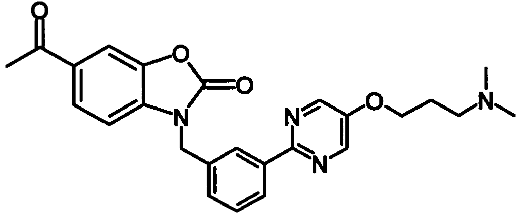 |
| "A84" | 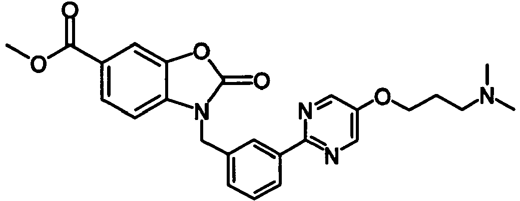 |
| "A85" | 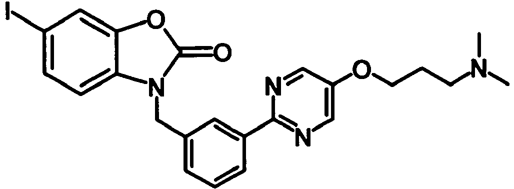 |
| "A86" | 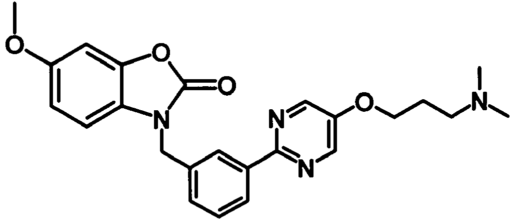 |
| "A87" | 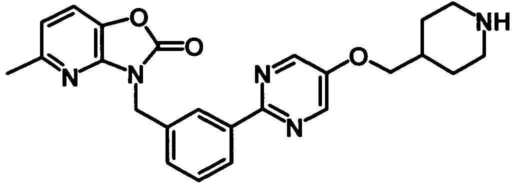 |
| "A88" | 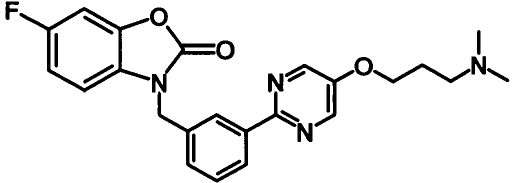 |
| "A89" | 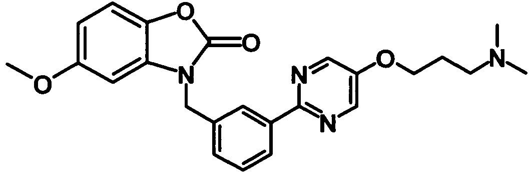 |
| "A90" | 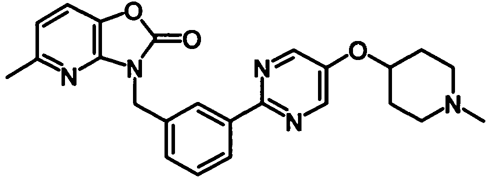 |
| "A91" | 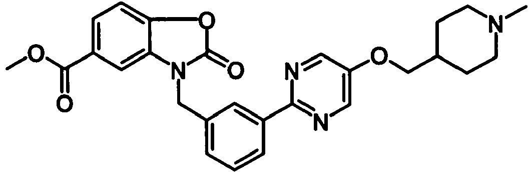 |
| "A92" | 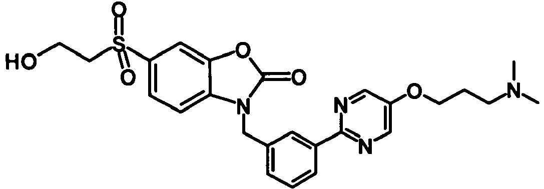 |
| "A93" | 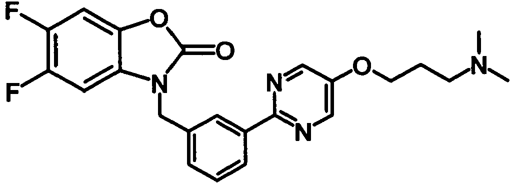 |
| "A94" | 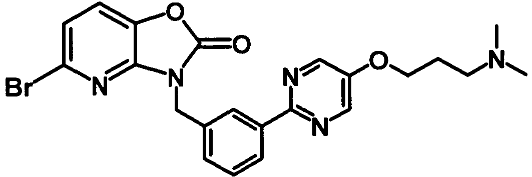 |
| "A95" | 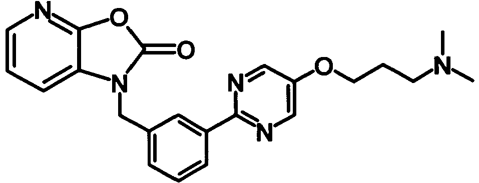 |
| "A96" | 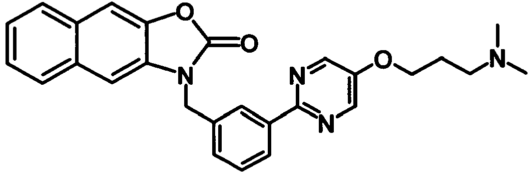 |
| "A97" | 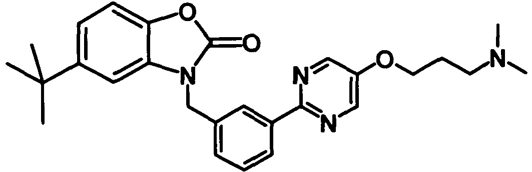 |
| "A98" | 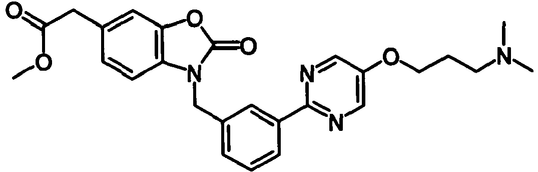 |
| "A99" | 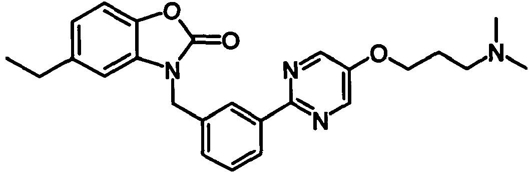 |
| "A100" | 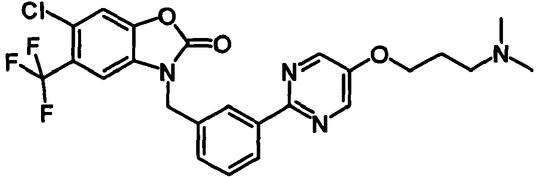 |
| "A101" | 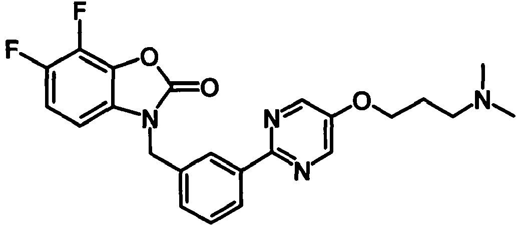 |
| "A102" | 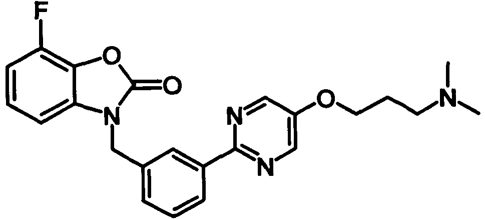 |
| "A103" | 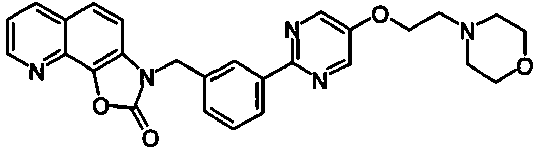 |
| "A104" | 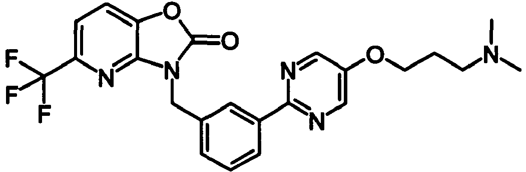 |
| "A105" | 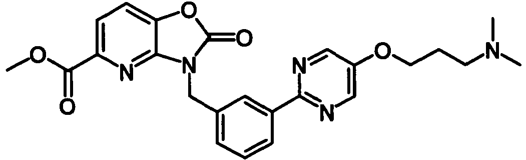 |
| "A106" | 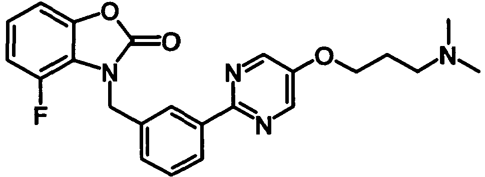 |
| "A107" | 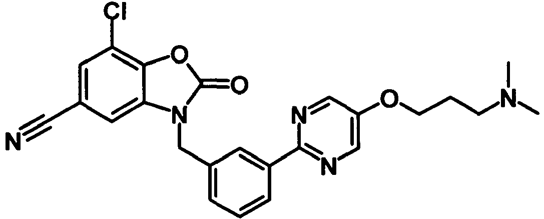 |
| "A108" | 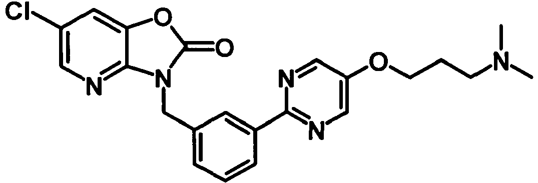 |
| "A109" | 3-{3-[5-(2-Dimethylaminoethoxy)pyrimidin-2-yl]benzyl}-5-methyl-3H-oxazolo[4,5-b]pyridin-2-one |
| "A110" | 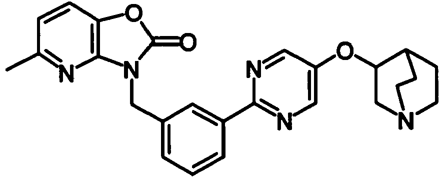 |
| "A111" | 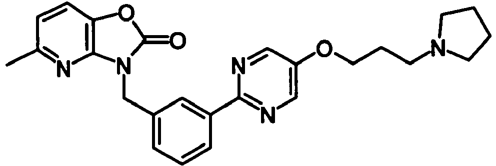 |
| "A112" | 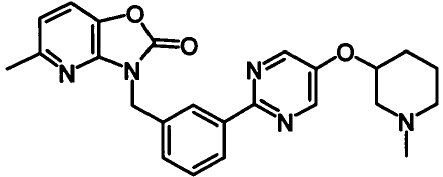 |
| "A113" | 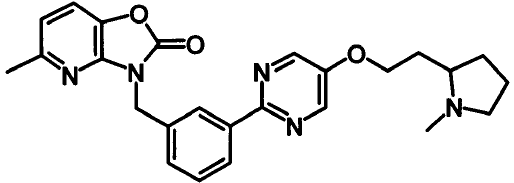 |
| "A114" | 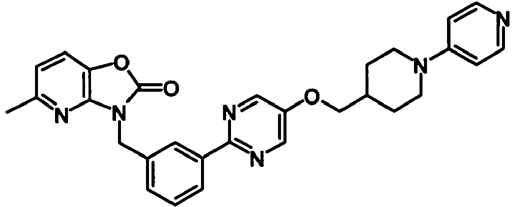 |
| "A115" | 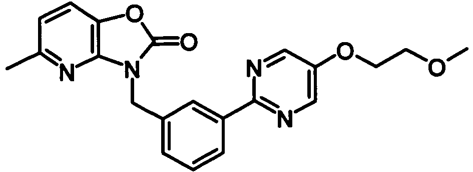 |
| "A116" | 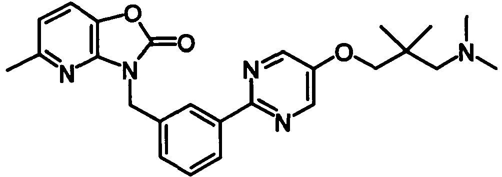 |
| "A117" | 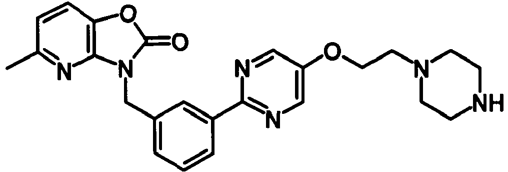 |
| "A118" | 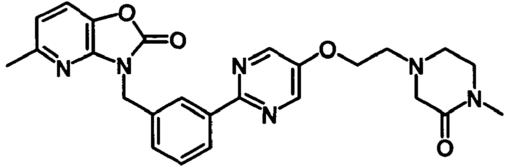 |
| "A119" | 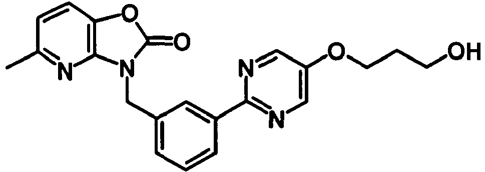 |
| "A120" | 3-{3-[5-(2,3-Dihydroxypropoxy)pyrimidin-2-yl]benzyl}-5-methyl-3H-oxazolo[4,5-b]pyridin-2-one |
| "A121" | 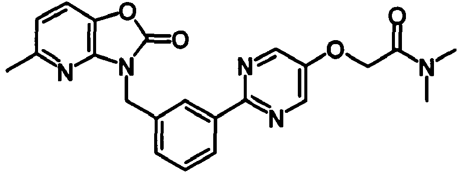 |
| "A122" | 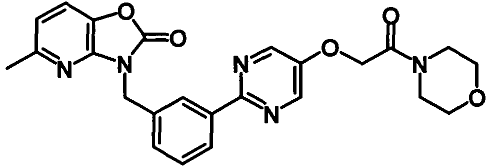 |
| "A123" | 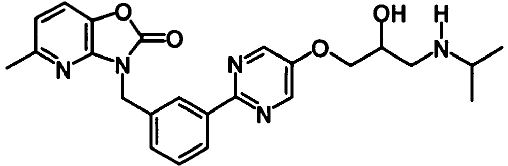 |
| "A124" | 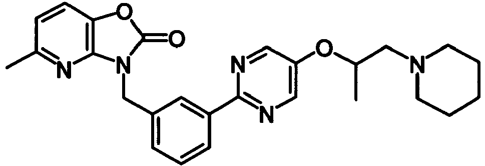 |
| "A125" | 5-Methyl-3-{3-[5-(4-methylpiperazin-1-yl)pyrimidin-2-yl]-benzyl}-3H-oxazolo[4,5-b]pyridin-2-one |
| "A126" | 5-Methyl-3-{3-[5-(4-methylpiperazin-1-yl)pyrimidin-2-yl]-benzyl}-3H-oxazolo[4,5-b]pyridin-2-one |
| "A127" | 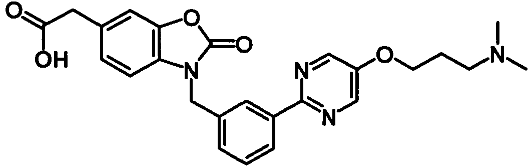 |
| "A128" | 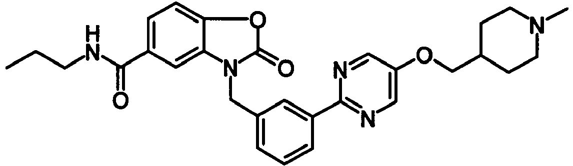 |
| "A129" | 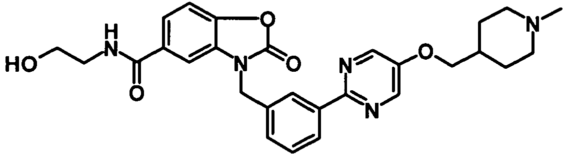 |
| "A130" | 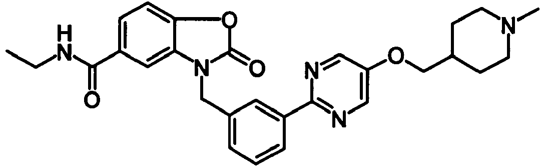 |
| "A131" | 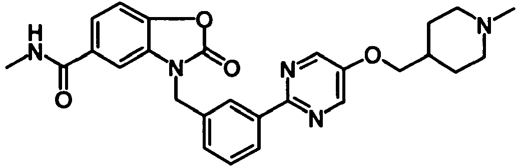 |
| "A132" | 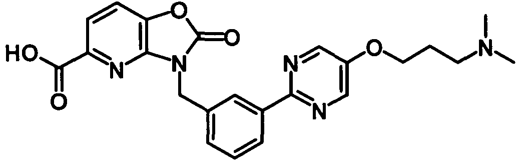 |
| "A133" | 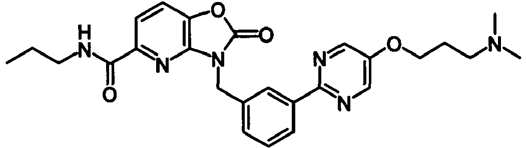 |
| "A134" | 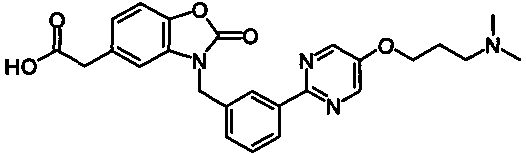 |
| "A135" | 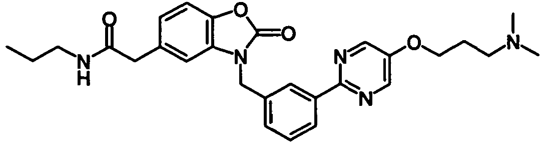 |
| "A136" | 3-{3-[5-(3-Dimethylaminopropoxy)pyrimidin-2-yl]benzyl}-6-(1-hydroxyethyl)-3H-benzoxazol-2-one |
| "A137" | 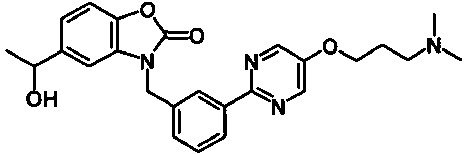 |
| "A138" | N-[3-(6-Chloro-2-oxobenzoxazol-3-ylmethyl)phenyl]-acetamide |
| "A139" | 1-[3-(6-Chloro-2-oxobenzoxazol-3-ylmethyl)phenyl]-3-[3-(4-methylpiperazin-1-yl)propyl]urea |
| "A140" | 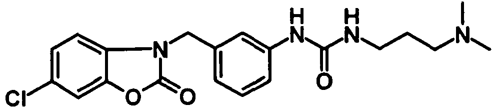 |
| "A141" | N-[3-(6-Chloro-2-oxobenzoxazol-3-ylmethyl)phenyl]-N'-(2-dimethylaminoethyl)oxalamide |
| "A142" | N-(4-Dimethylaminobutyl)-2-[3-(5-methyl-2-oxooxazolo[4,5-b]pyridin-3-ylmethyl)phenyl]pyrimidine-5-carboxamide |
| "A143" | 5-Methyl-3-[3-(3-methyl-6-oxo-6H-pyridazin-1-yl)benzyl]-3H-oxazolo[4,5-b]pyridin-2-one |
| "A144" | 5-Methyl-3-[3-(5-methylpyridin-2-yl)benzyl]-3H-oxazolo-[4,5-b]pyridin-2-one |
| "A145" | 5-Methyl-3-(3-pyrimidin-5-ylbenzyl)-3H-oxazolo[4,5-b]-pyridin-2-one |
| "A146" | 5-Methyl-3-[3-(4-piperazin-1-ylpyrimidin-2-yl)benzyl]-3H-oxazolo[4,5-b]pyridin-2-one |
| "A147" | 5-Methyl-3-{3-[5-(1-methyl-1H-pyrazol-4-yl)pyrimidin-2-yl]-benzyl}-3H-oxazolo[4,5-b]pyridin-2-one |
| "A148" | 3-{3-[5-(3-Dimethylaminopropoxy)pyrimidin-2-yl]benzyl}-5-(1-methyl-1 H-pyrazol-4-yl)-3H-oxazolo[4,5-b]pyridin-2-one |
and pharmaceutically usable solvates, salts, tautomers and stereoisomers thereof, including mixtures thereof in all ratios.

2. Medicaments comprising at least one compound according to Claim 1 and/or pharmaceutically usable salts, solvates, tautomers and stereoisomers thereof, including mixtures thereof in all ratios, and optionally excipients and/or adjuvants.

3. Use of compounds according to Claim 1 and pharmaceutically usable salts, solvates, tautomers and stereoisomers thereof, including mixtures thereof in all ratios,
for the preparation of a medicament for the treatment of diseases in which the inhibition, regulation and/or modulation of kinase signal transduction plays a role.

4. Use according to Claim 3 of compounds according to Claim 1, and pharmaceutically usable salts, solvates and stereoisomers thereof, including mixtures thereof in all ratios,
for the preparation of a medicament for the treatment of diseases which are influenced by inhibition of tyrosine kinases by the compounds according to Claim 1.

5. Use according to Claim 4 for the preparation of a medicament for the treatment of diseases which are influenced by inhibition of Met kinase by the compounds according to Claim 1.

6. Use according to Claim 4 or 5, where the disease to be treated is a solid tumour.

7. Use according to Claim 6, where the solid tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

8. Use according to Claim 6, where the solid tumour originates from the group monocytic leukaemia, lung adenocarcinoma, small-cell lung carcinomas, pancreatic cancer, glioblastomas and breast carcinoma.

9. Use according to Claim 6, where the solid tumour originates from the group of lung adenocarcinoma, small-cell lung carcinomas, pancreatic cancer, glioblastomas, colon carcinoma and breast carcinoma.

10. Use according to Claim 4 or 5, where the disease to be treated is a tumour of the blood and immune system.

11. Use according to Claim 10, where the tumour originates from the group of acute myeloid leukaemia, chronic myeloid leukaemia, acute lymphatic leukaemia and/or chronic lymphatic leukaemia.

12. Medicaments comprising at least one compound according to Claim 1, and/or pharmaceutically usable salts, solvates and stereoisomers thereof, including mixtures thereof in all ratios, and at least one further medicament active compound.

13. Set (kit) consisting of separate packs of
(a) an effective amount of a compound according to Claim 1, and/or pharmaceutically usable salts, solvates and stereoisomers thereof, including mixtures thereof in all ratios, and
(b) an effective amount of a further medicament active compound.

## Revendications

1. Composés choisis dans le groupe constitué par
| n° | Structure et/ou nom |
|---|---|
| "A1" | [3-(5-méthoxy-2-oxobenzoxazol-3-ylméthyl)phényl]-carbamate de 3-(4-méthylpipérazin-1-yl)propyle |
| | 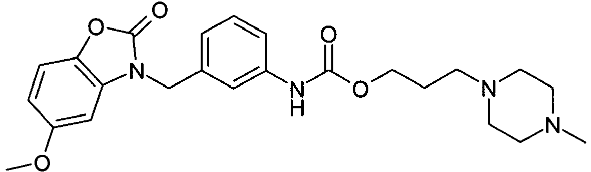 |
| "B1" | [3-(5-méthyl-2-oxooxazolo[4,5-b]pyridin-3-ylméthyl)phényl]-carbamate de 3-(4-méthylpipérazin-1-yl)propyle |
| "A2" | [3-(5-chloro-2-oxobenzoxazol-3-ylméthyl)phényl]carbamate de 3-(4-méthylpipérazin-1-yl)propyle |
| | 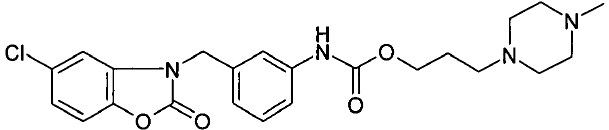 |
| | |
| "A3" | [3-(5-diméthylcarbamoyl-2-oxobenzoxazol-3-ylméthyl)-phényl]carbamate de 3-(4-méthylpipérazin-1-yl)propyle |
| | 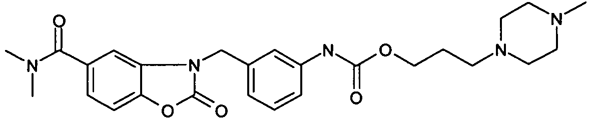 |
| "A4" | [3-(5-d iméthylcarbamoyl-2-oxobenzoxazol-3-ylméthyl)-phényl]carbamate de 3-pipérazin-1-ylpropyle |
| "A5" | [3-(5-propylcarbamoyl-2-oxobenzoxazol-3-ylméthyl)phényl]-carbamate de 3-(4-méthylpipérazin-1-yl)propyle |
| "A6" | [3-(6-chloro-2-oxobenzoxazol-3-ylméthyl)phényl]carbamate de 3-(4-méthylpipérazin-1-yl)propyle |
| "A7" | [3-(5-méthyl-2-oxobenzoxazol-3-ylméthyl)phényl]carbamate de 3-(4-méthylpipérazin-1-yl)propyle |
| "A8" | [3-(5-acétyl-2-oxobenzoxazol-3-ylméthyl)phényl]carbamate de 3-(4-méthylpipérazin-1-yl)propyle |
| "A9" | {3-[6-(1-hydroxyéthyl)-2-oxobenzoxazol-3-ylméthyl]-phényl}carbamate de 3-(4-méthylpipérazin-1-yl)propyle |
| | 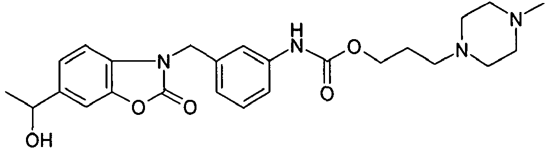 |
| "A10" | [3-(5-propylcarbamoyl-2-oxobenzoxazol-3-ylméthyl)phényl]carbamate de 3-pipérazin-1-ylpropyle |
| "A11" | [3-(2-oxooxazolo[4,5-b]pyridin-3-ylméthyl)phényl]carbamate de 3-diméthylaminopropyle |
| | 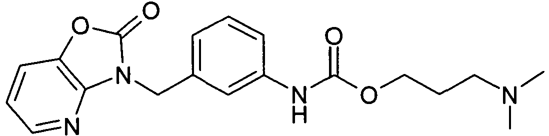 |
| "A12" | [3-(5-diméthylcarbamoyl-2-oxobenzoxazol-3-ylméthyl)-phényl]carbamate de 3-diméthylaminopropyle |
| "A 13" | [3-(5-propylcarbamoyl-2-oxobenzoxazol-3-ylméthyl)-phényl]carbamate de 3-diméthylaminopropyle |
| "A14" | [3-(6-méthoxycarbonyl-2-oxobenzoxazol-3-ylméthyl)-phényl]carbamate de 3-diméthylaminopropyle |
| "A15" | [3-(5-méthoxycarbonyl-2-oxobenzoxazol-3-ylméthyl)-phényl]carbamate de 3-diméthylaminopropyle |
| "A16" | [3-(2-oxobenzoxazol-3-ylméthyl)phényl]carbamate de 3-diméthylaminopropyle |
| "A17" | [3-(2-oxobenzoxazol-3-ylméthyl)phényl]carbamate de 2-(4-méthylpipérazin-1-yl)éthyle |
| | 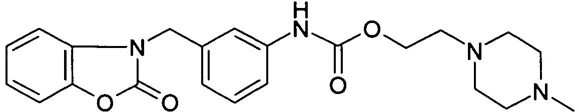 |
| "A18" | [3-(2-oxobenzoxazol-3-ylméthyl)phényl]carbamate de 3-(4-méthylpipérazin-1-yl)propyle |
| "A19" | [3-(5,6-difluoro-2-oxobenzoxazol-3-ylméthyl)phényl]-carbamate de 3-(4-méthylpipérazin-1-yl)propyle |
| "A20" | [3-(6-méthoxy-2-oxobenzoxazol-3-ylméthyl)phényl]-carbamate de 3-(4-méthylpipérazin-1-yl)propyle |
| "A21" | [3-(6-méthyl-2-oxobenzoxazol-3-ylméthyl)phényl]carbamate de 3-(4-méthylpipérazin-1-yl)propyle |
| "A22" | [3-(6-acétyl-2-oxobenzoxazol-3-ylméthyl)phényl]carbamate de 3-(4-méthylpipérazin-1-yl)propyle |
| "A23" | {3-[5-(4-méthylpipérazin-1-yl)-2-oxobenzoxazol-3-ylméthyl]phényl}carbamate d'éthyle |
| | 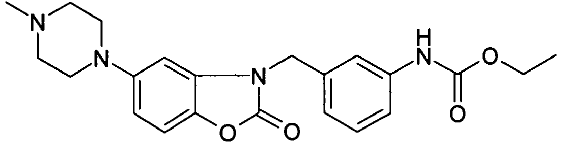 |
| "A24" | [3-(5-cyano-2-oxobenzoxazol-3-ylméthyl)phényl]carbamate de 3-(4-méthylpipérazin-1-yl)propyle |
| "A25" | [3-(5-éthylsulfonyl-2-oxobenzoxazol-3-ylméthyl)phényl]-carbamate de 3-(4-méthylpipérazin-1-yl)propyle |
| "A26" | {3-[1-(5,6-difluoro-2-oxobenzoxazol-3-yl)éthyl]phényl}-carbamate de 3-(4-méthylpipérazin-1-yl)propyle |
| | 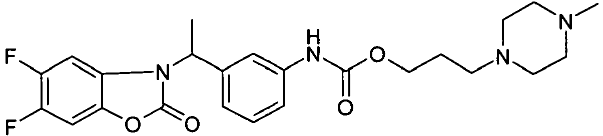 |
| "A27" | {3-[1-(5,6-difluoro-2-oxobenzoxazol-3-yl)éthyl]phényl}-carbamate de 2-(4-méthylpipérazin-1-yl)éthyle |
| "A28" | {3-[5-(1-hydroxyéthyl)-2-oxobenzoxazol-3-ylméthyl]phényl}-carbamate de 3-(4-méthylpipérazin-1-yl)propyle |
| | 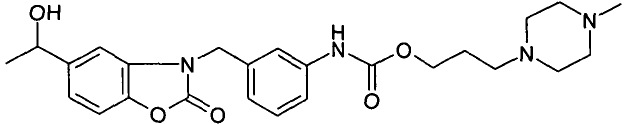 |
| "A28a" | 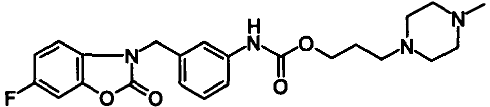 |
| "A28b" | 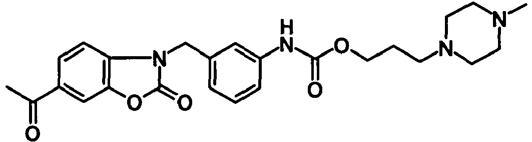 |
| "A29" | {3-[6-(2-diméthylaminoéthylcarbamoyl)-2-oxobenzoxazol-3-ylméthyl]phényl}carbamate d'éthyle |
| | 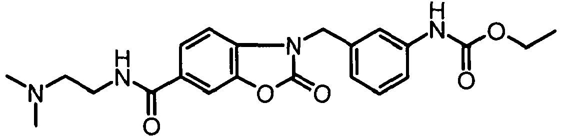 |
| "A30" | {3-[5-(2-diméthylaminoéthylcarbamoyl)-2-oxobenzoxazol-3-ylméthyl]phényl}carbamate d'éthyle |
| "A31" | {3-[5-(3-diméthylaminopropylcarbamoyl)-2-oxobenzoxazol-3-ylméthyl]phényl}carbamate d'éthyle |
| "A32" | (3-{1-[5-(4-diméthylaminobutylcarbamoyl)-2-oxobenzoxazol-3-yl]éthyl}phényl)carbamate d'éthyle |
| | 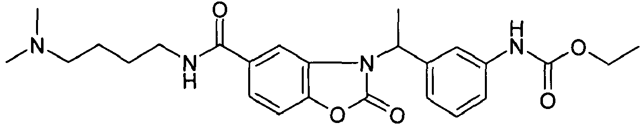 |
| "A33" | (3-{1-[5-(2-diméthylaminoéthylcarbamoyl)-2-oxobenzoxazol-3-yl]éthyl}phényl)carbamate d'éthyle |
| "A34" | N-(4-diméthylaminobutyl)-2-oxo-3-(2-oxo-2,3-dihydro-1 H-benzimidazol-5-ylméthyl)-2,3-dihydrobenzoxazole-5-carboxamide |
| | 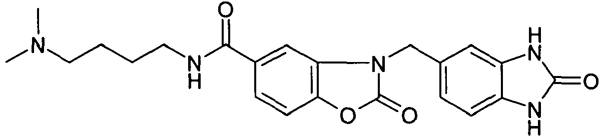 |
| "A35" | N-(2-diméthylaminoéthyl)-2-oxo-3-(2-oxo-2,3-dihydro-1 H-benzimidazol-5-ylméthyl)-2,3-dihydrobenzoxazole-5-carboxamide |
| "A36" | (3-{1-[5-(3-diméthylaminopropylcarbamoyl)-2-oxobenzoxazol-3-yl]éthyl}phényl)carbamate d'éthyle |
| "A37" | 3-(3-éthoxycarbonylaminobenzyl)-2-oxo-2,3-dihydrobenzoxazole-6-carboxylate de méthyle |
| | 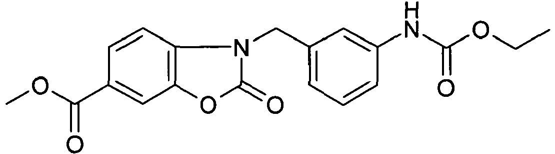 |
| "A38" | {3-[6-(3-méthylaminopropylcarbamoyl)-2-oxobenzoxazol-3-ylméthyl]phényl}carbamate d'éthyle |
| "A39" | {3-[6-(3-diméthylaminopropylcarbamoyl)-2-oxobenzoxazol-3-ylméthyl]phényl}carbamate d'éthyle |
| "A40" | (3-{6-[3-(tertio-butoxycarbonylméthylamino)propyl-carbamoyl]-2-oxobenzoxazol-3-ylméthyl}phényl)carbamate d'éthyle |
| | 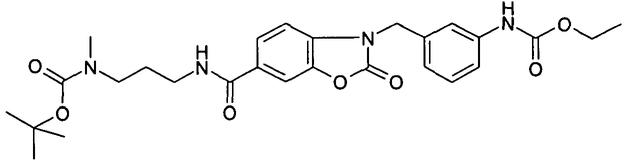 |
| "A41" | 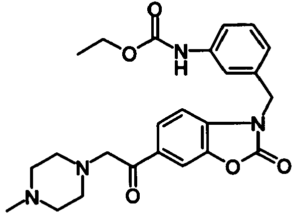 |
| "A42" | 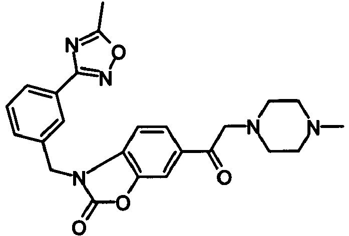 |
| "A43" | 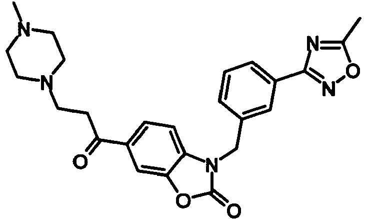 |
| "A43a" | 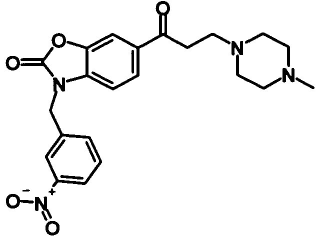 |
| "A44" | 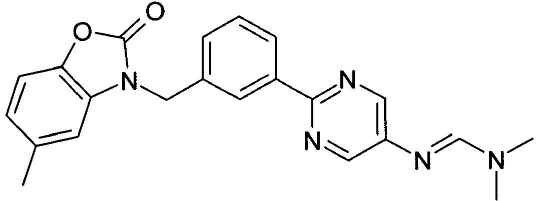 |
| "A45" | 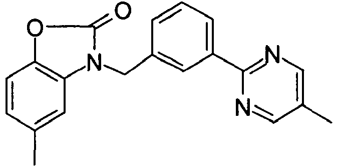 |
| "A46" | 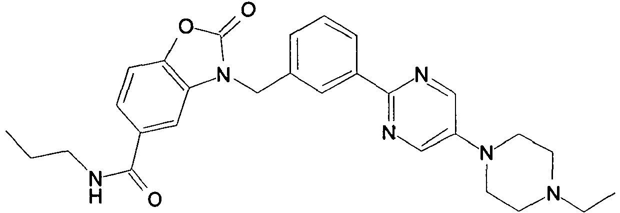 |
| "A47" | 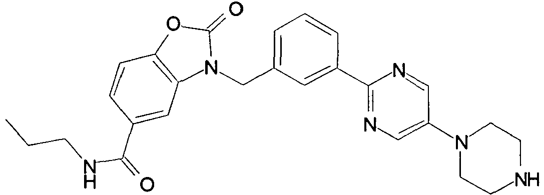 |
| "A48" | 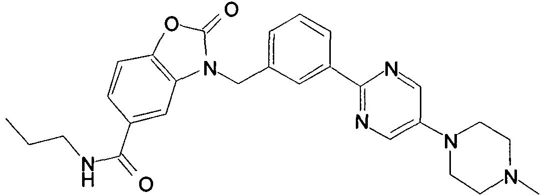 |
| "A49" | N-propyl-3-{3-[5-(3-diméthylaminopropoxy)pyrimidin-2-yl]benzyl}-2-oxo-2,3-dihydrobenzoxazole-5-carboxamide |
| "A50" | 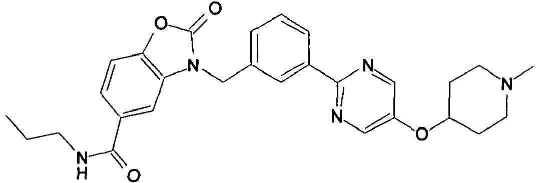 |
| "A51" | 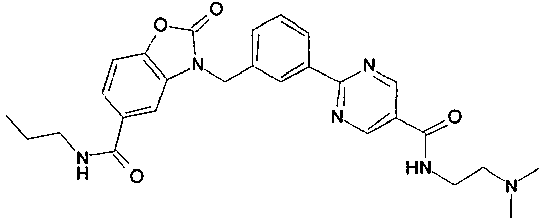 |
| "A52" | 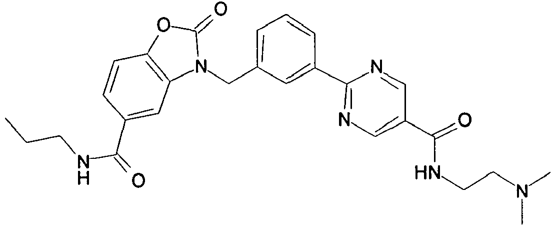 |
| "A53" | 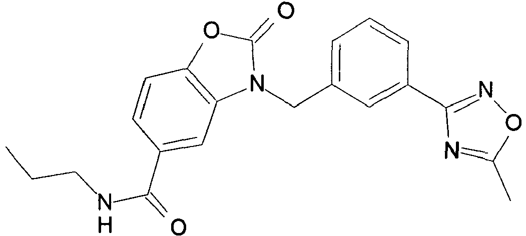 |
| "A54" | N-propyl-3-{3-[6-(3-diméthylaminopropoxy)pyridazin-3-yl]benzyl}-2-oxo-2,3-dihydrobenzoxazole-5-carboxamide |
| "A55" | 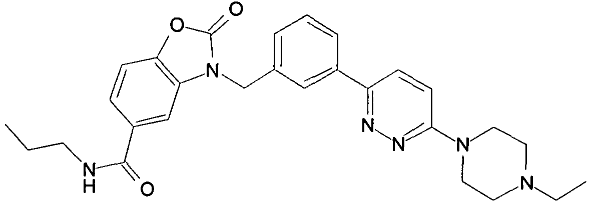 |
| "A56" | 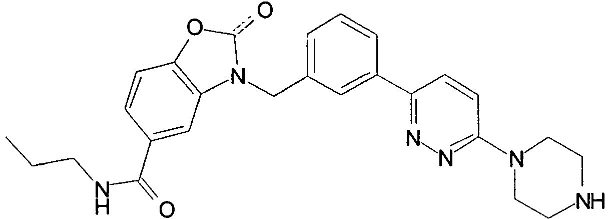 |
| "A57" | 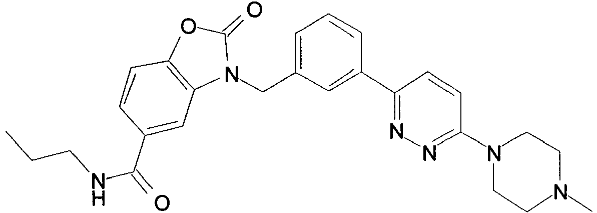 |
| "A58" | 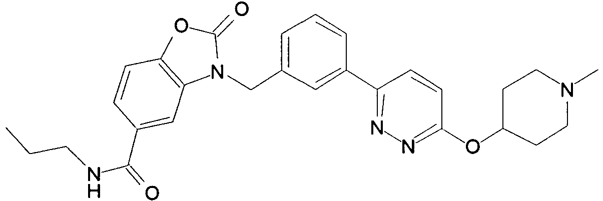 |
| "A59" | 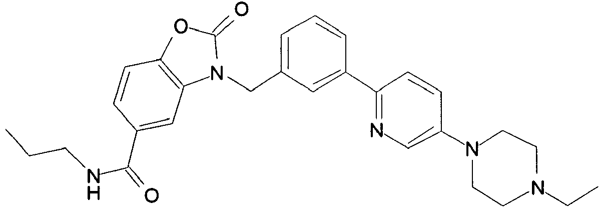 |
| "A60" | 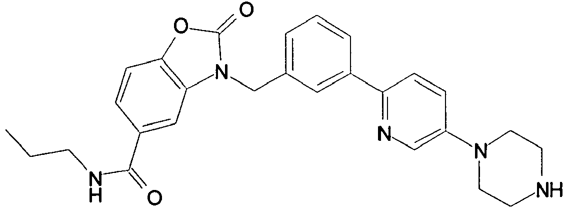 |
| "A61" | 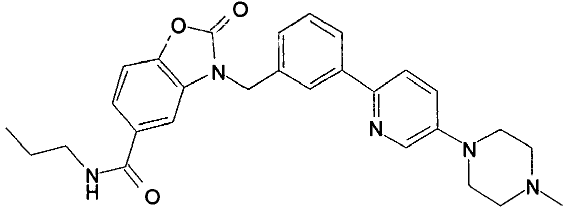 |
| "A62" | 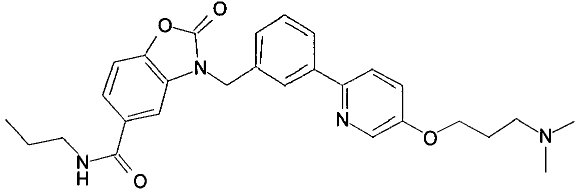 |
| "A63" | 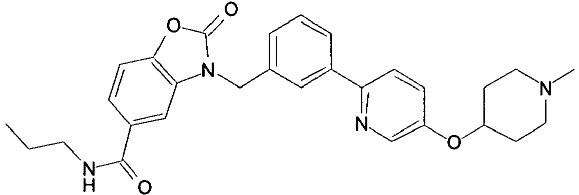 |
| "A64" | 3-{3-[5-(3-diméthylaminopropoxy)pyrimidin-2-yl]benzyl}-2-oxo-2,3-dihydrobenzoxazole-5-carboxylate de méthyle |
| "A65" | 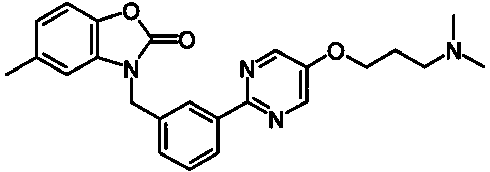 |
| "A66" | 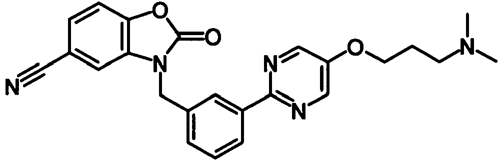 |
| "A67" | 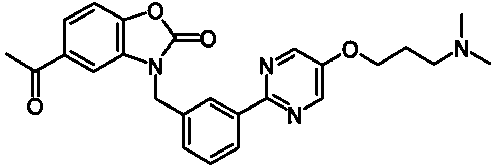 |
| "A68" | 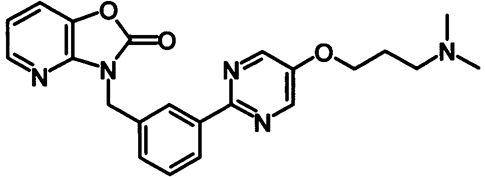 |
| "A69" | 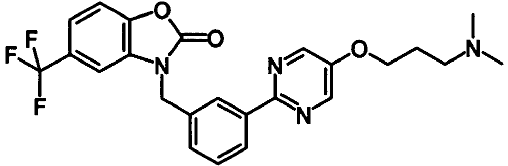 |
| "A70" | 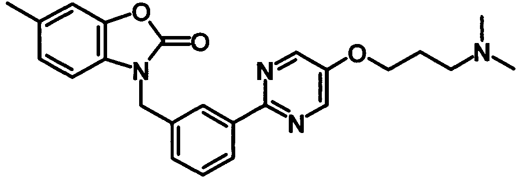 |
| "A71" | 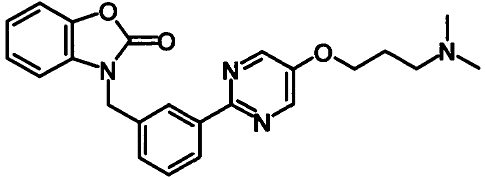 |
| "A72" | 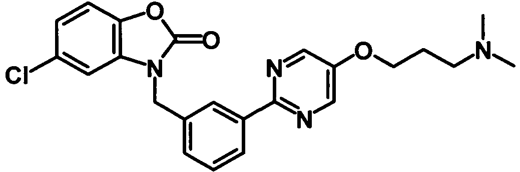 |
| "A73" | 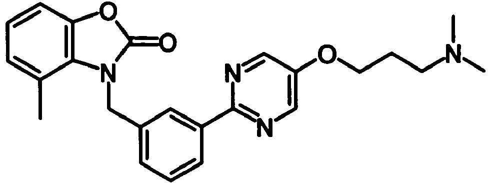 |
| "A74" | 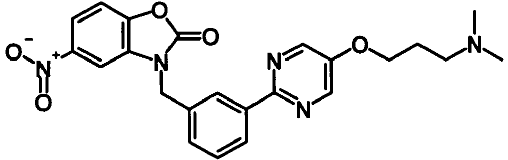 |
| "A75" | 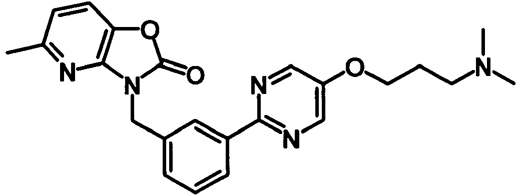 |
| "A76" | 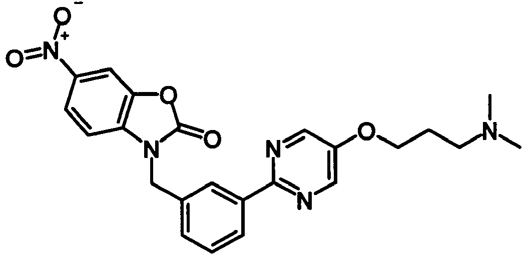 |
| "A77" | 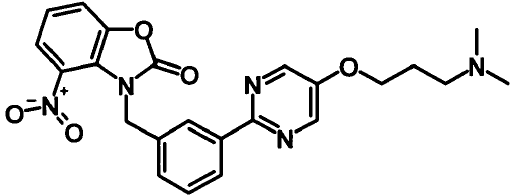 |
| "A78" | 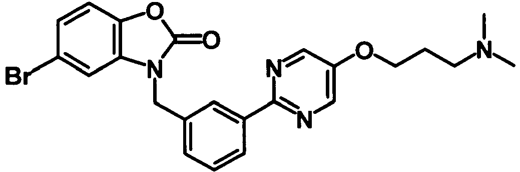 |
| "A79" | 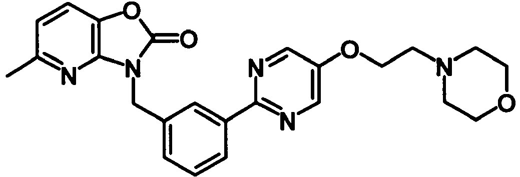 |
| "A80" | 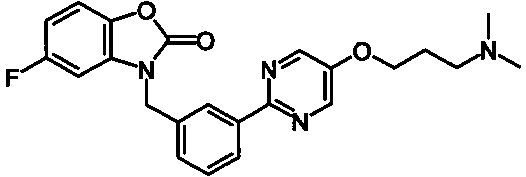 |
| "A81" | 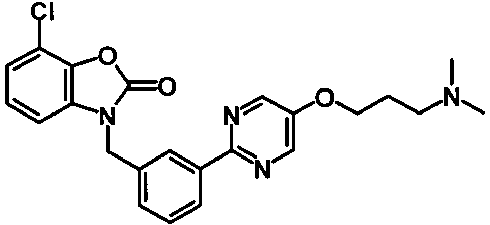 |
| "A82" | 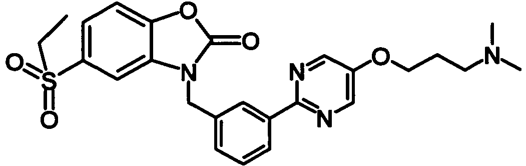 |
| "A83" | 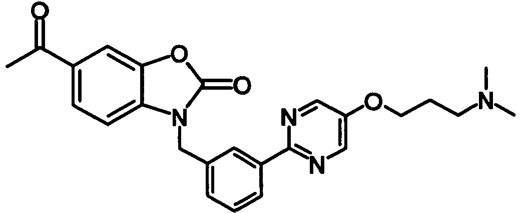 |
| "A84" | 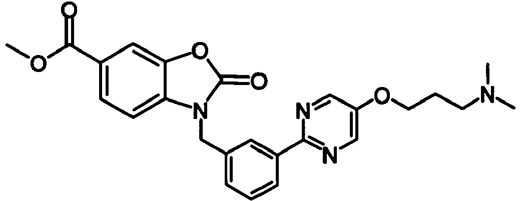 |
| "A85" | 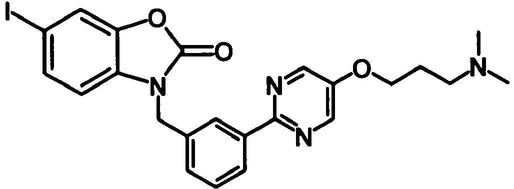 |
| "A86" | 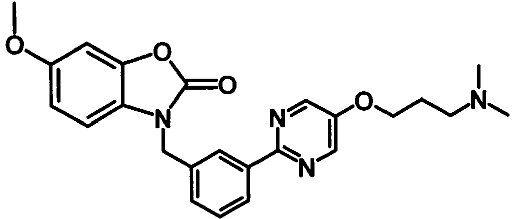 |
| "A87" | 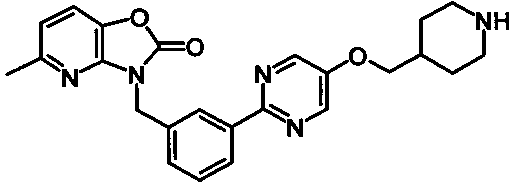 |
| "A88" | 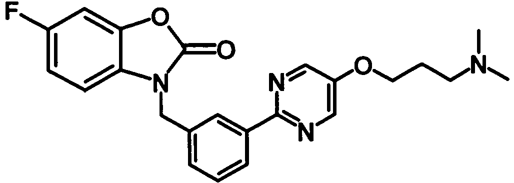 |
| "A89" | 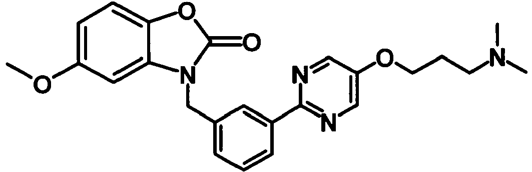 |
| "A90" | 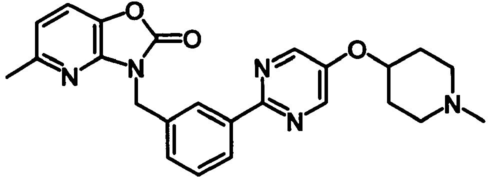 |
| "A91" | 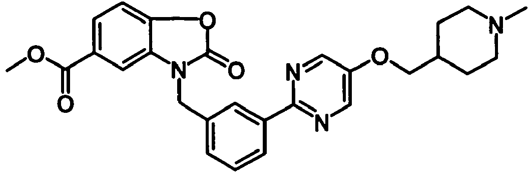 |
| "A92" | 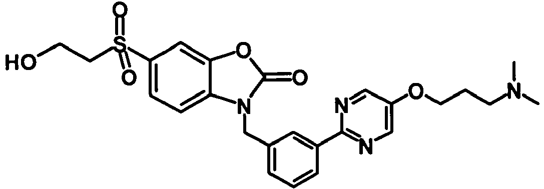 |
| "A93" | 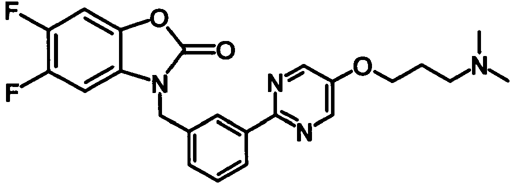 |
| "A94" | 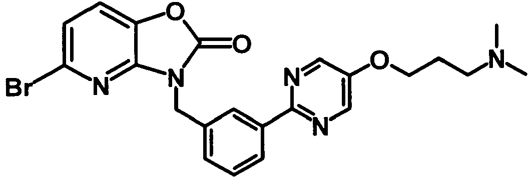 |
| "A95" | 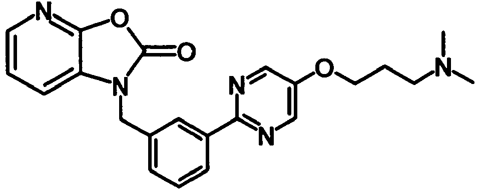 |
| "A96" | 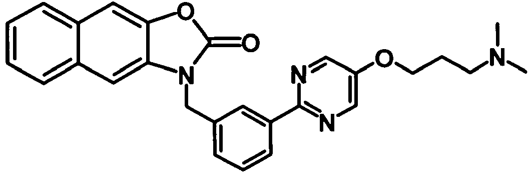 |
| "A97" | 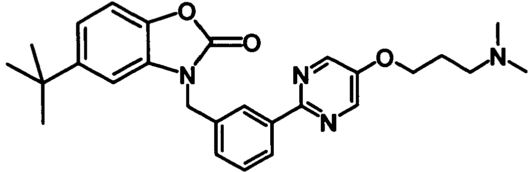 |
| "A98" | 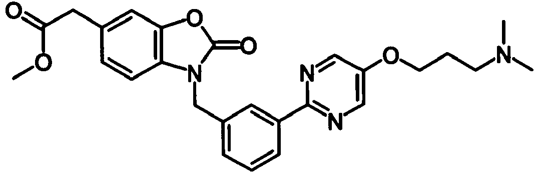 |
| "A99" | 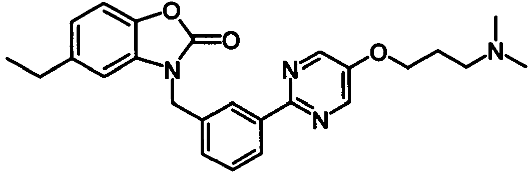 |
| "A100" | 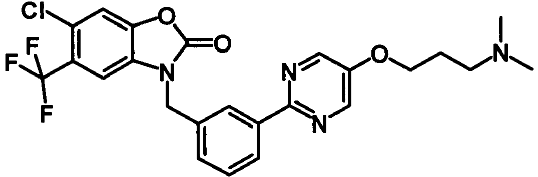 |
| "A101" | 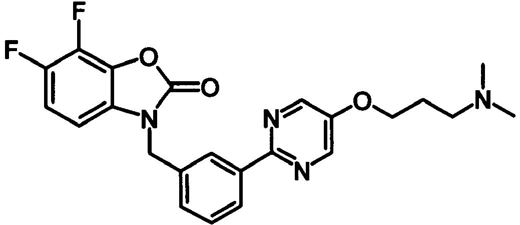 |
| "A102" | 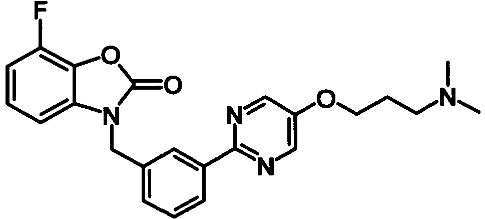 |
| "A103" | 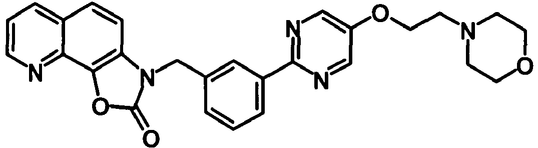 |
| "A104" | 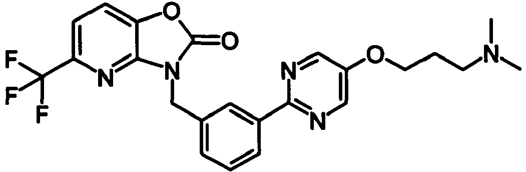 |
| "A105" | 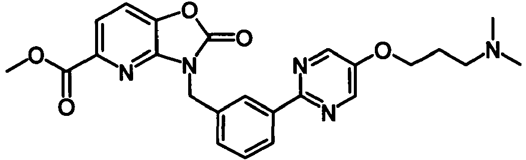 |
| "A106" | 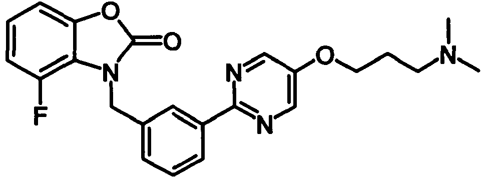 |
| "A107" | 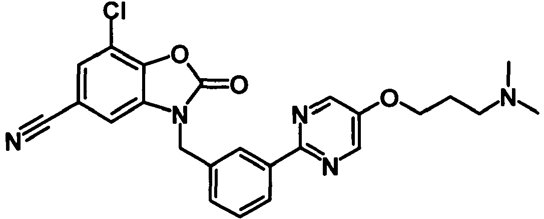 |
| "A108" | 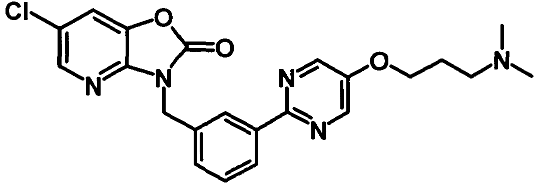 |
| "A109" | 3-{3-[5-(2-diméthylaminoéthoxy)pyrimidin-2-yl]benzyl}-5-méthyl-3H-oxazolo[4,5-b]pyridin-2-one |
| "A110" | 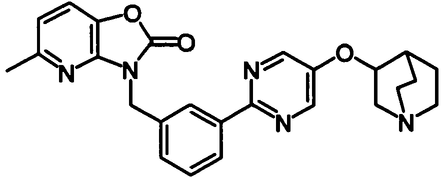 |
| "A111" | 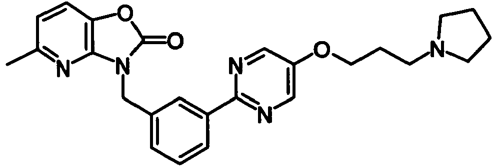 |
| "A112" | 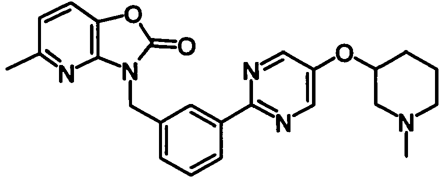 |
| "A113" | 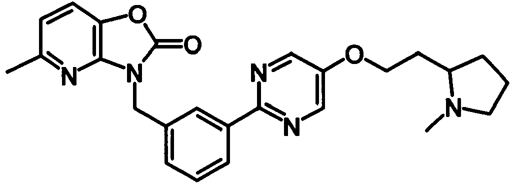 |
| "A114" | 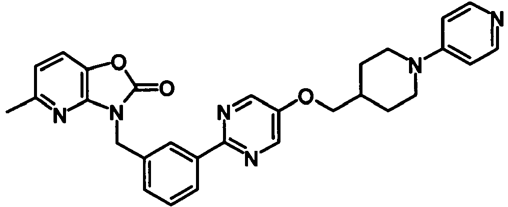 |
| "A115" | 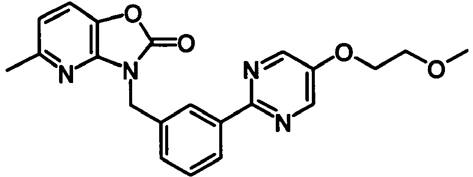 |
| "A116" | 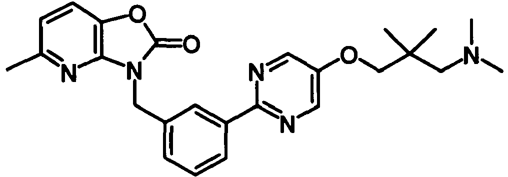 |
| "A117" | 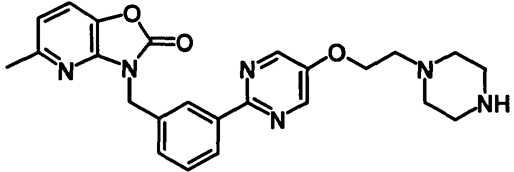 |
| "A118" | 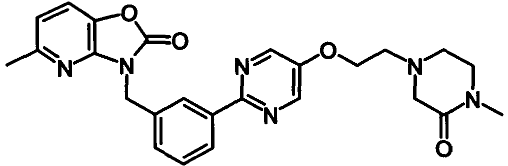 |
| "A119" | 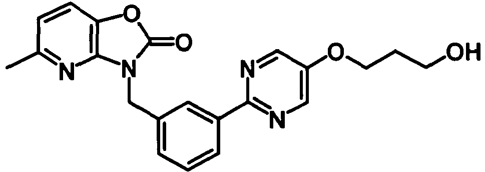 |
| "A120" | 3-{3-[5-(2,3-dihydroxypropoxy)pyrimidin-2-yl]benzyl}-5-méthyl-3H-oxazolo[4,5-b]pyridin-2-one |
| "A121" | 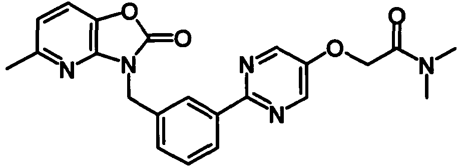 |
| "A122" | 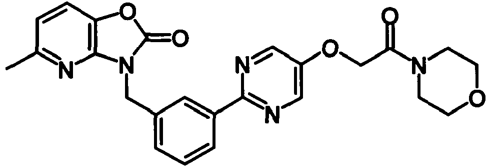 |
| "A123" | 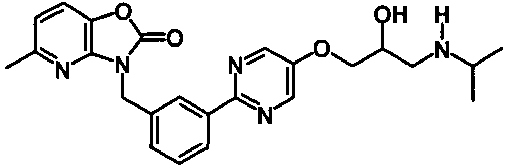 |
| "A124" | 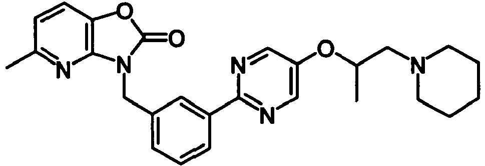 |
| "A125" | 5-méthyl-3-{3-[5-(4-méthylpipérazin-1-yl)pyrimidin-2-yl]benzyl}-3H-oxazolo[4,5-b]pyridin-2-one |
| "A126" | 5-méthyl-3-{3-[5-(4-méthylpipérazin-1-yl)pyrimidin-2-yl]benzyl}-3H-oxazolo[4,5-b]pyridin-2-one |
| "A127" | 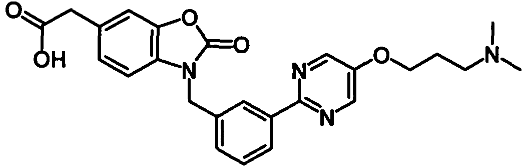 |
| "A128" | 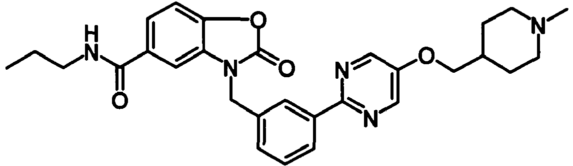 |
| "A129" | 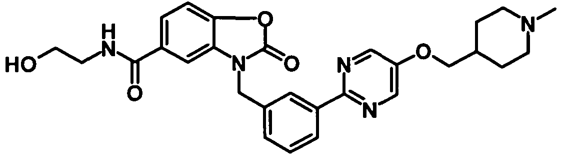 |
| "A130" | 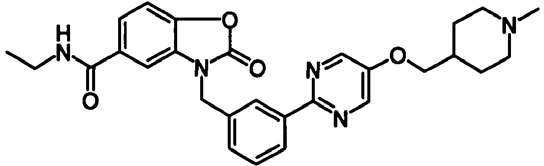 |
| "A131" | 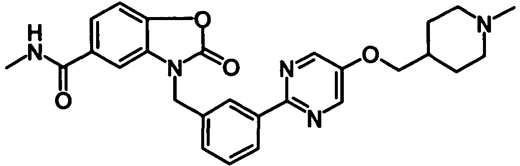 |
| "A132" | 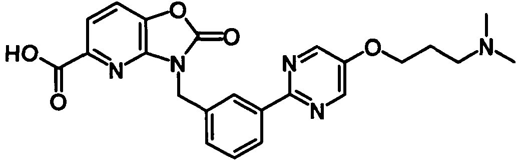 |
| "A133" | 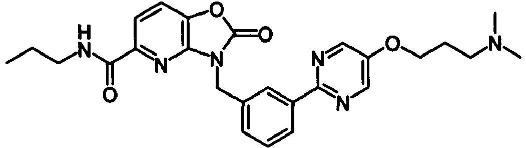 |
| "A134" | 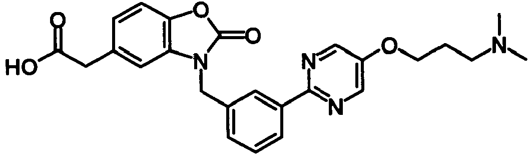 |
| "A135" | 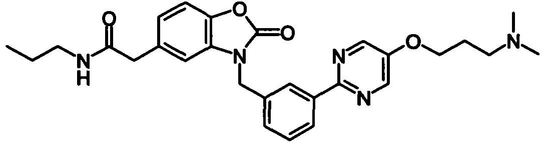 |
| "A136" | 3-{3-[5-(3-diméthylaminopropoxy)pyrimidin-2-yl]benzyl}-6-(1-hydroxyéthyl)-3H-benzoxazol-2-one |
| "A137" | 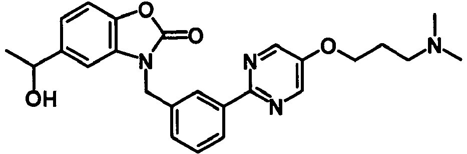 |
| "A138" | N-[3-(6-chloro-2-oxobenzoxazol-3-ylméthyl)-phényl]acétamide |
| "A139" | 1-[3-(6-chloro-2-oxobenzoxazol-3-ylméthyl)phényl]-3-[3-(4-méthylpipérazin-1-yl)propyl]urée |
| "A140" | 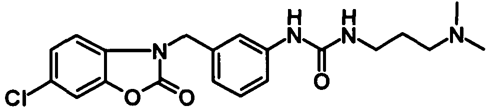 |
| "A141" | N-[3-(6-chloro-2-oxobenzoxazol-3-ylméthyl)phényl]-N'-(2-diméthylaminoéthyl)oxalamide |
| "A142" | N-(4-diméthylaminobutyl)-2-[3-(5-méthyl-2-oxooxazolo[4,5-b]pyridin-3-ylméthyl)phényl]pyrimidine-5-carboxamide |
| "A143" | 5-méthyl-3-[3-(3-méthyl-6-oxo-6H-pyridazin-1-yl)benzyl]-3H-oxazolo[4,5-b]pyridin-2-one |
| "A144" | 5-méthyl-3-[3-(5-méthylpyridin-2-yl)benzyl]-3H-oxazolo[4,5-b]pyridin-2-one |
| "A145" | 5-méthyl-3-(3-pyrimidin-5-ylbenzyl)-3H-oxazolo[4,5-b]pyridin-2-one |
| "A146" | 5-méthyl-3-[3-(4-pipérazin-1-ylpyrimidin-2-yl)benzyl]-3H-oxazolo[4,5-b]pyridin-2-one |
| "A147" | 5-méthyl-3-{3-[5-(1-méthyl-1H-pyrazol-4-yl)pyrimidin-2-yl]benzyl}-3H-oxazolo[4,5-b]pyridin-2-one |
| "A148" | 3-{3-[5-(3-diméthylaminopropoxy)pyrimidin-2-yl]benzyl}-5-(1-méthyl-1 H-pyrazol-4-yl)-3H-oxazolo[4,5-b]pyridin-2-one |
et les solvats, sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

3. Utilisation de composés selon la revendication 1 et de sels, solvats, tautomères et stéréoisomères pharmaceutiquement utilisables de ceux-ci, y compris des mélanges de ceux-ci en toutes proportions, pour la préparation d'un médicament destiné au traitement de maladies dans lesquelles l'inhibition, la régulation et/ou la modulation de la transduction des signaux de kinases joue un rôle.

4. Utilisation selon la revendication 3 de composés selon la revendication 1 et de sels, solvats et stéréoisomères pharmaceutiquement utilisables de ceux-ci, y compris des mélanges de ceux-ci en toutes proportions,
pour la préparation d'un médicament destiné au traitement de maladies qui sont influencées par l'inhibition de tyrosine kinases par les composés selon la revendication 1.

5. Utilisation selon la revendication 4, pour la préparation d'un médicament destiné au traitement de maladies qui sont influencées par l'inhibition de Met kinase par les composés selon la revendication 1.

6. Utilisation selon la revendication 4 ou 5, dans laquelle la maladie à traiter est une tumeur solide.

7. Utilisation selon la revendication 6, dans laquelle la tumeur solide provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

8. Utilisation selon la revendication 6, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

9. Utilisation selon la revendication 6, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

10. Utilisation selon la revendication 4 ou 5, dans laquelle la maladie à traiter est une tumeur du sang et du système immunitaire.

11. Utilisation selon la revendication 10, dans laquelle la tumeur provient du groupe constitué par la leucémie myéloïde aiguë, la leucémie myéloïde chronique, la leucémie lymphatique aiguë et/ou la leucémie lymphatique chronique.

12. Médicaments comprenant au moins un composé selon la revendication 1, et/ou des sels, solvats et stéréoisomères pharmaceutiquement utilisables de celui-ci, y compris des mélanges de ceux-ci en toutes proportions, et au moins un autre composé actif médicamenteux.

13. Ensemble (kit) constitué de conditionnements séparés
(a) d'une quantité efficace d'un composé selon la revendication 1, et/ou de sels, solvats et stéréoisomères pharmaceutiquement utilisables de celui-ci, y compris des mélanges de ceux-ci en toutes proportions,
et
(b) d'une quantité efficace d'un autre composé actif médicamenteux.
